# EUROPEAN PATENT APPLICATION

(11) **EP 2 366 782 A1**
(43) Date of publication of application: **21.09.2011**
(21) Application number: 10002448.8
(22) Date of filing: 09.03.2010
(51) Int. Cl.: C12N 9/10, C12N 15/09

(54) **Polypeptide having diterpene synthase activity**

(71) Applicant: Sandoz AG, 4056 Basel (CH)
(72) Inventor: The designation of the inventor has not yet been filed
(74) Representative: Wichmann, Hendrik

(57) **Abstract**

The present application, among others, relates to novel polypeptides having diterpene synthase activity, nucleic acid molecules encoding same, as well as to a gene cluster from *Clitopilus passeckerianus* which is thought to be involved in the biosynthetic pathway for producing pleuromutilin.

SEQUENCE LISTING

<110>  Sandoz AG

<120>  Polypeptide Having Diterpene Synthase Activity

<130>  S66978EP

<160>  15

<170>  PatentIn version 3.5

<210>  1
<211>  31
<212>  PRT
<213>  Artificial Sequence

<220>
<223>  partial protein sequence of Clitopilus pinsitus

<400>  1

Gly Asn Phe Met Ala Thr Pro Ser Thr Thr Ala Ala Tyr Leu Met Lys
1               5                   10                  15

Ala Thr Lys Trp Asp Asp Arg Ala Glu Asp Tyr Leu Arg His Val
            20                  25                  30

<210>  2
<211>  29
<212>  PRT
<213>  Artificial Sequence

<220>
<223>  partial protein sequence of Clitopilus pinsitus

<400>  2

Phe Glu Ala Pro Thr Tyr Phe Arg Cys Tyr Ser Phe Glu Arg Asn Ala
1               5                   10                  15

Ser Val Thr Val Asn Ser Asn Cys Leu Met Ser Leu Leu
            20                  25

<210>  3
<211>  26
<212>  PRT
<213>  Artificial Sequence

<220>
<223>  partial protein sequence of Clitopilus pinsitus

<400>  3

Arg Leu Ala Asn Asp Leu His Ser Ile Ser Arg Asp Phe Asn Glu Val
1               5                   10                  15

Asn Leu Asn Ser Ile Met Phe Ser Glu Phe
            20                  25

<210>  4
<211>  30
<212>  PRT

<213>  Artificial Sequence

<220>
<223>  partial protein sequence of Clitopilus pinsitus

<400>  4

Asp Tyr Ile Asn Ile Ile Arg Val Thr Tyr Leu His Thr Ala Leu Tyr
1               5                   10                  15

Asp Asp Leu Gly Arg Leu Thr Arg Ala Asp Ile Ser Asn Ala
            20                  25                  30

<210>  5
<211>  31
<212>  PRT
<213>  Artificial Sequence

<220>
<223>  partial protein sequence of Clitopilus pinsitus

<400>  5

Tyr Ser Leu Leu Asn His Pro Arg Ala Gln Leu Ala Ser Asp Asn Asp
1               5                   10                  15

Lys Gly Leu Leu Arg Ser Glu Ile Glu His Tyr Phe Leu Ala Gly
            20                  25                  30

<210>  6
<211>  28
<212>  PRT
<213>  Artificial Sequence

<220>
<223>  partial protein sequence of Clitopilus pinsitus

<400>  6

Ser His Tyr Arg Trp Thr His Val Val Gly Ala Asp Asn Val Ala Gly
1               5                   10                  15

Thr Ile Ala Leu Val Phe Ala Leu Cys Leu Leu Gly
            20                  25

<210>  7
<211>  35
<212>  PRT
<213>  Artificial Sequence

<220>
<223>  partial protein sequence of Clitopilus pinsitus

<400>  7

Pro Ser Ser Thr Phe Ala Lys Val Glu Lys Gly Ala Ala Gly Lys Trp
1               5                   10                  15

Phe Glu Phe Leu Pro Tyr Met Thr Ile Ala Pro Ser Ser Leu Glu Gly
            20                  25                  30

Thr Pro Ile
        35

<210>  8
<211>  2880
<212>  DNA
<213>  Clitopilus pinsitus

<220>
<221>  CDS
<222>  (1)..(2880)

<400>  8
atg ggt cta tcc gaa gat ctt cat gca cgc gcc cga acc ctc atg cag      48
Met Gly Leu Ser Glu Asp Leu His Ala Arg Ala Arg Thr Leu Met Gln
1               5                   10                  15

act ctc gag tct gcg ctc aat acg cca ggt tct agg ggt att ggc acc      96
Thr Leu Glu Ser Ala Leu Asn Thr Pro Gly Ser Arg Gly Ile Gly Thr
            20                  25                  30

gcg aat ccg act atc tac gac act gct tgg gta gcc atg gtc tcc cgt      144
Ala Asn Pro Thr Ile Tyr Asp Thr Ala Trp Val Ala Met Val Ser Arg
        35                  40                  45

gag atc gac ggc aaa caa gtc ttc gtc ttc cca gag acc ttc acc tac      192
Glu Ile Asp Gly Lys Gln Val Phe Val Phe Pro Glu Thr Phe Thr Tyr
    50                  55                  60

atc tac gag cac cag gag gct gac ggc agt tgg tca ggg gat gga tcc      240
Ile Tyr Glu His Gln Glu Ala Asp Gly Ser Trp Ser Gly Asp Gly Ser
65                  70                  75                  80

ctc att gac tcc atc gtc aat act ctg gcc tgc ctt gtc gct ctc aag      288
Leu Ile Asp Ser Ile Val Asn Thr Leu Ala Cys Leu Val Ala Leu Lys
                85                  90                  95

atg cac gag agc aac gcc tca aaa ccc gac ata cct gcc cgt gcc aga      336
Met His Glu Ser Asn Ala Ser Lys Pro Asp Ile Pro Ala Arg Ala Arg
            100                 105                 110

gcc gct caa aat tat ctc gac gat gcc cta aag cgc tgg gac atc atg      384
Ala Ala Gln Asn Tyr Leu Asp Asp Ala Leu Lys Arg Trp Asp Ile Met
        115                 120                 125

gag act gag cgt gtc gcg tac gag atg atc gta ccc tgc ctc ctc aaa      432
Glu Thr Glu Arg Val Ala Tyr Glu Met Ile Val Pro Cys Leu Leu Lys
    130                 135                 140

caa ctc gac gcc ttt ggc gta tcc ttc acc ttc ccc cat cat gac ctc      480
Gln Leu Asp Ala Phe Gly Val Ser Phe Thr Phe Pro His His Asp Leu
145                 150                 155                 160

ctg tac aac atg tac gcc gga aag ctg gcg aag ctt aac tgg gag gct      528
Leu Tyr Asn Met Tyr Ala Gly Lys Leu Ala Lys Leu Asn Trp Glu Ala
                165                 170                 175

atc tac gcc aag aac agc tct ttg ctt cac tgc atg gag gca ttc gtc      576
Ile Tyr Ala Lys Asn Ser Ser Leu Leu His Cys Met Glu Ala Phe Val
            180                 185                 190

ggt gtc tgc gac ttc gat cgc atg cct cat ctc cta cgt gat ggt aac      624
Gly Val Cys Asp Phe Asp Arg Met Pro His Leu Leu Arg Asp Gly Asn
        195                 200                 205

ttc atg gct acg cca tcc acc acc gct gcg tac ctc atg aag gcc act      672
Phe Met Ala Thr Pro Ser Thr Thr Ala Ala Tyr Leu Met Lys Ala Thr

210                 215                 220

aag tgg gat gac cga gcg gaa gat tac ctt cgc cac gtt atc gag gtc          720
Lys Trp Asp Asp Arg Ala Glu Asp Tyr Leu Arg His Val Ile Glu Val
225                 230                 235                 240

tac gca ccc cat ggc cga gat gtt gtt cct aat ctc tgg ccg atg acc          768
Tyr Ala Pro His Gly Arg Asp Val Val Pro Asn Leu Trp Pro Met Thr
                245                 250                 255

ttc ttc gag atc gta tgg tcc ctc agc tcc ctt tat gac aac aac ctc          816
Phe Phe Glu Ile Val Trp Ser Leu Ser Ser Leu Tyr Asp Asn Asn Leu
            260                 265                 270

gaa ttt gca caa atg gat ccg gaa tgc ttg gat cgc att gcc ctc aaa          864
Glu Phe Ala Gln Met Asp Pro Glu Cys Leu Asp Arg Ile Ala Leu Lys
        275                 280                 285

tta cgt gaa ttc ctt gtg gca gga aaa ggt gtc tta ggc ttt gtt ccc          912
Leu Arg Glu Phe Leu Val Ala Gly Lys Gly Val Leu Gly Phe Val Pro
    290                 295                 300

gga acc act cac gac gct gac atg agc tcg aaa acc ctg atg ctc tta          960
Gly Thr Thr His Asp Ala Asp Met Ser Ser Lys Thr Leu Met Leu Leu
305                 310                 315                 320

caa gtt ctc aac cac cca tac tcc cat gac gaa ttc gtt aca gag ttt          1008
Gln Val Leu Asn His Pro Tyr Ser His Asp Glu Phe Val Thr Glu Phe
                325                 330                 335

gag gca cct acc tac ttc cgt tgc tac tcc ttc gaa agg aac gca agc          1056
Glu Ala Pro Thr Tyr Phe Arg Cys Tyr Ser Phe Glu Arg Asn Ala Ser
            340                 345                 350

gtg acc gtc aac tcc aac tgc ctt atg tcg ctc ctc cac gcc cct gat          1104
Val Thr Val Asn Ser Asn Cys Leu Met Ser Leu Leu His Ala Pro Asp
        355                 360                 365

gtc aac aag tac gaa tcc caa atc gtc aag atc gcc act tat gtc gcc          1152
Val Asn Lys Tyr Glu Ser Gln Ile Val Lys Ile Ala Thr Tyr Val Ala
    370                 375                 380

gat gtc tgg tgg aca tca gca ggt gtc gtc aaa gac aaa tgg aat gta          1200
Asp Val Trp Trp Thr Ser Ala Gly Val Val Lys Asp Lys Trp Asn Val
385                 390                 395                 400

tca gaa tgg tac tcc tcc atg ctg tcc tca cag gcg ctt gtc cgt ctc          1248
Ser Glu Trp Tyr Ser Ser Met Leu Ser Ser Gln Ala Leu Val Arg Leu
                405                 410                 415

ctt ttc gag cac gga aag ggc aac ctt aaa tcc ata tcc gag aag ctc          1296
Leu Phe Glu His Gly Lys Gly Asn Leu Lys Ser Ile Ser Glu Lys Leu
            420                 425                 430

ctg tct agg gtg tcc atc gcc tgc ttc acg atg atc agt cgt att ctc          1344
Leu Ser Arg Val Ser Ile Ala Cys Phe Thr Met Ile Ser Arg Ile Leu
        435                 440                 445

cag agc cag aag ccc gat ggc tct tgg gga tgc gct gaa gaa acc tca          1392
Gln Ser Gln Lys Pro Asp Gly Ser Trp Gly Cys Ala Glu Glu Thr Ser
    450                 455                 460

tac gct ctc att aca ctc gcc aac gtc gct tct ctt ccc act tgc gac          1440
Tyr Ala Leu Ile Thr Leu Ala Asn Val Ala Ser Leu Pro Thr Cys Asp
465                 470                 475                 480

ctc atc cgc gac cac ctg tac aaa gtc att gaa tcc gcg aag gca tac          1488
Leu Ile Arg Asp His Leu Tyr Lys Val Ile Glu Ser Ala Lys Ala Tyr
                485                 490                 495

ctc acc ccc atc ttc tac gcc cgc cct gct gcc aaa ccg gag gac cgt 1536
Leu Thr Pro Ile Phe Tyr Ala Arg Pro Ala Ala Lys Pro Glu Asp Arg
            500                 505                 510

gtc tgg att gac aag gtt aca tac agc gtc gag tca ttc cgc gat gcc 1584
Val Trp Ile Asp Lys Val Thr Tyr Ser Val Glu Ser Phe Arg Asp Ala
        515                 520                 525

tac ctt gtt tct gct ctc aac gta ccc atc ccc cgc ttc gat cca tct 1632
Tyr Leu Val Ser Ala Leu Asn Val Pro Ile Pro Arg Phe Asp Pro Ser
    530                 535                 540

tcc atc agc act ctt cct gct atc tcg caa acc ttg cca aag gaa ctc 1680
Ser Ile Ser Thr Leu Pro Ala Ile Ser Gln Thr Leu Pro Lys Glu Leu
545                 550                 555                 560

tct aag ttc ttc ggg cgt ctt gac atg ttc aag cct gct cct gaa tgg 1728
Ser Lys Phe Phe Gly Arg Leu Asp Met Phe Lys Pro Ala Pro Glu Trp
                565                 570                 575

cgc aag ctt acg tgg ggc att gag gcc act ctc atg ggc ccc gag ctt 1776
Arg Lys Leu Thr Trp Gly Ile Glu Ala Thr Leu Met Gly Pro Glu Leu
            580                 585                 590

aac cgt gtt cca tcg tcc acg ttc gcc aag gta gag aag gga gcg gcg 1824
Asn Arg Val Pro Ser Ser Thr Phe Ala Lys Val Glu Lys Gly Ala Ala
        595                 600                 605

ggc aaa tgg ttc gag ttc ttg cca tac atg acc atc gct cca agt agc 1872
Gly Lys Trp Phe Glu Phe Leu Pro Tyr Met Thr Ile Ala Pro Ser Ser
    610                 615                 620

ttg gaa ggc act cct atc agt tca caa ggg atg ctg gac gtg ctc gtt 1920
Leu Glu Gly Thr Pro Ile Ser Ser Gln Gly Met Leu Asp Val Leu Val
625                 630                 635                 640

ctc atc cgc ggt ctt tac aac acc gac gac tac ctc gat atg acc ctc 1968
Leu Ile Arg Gly Leu Tyr Asn Thr Asp Asp Tyr Leu Asp Met Thr Leu
                645                 650                 655

atc aag gcc acc aat gag gac tta gac gat ctc aag aag aag atc cgc 2016
Ile Lys Ala Thr Asn Glu Asp Leu Asp Asp Leu Lys Lys Lys Ile Arg
            660                 665                 670

gac cta ttc gcg gat ccg aag tcg ttc tcg acc ctc agc gag gtc ccg 2064
Asp Leu Phe Ala Asp Pro Lys Ser Phe Ser Thr Leu Ser Glu Val Pro
        675                 680                 685

gat gac cgg atg cct acg cac atc gag gtc att gag cgc ttt gcc tat 2112
Asp Asp Arg Met Pro Thr His Ile Glu Val Ile Glu Arg Phe Ala Tyr
    690                 695                 700

tcc ctg ttg aac cat cct cgt gcg cag ctc gcc agc gat aac gat aag 2160
Ser Leu Leu Asn His Pro Arg Ala Gln Leu Ala Ser Asp Asn Asp Lys
705                 710                 715                 720

ggt ctc ctc cgc tcc gaa att gag cac tat ttc ctg gca ggt att gct 2208
Gly Leu Leu Arg Ser Glu Ile Glu His Tyr Phe Leu Ala Gly Ile Ala
                725                 730                 735

cag tgc gaa gaa aac att ctc ctt cgt gaa cgt gga ctc gac aag gag 2256
Gln Cys Glu Glu Asn Ile Leu Leu Arg Glu Arg Gly Leu Asp Lys Glu
            740                 745                 750

cgc atc gga acc tct cac tat cgc tgg aca cat gtc gtt ggc gct gat 2304
Arg Ile Gly Thr Ser His Tyr Arg Trp Thr His Val Val Gly Ala Asp
        755                 760                 765

aac gtc gct ggg acc atc gcc ctc gtc ttc gcc ctt tgt ctt ctt ggt 2352
Asn Val Ala Gly Thr Ile Ala Leu Val Phe Ala Leu Cys Leu Leu Gly
    770                 775                 780

cat cag atc aat gaa gaa cga ggc tct cgc gat ttg gtg gac gtt ttc 2400
His Gln Ile Asn Glu Glu Arg Gly Ser Arg Asp Leu Val Asp Val Phe
785                 790                 795                 800

ccc tcc cca gtc ctg aag tac ttg ttc aac gac tgt gtc atg cac ttt 2448
Pro Ser Pro Val Leu Lys Tyr Leu Phe Asn Asp Cys Val Met His Phe
                805                 810                 815

ggt aca ttc tca agg ctc gcc aac gac ctt cac agt atc tcc cgc gac 2496
Gly Thr Phe Ser Arg Leu Ala Asn Asp Leu His Ser Ile Ser Arg Asp
            820                 825                 830

ttc aac gaa gtc aat ctc aac tcc atc atg ttc tcc gaa ttc acc gga 2544
Phe Asn Glu Val Asn Leu Asn Ser Ile Met Phe Ser Glu Phe Thr Gly
        835                 840                 845

cca aag tct ggt acc gat aca gag aag gct cgt gaa gct gct ctg ctt 2592
Pro Lys Ser Gly Thr Asp Thr Glu Lys Ala Arg Glu Ala Ala Leu Leu
    850                 855                 860

gaa ttg acc aaa ttc gaa cgc aag gct acc gac gat ggt ttc gag tac 2640
Glu Leu Thr Lys Phe Glu Arg Lys Ala Thr Asp Asp Gly Phe Glu Tyr
865                 870                 875                 880

ttg gtc cag caa ctc act cca cat gtc ggg gcc aaa cgc gca cgg gat 2688
Leu Val Gln Gln Leu Thr Pro His Val Gly Ala Lys Arg Ala Arg Asp
                885                 890                 895

tat atc aat ata atc cgc gtc acc tac ctg cac acg gcc ctc tac gat 2736
Tyr Ile Asn Ile Ile Arg Val Thr Tyr Leu His Thr Ala Leu Tyr Asp
            900                 905                 910

gac ctc ggt cgt ctc act cgt gca gat atc agc aac gcc aac cag gag 2784
Asp Leu Gly Arg Leu Thr Arg Ala Asp Ile Ser Asn Ala Asn Gln Glu
        915                 920                 925

gtg tcc aaa ggt acc aat ggg gtc aag aaa atc aat ggg tca tcg aca 2832
Val Ser Lys Gly Thr Asn Gly Val Lys Lys Ile Asn Gly Ser Ser Thr
    930                 935                 940

aat ggg acc aag gtc aca gca aat ggg agc aat gga atc cac cat tga 2880
Asn Gly Thr Lys Val Thr Ala Asn Gly Ser Asn Gly Ile His His
945                 950                 955

<210> 9
<211> 959
<212> PRT
<213> Clitopilus pinsitus

<400> 9

Met Gly Leu Ser Glu Asp Leu His Ala Arg Ala Arg Thr Leu Met Gln
1               5                   10                  15

Thr Leu Glu Ser Ala Leu Asn Thr Pro Gly Ser Arg Gly Ile Gly Thr
            20                  25                  30

Ala Asn Pro Thr Ile Tyr Asp Thr Ala Trp Val Ala Met Val Ser Arg
        35                  40                  45

Glu Ile Asp Gly Lys Gln Val Phe Val Phe Pro Glu Thr Phe Thr Tyr

    50                  55                  60

Ile Tyr Glu His Gln Glu Ala Asp Gly Ser Trp Ser Gly Asp Gly Ser
65                  70                  75                  80

Leu Ile Asp Ser Ile Val Asn Thr Leu Ala Cys Leu Val Ala Leu Lys
                85                  90                  95

Met His Glu Ser Asn Ala Ser Lys Pro Asp Ile Pro Ala Arg Ala Arg
            100                 105                 110

Ala Ala Gln Asn Tyr Leu Asp Asp Ala Leu Lys Arg Trp Asp Ile Met
        115                 120                 125

Glu Thr Glu Arg Val Ala Tyr Glu Met Ile Val Pro Cys Leu Leu Lys
    130                 135                 140

Gln Leu Asp Ala Phe Gly Val Ser Phe Thr Phe Pro His His Asp Leu
145                 150                 155                 160

Leu Tyr Asn Met Tyr Ala Gly Lys Leu Ala Lys Leu Asn Trp Glu Ala
                165                 170                 175

Ile Tyr Ala Lys Asn Ser Ser Leu Leu His Cys Met Glu Ala Phe Val
            180                 185                 190

Gly Val Cys Asp Phe Asp Arg Met Pro His Leu Leu Arg Asp Gly Asn
        195                 200                 205

Phe Met Ala Thr Pro Ser Thr Thr Ala Ala Tyr Leu Met Lys Ala Thr
    210                 215                 220

Lys Trp Asp Asp Arg Ala Glu Asp Tyr Leu Arg His Val Ile Glu Val
225                 230                 235                 240

Tyr Ala Pro His Gly Arg Asp Val Val Pro Asn Leu Trp Pro Met Thr
                245                 250                 255

Phe Phe Glu Ile Val Trp Ser Leu Ser Ser Leu Tyr Asp Asn Asn Leu
            260                 265                 270

Glu Phe Ala Gln Met Asp Pro Glu Cys Leu Asp Arg Ile Ala Leu Lys
        275                 280                 285

Leu Arg Glu Phe Leu Val Ala Gly Lys Gly Val Leu Gly Phe Val Pro
    290                 295                 300

Gly Thr Thr His Asp Ala Asp Met Ser Ser Lys Thr Leu Met Leu Leu
305                 310                 315                 320

Gln Val Leu Asn His Pro Tyr Ser His Asp Glu Phe Val Thr Glu Phe
                325                 330                 335

Glu Ala Pro Thr Tyr Phe Arg Cys Tyr Ser Phe Glu Arg Asn Ala Ser
            340                 345                 350

Val Thr Val Asn Ser Asn Cys Leu Met Ser Leu Leu His Ala Pro Asp
        355                 360                 365

Val Asn Lys Tyr Glu Ser Gln Ile Val Lys Ile Ala Thr Tyr Val Ala
    370                 375                 380

Asp Val Trp Trp Thr Ser Ala Gly Val Val Lys Asp Lys Trp Asn Val
385                 390                 395                 400

Ser Glu Trp Tyr Ser Ser Met Leu Ser Ser Gln Ala Leu Val Arg Leu
                405                 410                 415

Leu Phe Glu His Gly Lys Gly Asn Leu Lys Ser Ile Ser Glu Lys Leu
            420                 425                 430

Leu Ser Arg Val Ser Ile Ala Cys Phe Thr Met Ile Ser Arg Ile Leu
        435                 440                 445

Gln Ser Gln Lys Pro Asp Gly Ser Trp Gly Cys Ala Glu Glu Thr Ser
    450                 455                 460

Tyr Ala Leu Ile Thr Leu Ala Asn Val Ala Ser Leu Pro Thr Cys Asp
465                 470                 475                 480

Leu Ile Arg Asp His Leu Tyr Lys Val Ile Glu Ser Ala Lys Ala Tyr
                485                 490                 495

Leu Thr Pro Ile Phe Tyr Ala Arg Pro Ala Ala Lys Pro Glu Asp Arg
            500                 505                 510

Val Trp Ile Asp Lys Val Thr Tyr Ser Val Glu Ser Phe Arg Asp Ala
        515                 520                 525

Tyr Leu Val Ser Ala Leu Asn Val Pro Ile Pro Arg Phe Asp Pro Ser
    530                 535                 540

Ser Ile Ser Thr Leu Pro Ala Ile Ser Gln Thr Leu Pro Lys Glu Leu
545                 550                 555                 560

Ser Lys Phe Phe Gly Arg Leu Asp Met Phe Lys Pro Ala Pro Glu Trp
                565                 570                 575

Arg Lys Leu Thr Trp Gly Ile Glu Ala Thr Leu Met Gly Pro Glu Leu
            580                 585                 590

Asn Arg Val Pro Ser Ser Thr Phe Ala Lys Val Glu Lys Gly Ala Ala
        595                 600                 605

Gly Lys Trp Phe Glu Phe Leu Pro Tyr Met Thr Ile Ala Pro Ser Ser
    610                 615                 620

Leu Glu Gly Thr Pro Ile Ser Ser Gln Gly Met Leu Asp Val Leu Val
625                 630                 635                 640

Leu Ile Arg Gly Leu Tyr Asn Thr Asp Asp Tyr Leu Asp Met Thr Leu
                645                 650                 655

Ile Lys Ala Thr Asn Glu Asp Leu Asp Asp Leu Lys Lys Lys Ile Arg
            660                 665                 670

Asp Leu Phe Ala Asp Pro Lys Ser Phe Ser Thr Leu Ser Glu Val Pro
        675                 680                 685

Asp Asp Arg Met Pro Thr His Ile Glu Val Ile Glu Arg Phe Ala Tyr
    690                 695                 700

Ser Leu Leu Asn His Pro Arg Ala Gln Leu Ala Ser Asp Asn Asp Lys
705                 710                 715                 720

Gly Leu Leu Arg Ser Glu Ile Glu His Tyr Phe Leu Ala Gly Ile Ala
                725                 730                 735

Gln Cys Glu Glu Asn Ile Leu Leu Arg Glu Arg Gly Leu Asp Lys Glu
            740                 745                 750

Arg Ile Gly Thr Ser His Tyr Arg Trp Thr His Val Val Gly Ala Asp
        755                 760                 765

Asn Val Ala Gly Thr Ile Ala Leu Val Phe Ala Leu Cys Leu Leu Gly
    770                 775                 780

His Gln Ile Asn Glu Glu Arg Gly Ser Arg Asp Leu Val Asp Val Phe
785                 790                 795                 800

Pro Ser Pro Val Leu Lys Tyr Leu Phe Asn Asp Cys Val Met His Phe
                805                 810                 815

Gly Thr Phe Ser Arg Leu Ala Asn Asp Leu His Ser Ile Ser Arg Asp
            820                 825                 830

Phe Asn Glu Val Asn Leu Asn Ser Ile Met Phe Ser Glu Phe Thr Gly
        835                 840                 845

Pro Lys Ser Gly Thr Asp Thr Glu Lys Ala Arg Glu Ala Ala Leu Leu
    850                 855                 860

Glu Leu Thr Lys Phe Glu Arg Lys Ala Thr Asp Asp Gly Phe Glu Tyr
865                 870                 875                 880

Leu Val Gln Gln Leu Thr Pro His Val Gly Ala Lys Arg Ala Arg Asp
                885                 890                 895

Tyr Ile Asn Ile Ile Arg Val Thr Tyr Leu His Thr Ala Leu Tyr Asp
            900                 905                 910

Asp Leu Gly Arg Leu Thr Arg Ala Asp Ile Ser Asn Ala Asn Gln Glu
        915                 920                 925

Val Ser Lys Gly Thr Asn Gly Val Lys Lys Ile Asn Gly Ser Ser Thr
    930                 935                 940

Asn Gly Thr Lys Val Thr Ala Asn Gly Ser Asn Gly Ile His His
945                 950                 955

<210>  10
<211>  93
<212>  DNA
<213>  Artificial Sequence

<220>
<223>  partial nucleic acid sequence of Clitopilus pinsitus

<400>  10
ggtaacttca tggctacgcc atccaccacc gctgcgtacc tcatgaaggc cactaagtgg      60

gatgaccgag cggaagatta ccttcgccac gtt                                   93

<210>  11
<211>  87
<212>  DNA
<213>  Artificial Sequence

<220>
<223>  partial nucleic acid sequence of Clitopilus pinsitus

<400>  11
tttgaggcac ctacctactt ccgttgctac tccttcgaaa ggaacgcaag cgtgaccgtc      60

aactccaact gccttatgtc gctcctc                                          87

<210>  12
<211>  78
<212>  DNA
<213>  Artificial Sequence

<220>
<223>  partial nucleic acid sequence of Clitopilus pinsitus

<400>  12
aggctcgcca acgaccttca cagtatctcc cgcgacttca acgaagtcaa tctcaactcc      60

atcatgttct ccgaattc                                                    78

<210>  13
<211>  3041
<212>  DNA
<213>  Clitopilus pinsitus

<220>

<221>  CDS
<222>  (1)..(786)

<220>
<221>  CDS
<222>  (841)..(961)

<220>
<221>  CDS
<222>  (1017)..(1303)

<220>
<221>  CDS
<222>  (1356)..(3038)

<400>  13
atg ggt cta tcc gaa gat ctt cat gca cgc gcc cga acc ctc atg cag      48
Met Gly Leu Ser Glu Asp Leu His Ala Arg Ala Arg Thr Leu Met Gln
1               5                   10                  15

act ctc gag tct gcg ctc aat acg cca ggt tct agg ggt att ggc acc      96
Thr Leu Glu Ser Ala Leu Asn Thr Pro Gly Ser Arg Gly Ile Gly Thr
            20                  25                  30

gcg aat ccg act atc tac gac act gct tgg gta gcc atg gtc tcc cgt      144
Ala Asn Pro Thr Ile Tyr Asp Thr Ala Trp Val Ala Met Val Ser Arg
        35                  40                  45

gag atc gac ggc aaa caa gtc ttc gtc ttc cca gag acc ttc acc tac      192
Glu Ile Asp Gly Lys Gln Val Phe Val Phe Pro Glu Thr Phe Thr Tyr
    50                  55                  60

atc tac gag cac cag gag gct gac ggc agt tgg tca ggg gat gga tcc      240
Ile Tyr Glu His Gln Glu Ala Asp Gly Ser Trp Ser Gly Asp Gly Ser
65                  70                  75                  80

ctc att gac tcc atc gtc aat act ctg gcc tgc ctt gtc gct ctc aag      288
Leu Ile Asp Ser Ile Val Asn Thr Leu Ala Cys Leu Val Ala Leu Lys
                85                  90                  95

atg cac gag agc aac gcc tca aaa ccc gac ata cct gcc cgt gcc aga      336
Met His Glu Ser Asn Ala Ser Lys Pro Asp Ile Pro Ala Arg Ala Arg
            100                 105                 110

gcc gct caa aat tat ctc gac gat gcc cta aag cgc tgg gac atc atg      384
Ala Ala Gln Asn Tyr Leu Asp Asp Ala Leu Lys Arg Trp Asp Ile Met
        115                 120                 125

gag act gag cgt gtc gcg tac gag atg atc gta ccc tgc ctc ctc aaa      432
Glu Thr Glu Arg Val Ala Tyr Glu Met Ile Val Pro Cys Leu Leu Lys
    130                 135                 140

caa ctc gac gcc ttt ggc gta tcc ttc acc ttc ccc cat cat gac ctc      480
Gln Leu Asp Ala Phe Gly Val Ser Phe Thr Phe Pro His His Asp Leu
145                 150                 155                 160

ctg tac aac atg tac gcc gga aag ctg gcg aag ctt aac tgg gag gct      528
Leu Tyr Asn Met Tyr Ala Gly Lys Leu Ala Lys Leu Asn Trp Glu Ala
                165                 170                 175

atc tac gcc aag aac agc tct ttg ctt cac tgc atg gag gca ttc gtc      576
Ile Tyr Ala Lys Asn Ser Ser Leu Leu His Cys Met Glu Ala Phe Val
            180                 185                 190

ggt gtc tgc gac ttc gat cgc atg cct cat ctc cta cgt gat ggt aac      624
Gly Val Cys Asp Phe Asp Arg Met Pro His Leu Leu Arg Asp Gly Asn
        195                 200                 205

ttc atg gct acg cca tcc acc acc gct gcg tac ctc atg aag gcc act      672

Phe Met Ala Thr Pro Ser Thr Thr Ala Ala Tyr Leu Met Lys Ala Thr
    210                 215                 220

aag tgg gat gac cga gcg gaa gat tac ctt cgc cac gtt atc gag gtc       720
Lys Trp Asp Asp Arg Ala Glu Asp Tyr Leu Arg His Val Ile Glu Val
225                 230                 235                 240

tac gca ccc cat ggc cga gat gtt gtt cct aat ctc tgg ccg atg acc       768
Tyr Ala Pro His Gly Arg Asp Val Val Pro Asn Leu Trp Pro Met Thr
                245                 250                 255

ttc ttc gag atc gta tgg gtatgttctc tcattattta tttactaact              816
Phe Phe Glu Ile Val Trp
            260

cagtactaac tacctggttt ccag tcc ctc agc tcc ctt tat gac aac aac        867
                           Ser Leu Ser Ser Leu Tyr Asp Asn Asn
                                   265                 270

ctc gaa ttt gca caa atg gat ccg gaa tgc ttg gat cgc att gcc ctc       915
Leu Glu Phe Ala Gln Met Asp Pro Glu Cys Leu Asp Arg Ile Ala Leu
            275                 280                 285

aaa tta cgt gaa ttc ctt gtg gca gga aaa ggt gtc tta ggc ttt g         961
Lys Leu Arg Glu Phe Leu Val Ala Gly Lys Gly Val Leu Gly Phe
        290                 295                 300

gtcagtcctt cttcgagcat tttgacgtat catggctgat gaacgacctg gatag tt      1018
                                                              Val

ccc gga acc act cac gac gct gac atg agc tcg aaa acc ctg atg ctc      1066
Pro Gly Thr Thr His Asp Ala Asp Met Ser Ser Lys Thr Leu Met Leu
    305                 310                 315

tta caa gtt ctc aac cac cca tac tcc cat gac gaa ttc gtt aca gag      1114
Leu Gln Val Leu Asn His Pro Tyr Ser His Asp Glu Phe Val Thr Glu
320                 325                 330                 335

ttt gag gca cct acc tac ttc cgt tgc tac tcc ttc gaa agg aac gca      1162
Phe Glu Ala Pro Thr Tyr Phe Arg Cys Tyr Ser Phe Glu Arg Asn Ala
                340                 345                 350

agc gtg acc gtc aac tcc aac tgc ctt atg tcg ctc ctc cac gcc cct      1210
Ser Val Thr Val Asn Ser Asn Cys Leu Met Ser Leu Leu His Ala Pro
            355                 360                 365

gat gtc aac aag tac gaa tcc caa atc gtc aag atc gcc act tat gtc      1258
Asp Val Asn Lys Tyr Glu Ser Gln Ile Val Lys Ile Ala Thr Tyr Val
        370                 375                 380

gcc gat gtc tgg tgg aca tca gca ggt gtc gtc aaa gac aaa tgg          1303
Ala Asp Val Trp Trp Thr Ser Ala Gly Val Val Lys Asp Lys Trp
    385                 390                 395

gtaagccata ccttatcaat tgatctgact gtcaactaaa ctatcctttc ag aat gta    1361
                                                          Asn Val
                                                              400

tca gaa tgg tac tcc tcc atg ctg tcc tca cag gcg ctt gtc cgt ctc      1409
Ser Glu Trp Tyr Ser Ser Met Leu Ser Ser Gln Ala Leu Val Arg Leu
                405                 410                 415

ctt ttc gag cac gga aag ggc aac ctt aaa tcc ata tcc gag aag ctc      1457
Leu Phe Glu His Gly Lys Gly Asn Leu Lys Ser Ile Ser Glu Lys Leu
            420                 425                 430

ctg tct agg gtg tcc atc gcc tgc ttc acg atg atc agt cgt att ctc      1505
Leu Ser Arg Val Ser Ile Ala Cys Phe Thr Met Ile Ser Arg Ile Leu

435                 440                 445

cag agc cag aag ccc gat ggc tct tgg gga tgc gct gaa gaa acc tca          1553
Gln Ser Gln Lys Pro Asp Gly Ser Trp Gly Cys Ala Glu Glu Thr Ser
    450                 455                 460

tac gct ctc att aca ctc gcc aac gtc gct tct ctt ccc act tgc gac          1601
Tyr Ala Leu Ile Thr Leu Ala Asn Val Ala Ser Leu Pro Thr Cys Asp
465                 470                 475                 480

ctc atc cgc gac cac ctg tac aaa gtc att gaa tcc gcg aag gca tac          1649
Leu Ile Arg Asp His Leu Tyr Lys Val Ile Glu Ser Ala Lys Ala Tyr
                485                 490                 495

ctc acc ccc atc ttc tac gcc cgc cct gct gcc aaa ccg gag gac cgt          1697
Leu Thr Pro Ile Phe Tyr Ala Arg Pro Ala Ala Lys Pro Glu Asp Arg
            500                 505                 510

gtc tgg att gac aag gtt aca tac agc gtc gag tca ttc cgc gat gcc          1745
Val Trp Ile Asp Lys Val Thr Tyr Ser Val Glu Ser Phe Arg Asp Ala
        515                 520                 525

tac ctt gtt tct gct ctc aac gta ccc atc ccc cgc ttc gat cca tct          1793
Tyr Leu Val Ser Ala Leu Asn Val Pro Ile Pro Arg Phe Asp Pro Ser
    530                 535                 540

tcc atc agc act ctt cct gct atc tcg caa acc ttg cca aag gaa ctc          1841
Ser Ile Ser Thr Leu Pro Ala Ile Ser Gln Thr Leu Pro Lys Glu Leu
545                 550                 555                 560

tct aag ttc ttc ggg cgt ctt gac atg ttc aag cct gct cct gaa tgg          1889
Ser Lys Phe Phe Gly Arg Leu Asp Met Phe Lys Pro Ala Pro Glu Trp
                565                 570                 575

cgc aag ctt acg tgg ggc att gag gcc act ctc atg ggc ccc gag ctt          1937
Arg Lys Leu Thr Trp Gly Ile Glu Ala Thr Leu Met Gly Pro Glu Leu
            580                 585                 590

aac cgt gtt cca tcg tcc acg ttc gcc aag gta gag aag gga gcg gcg          1985
Asn Arg Val Pro Ser Ser Thr Phe Ala Lys Val Glu Lys Gly Ala Ala
        595                 600                 605

ggc aaa tgg ttc gag ttc ttg cca tac atg acc atc gct cca agt agc          2033
Gly Lys Trp Phe Glu Phe Leu Pro Tyr Met Thr Ile Ala Pro Ser Ser
    610                 615                 620

ttg gaa ggc act cct atc agt tca caa ggg atg ctg gac gtg ctc gtt          2081
Leu Glu Gly Thr Pro Ile Ser Ser Gln Gly Met Leu Asp Val Leu Val
625                 630                 635                 640

ctc atc cgc ggt ctt tac aac acc gac gac tac ctc gat atg acc ctc          2129
Leu Ile Arg Gly Leu Tyr Asn Thr Asp Asp Tyr Leu Asp Met Thr Leu
                645                 650                 655

atc aag gcc acc aat gag gac tta gac gat ctc aag aag aag atc cgc          2177
Ile Lys Ala Thr Asn Glu Asp Leu Asp Asp Leu Lys Lys Lys Ile Arg
            660                 665                 670

gac cta ttc gcg gat ccg aag tcg ttc tcg acc ctc agc gag gtc ccg          2225
Asp Leu Phe Ala Asp Pro Lys Ser Phe Ser Thr Leu Ser Glu Val Pro
        675                 680                 685

gat gac cgg atg cct acg cac atc gag gtc att gag cgc ttt gcc tat          2273
Asp Asp Arg Met Pro Thr His Ile Glu Val Ile Glu Arg Phe Ala Tyr
    690                 695                 700

tcc ctg ttg aac cat cct cgt gcg cag ctc gcc agc gat aac gat aag          2321
Ser Leu Leu Asn His Pro Arg Ala Gln Leu Ala Ser Asp Asn Asp Lys
705                 710                 715                 720

ggt ctc ctc cgc tcc gaa att gag cac tat ttc ctg gca ggt att gct 2369
Gly Leu Leu Arg Ser Glu Ile Glu His Tyr Phe Leu Ala Gly Ile Ala
                725                 730                 735

cag tgc gaa gaa aac att ctc ctt cgt gaa cgt gga ctc gac aag gag 2417
Gln Cys Glu Glu Asn Ile Leu Leu Arg Glu Arg Gly Leu Asp Lys Glu
            740                 745                 750

cgc atc gga acc tct cac tat cgc tgg aca cat gtc gtt ggc gct gat 2465
Arg Ile Gly Thr Ser His Tyr Arg Trp Thr His Val Val Gly Ala Asp
        755                 760                 765

aac gtc gct ggg acc atc gcc ctc gtc ttc gcc ctt tgt ctt ctt ggt 2513
Asn Val Ala Gly Thr Ile Ala Leu Val Phe Ala Leu Cys Leu Leu Gly
    770                 775                 780

cat cag atc aat gaa gaa cga ggc tct cgc gat ttg gtg gac gtt ttc 2561
His Gln Ile Asn Glu Glu Arg Gly Ser Arg Asp Leu Val Asp Val Phe
785                 790                 795                 800

ccc tcc cca gtc ctg aag tac ttg ttc aac gac tgt gtc atg cac ttt 2609
Pro Ser Pro Val Leu Lys Tyr Leu Phe Asn Asp Cys Val Met His Phe
                805                 810                 815

ggt aca ttc tca agg ctc gcc aac gac ctt cac agt atc tcc cgc gac 2657
Gly Thr Phe Ser Arg Leu Ala Asn Asp Leu His Ser Ile Ser Arg Asp
            820                 825                 830

ttc aac gaa gtc aat ctc aac tcc atc atg ttc tcc gaa ttc acc gga 2705
Phe Asn Glu Val Asn Leu Asn Ser Ile Met Phe Ser Glu Phe Thr Gly
        835                 840                 845

cca aag tct ggt acc gat aca gag aag gct cgt gaa gct gct ctg ctt 2753
Pro Lys Ser Gly Thr Asp Thr Glu Lys Ala Arg Glu Ala Ala Leu Leu
    850                 855                 860

gaa ttg acc aaa ttc gaa cgc aag gct acc gac gat ggt ttc gag tac 2801
Glu Leu Thr Lys Phe Glu Arg Lys Ala Thr Asp Asp Gly Phe Glu Tyr
865                 870                 875                 880

ttg gtc cag caa ctc act cca cat gtc ggg gcc aaa cgc gca cgg gat 2849
Leu Val Gln Gln Leu Thr Pro His Val Gly Ala Lys Arg Ala Arg Asp
                885                 890                 895

tat atc aat ata atc cgc gtc acc tac ctg cac acg gcc ctc tac gat 2897
Tyr Ile Asn Ile Ile Arg Val Thr Tyr Leu His Thr Ala Leu Tyr Asp
            900                 905                 910

gac ctc ggt cgt ctc act cgt gca gat atc agc aac gcc aac cag gag 2945
Asp Leu Gly Arg Leu Thr Arg Ala Asp Ile Ser Asn Ala Asn Gln Glu
        915                 920                 925

gtg tcc aaa ggt acc aat ggg gtc aag aaa atc aat ggg tca tcg aca 2993
Val Ser Lys Gly Thr Asn Gly Val Lys Lys Ile Asn Gly Ser Ser Thr
    930                 935                 940

aat ggg acc aag gtc aca gca aat ggg agc aat gga atc cac cat tga 3041
Asn Gly Thr Lys Val Thr Ala Asn Gly Ser Asn Gly Ile His His
945                 950                 955

<210> 14
<211> 959
<212> PRT
<213> Clitopilus pinsitus

<400> 14

Met Gly Leu Ser Glu Asp Leu His Ala Arg Ala Arg Thr Leu Met Gln
1               5                   10                  15

Thr Leu Glu Ser Ala Leu Asn Thr Pro Gly Ser Arg Gly Ile Gly Thr
            20                  25                  30

Ala Asn Pro Thr Ile Tyr Asp Thr Ala Trp Val Ala Met Val Ser Arg
        35                  40                  45

Glu Ile Asp Gly Lys Gln Val Phe Val Phe Pro Glu Thr Phe Thr Tyr
    50                  55                  60

Ile Tyr Glu His Gln Glu Ala Asp Gly Ser Trp Ser Gly Asp Gly Ser
65                  70                  75                  80

Leu Ile Asp Ser Ile Val Asn Thr Leu Ala Cys Leu Val Ala Leu Lys
                85                  90                  95

Met His Glu Ser Asn Ala Ser Lys Pro Asp Ile Pro Ala Arg Ala Arg
            100                 105                 110

Ala Ala Gln Asn Tyr Leu Asp Asp Ala Leu Lys Arg Trp Asp Ile Met
        115                 120                 125

Glu Thr Glu Arg Val Ala Tyr Glu Met Ile Val Pro Cys Leu Leu Lys
    130                 135                 140

Gln Leu Asp Ala Phe Gly Val Ser Phe Thr Phe Pro His His Asp Leu
145                 150                 155                 160

Leu Tyr Asn Met Tyr Ala Gly Lys Leu Ala Lys Leu Asn Trp Glu Ala
                165                 170                 175

Ile Tyr Ala Lys Asn Ser Ser Leu Leu His Cys Met Glu Ala Phe Val
            180                 185                 190

Gly Val Cys Asp Phe Asp Arg Met Pro His Leu Leu Arg Asp Gly Asn
        195                 200                 205

Phe Met Ala Thr Pro Ser Thr Thr Ala Ala Tyr Leu Met Lys Ala Thr
    210                 215                 220

Lys Trp Asp Asp Arg Ala Glu Asp Tyr Leu Arg His Val Ile Glu Val
225                 230                 235                 240

Tyr Ala Pro His Gly Arg Asp Val Val Pro Asn Leu Trp Pro Met Thr
                245                 250                 255

Phe Phe Glu Ile Val Trp Ser Leu Ser Ser Leu Tyr Asp Asn Asn Leu
            260                 265                 270

Glu Phe Ala Gln Met Asp Pro Glu Cys Leu Asp Arg Ile Ala Leu Lys

275                 280                 285

Leu Arg Glu Phe Leu Val Ala Gly Lys Gly Val Leu Gly Phe Val Pro
    290                 295                 300

Gly Thr Thr His Asp Ala Asp Met Ser Ser Lys Thr Leu Met Leu Leu
305                 310                 315                 320

Gln Val Leu Asn His Pro Tyr Ser His Asp Glu Phe Val Thr Glu Phe
                325                 330                 335

Glu Ala Pro Thr Tyr Phe Arg Cys Tyr Ser Phe Glu Arg Asn Ala Ser
            340                 345                 350

Val Thr Val Asn Ser Asn Cys Leu Met Ser Leu Leu His Ala Pro Asp
        355                 360                 365

Val Asn Lys Tyr Glu Ser Gln Ile Val Lys Ile Ala Thr Tyr Val Ala
    370                 375                 380

Asp Val Trp Trp Thr Ser Ala Gly Val Val Lys Asp Lys Trp Asn Val
385                 390                 395                 400

Ser Glu Trp Tyr Ser Ser Met Leu Ser Ser Gln Ala Leu Val Arg Leu
                405                 410                 415

Leu Phe Glu His Gly Lys Gly Asn Leu Lys Ser Ile Ser Glu Lys Leu
            420                 425                 430

Leu Ser Arg Val Ser Ile Ala Cys Phe Thr Met Ile Ser Arg Ile Leu
        435                 440                 445

Gln Ser Gln Lys Pro Asp Gly Ser Trp Gly Cys Ala Glu Glu Thr Ser
    450                 455                 460

Tyr Ala Leu Ile Thr Leu Ala Asn Val Ala Ser Leu Pro Thr Cys Asp
465                 470                 475                 480

Leu Ile Arg Asp His Leu Tyr Lys Val Ile Glu Ser Ala Lys Ala Tyr
                485                 490                 495

Leu Thr Pro Ile Phe Tyr Ala Arg Pro Ala Ala Lys Pro Glu Asp Arg
            500                 505                 510

Val Trp Ile Asp Lys Val Thr Tyr Ser Val Glu Ser Phe Arg Asp Ala
        515                 520                 525

Tyr Leu Val Ser Ala Leu Asn Val Pro Ile Pro Arg Phe Asp Pro Ser
    530                 535                 540

Ser Ile Ser Thr Leu Pro Ala Ile Ser Gln Thr Leu Pro Lys Glu Leu
545                 550                 555                 560

Ser Lys Phe Phe Gly Arg Leu Asp Met Phe Lys Pro Ala Pro Glu Trp
                565                 570                 575

Arg Lys Leu Thr Trp Gly Ile Glu Ala Thr Leu Met Gly Pro Glu Leu
            580                 585                 590

Asn Arg Val Pro Ser Ser Thr Phe Ala Lys Val Glu Lys Gly Ala Ala
        595                 600                 605

Gly Lys Trp Phe Glu Phe Leu Pro Tyr Met Thr Ile Ala Pro Ser Ser
    610                 615                 620

Leu Glu Gly Thr Pro Ile Ser Ser Gln Gly Met Leu Asp Val Leu Val
625                 630                 635                 640

Leu Ile Arg Gly Leu Tyr Asn Thr Asp Asp Tyr Leu Asp Met Thr Leu
                645                 650                 655

Ile Lys Ala Thr Asn Glu Asp Leu Asp Asp Leu Lys Lys Lys Ile Arg
            660                 665                 670

Asp Leu Phe Ala Asp Pro Lys Ser Phe Ser Thr Leu Ser Glu Val Pro
        675                 680                 685

Asp Asp Arg Met Pro Thr His Ile Glu Val Ile Glu Arg Phe Ala Tyr
    690                 695                 700

Ser Leu Leu Asn His Pro Arg Ala Gln Leu Ala Ser Asp Asn Asp Lys
705                 710                 715                 720

Gly Leu Leu Arg Ser Glu Ile Glu His Tyr Phe Leu Ala Gly Ile Ala
                725                 730                 735

Gln Cys Glu Glu Asn Ile Leu Leu Arg Glu Arg Gly Leu Asp Lys Glu
            740                 745                 750

Arg Ile Gly Thr Ser His Tyr Arg Trp Thr His Val Val Gly Ala Asp
        755                 760                 765

Asn Val Ala Gly Thr Ile Ala Leu Val Phe Ala Leu Cys Leu Leu Gly
    770                 775                 780

His Gln Ile Asn Glu Glu Arg Gly Ser Arg Asp Leu Val Asp Val Phe
785                 790                 795                 800

Pro Ser Pro Val Leu Lys Tyr Leu Phe Asn Asp Cys Val Met His Phe
                805                 810                 815

Gly Thr Phe Ser Arg Leu Ala Asn Asp Leu His Ser Ile Ser Arg Asp
            820                 825                 830

Phe Asn Glu Val Asn Leu Asn Ser Ile Met Phe Ser Glu Phe Thr Gly
        835                 840                 845

Pro Lys Ser Gly Thr Asp Thr Glu Lys Ala Arg Glu Ala Ala Leu Leu
    850                 855                 860

Glu Leu Thr Lys Phe Glu Arg Lys Ala Thr Asp Asp Gly Phe Glu Tyr
865                 870                 875                 880

Leu Val Gln Gln Leu Thr Pro His Val Gly Ala Lys Arg Ala Arg Asp
                885                 890                 895

Tyr Ile Asn Ile Ile Arg Val Thr Tyr Leu His Thr Ala Leu Tyr Asp
            900                 905                 910

Asp Leu Gly Arg Leu Thr Arg Ala Asp Ile Ser Asn Ala Asn Gln Glu
        915                 920                 925

Val Ser Lys Gly Thr Asn Gly Val Lys Lys Ile Asn Gly Ser Ser Thr
    930                 935                 940

Asn Gly Thr Lys Val Thr Ala Asn Gly Ser Asn Gly Ile His His
945                 950                 955

<210>  15
<211>  27050
<212>  DNA
<213>  Clitopilus pinsitus

<400>  15
cgacatgcca aggtatattg gcgtcagggc agaatagata gaagtttaag ctctaaacga      60

gattgacacc atgcggccca ccctccactt caggccggtt catgtaatgg caacaccgag     120

ttgacctccg agcagttcgt gggctcaatc cacttgagct agttcgtcga tcgactgagc     180

caggacgaca tacattctgt gattgcatcc gagtcacaag cgctgccagc gtctcctacg     240

tggaaaaact gcggatggcg catgacagcc actgcctcct ctggtcgtat cacgctagcc     300

tgcgtgacgt gcattgttat cgggtggcaa taagtctgat aacgatcccg aatgggatct     360

tggctaggtg gactgtcatg cctcatatgc atgccaactt tacccaattg gtgcttacag     420

atatatctgt gttcgcacac tccacctcgc attgtccacg acctccacct tgacattctt     480

cgagactctt cccaacatct atggctccgt caacggaacg tgctctacca gtccttgtaa     540

tatggactgc tataggcttg gcctactgga tagactctca gaagaagaaa aagcagcacc     600

tgccgcctgg gccaaagaaa cttccaatta tcggcaacgt gatggaccta ccggcgaagg     660

tcgaatggga aacctatgct cgctggggta aagagtacag tacgtcgact ctatgttaga     720

atgacgcccg tagactcatt gaagccttct gaaaatagac tctgatatca tacatgttag     780

cgccatggga acctcgatcg taatactgaa ttctgccaac gccgccaatg acttgttgct     840

gaagaggtcg gcgatctact cgagcaggta tggtttcagc acggtattgc caatgtctac     900

ctgacacgct ctatagacca cacagcacga tgcaccacga actgtaagta tgctgttcgc 960
tacaaattag cactgaagat tcacatcacg ttaccaggtc aggctggggc tttacgtggg 1020
ccttaatgcc ttatggcgag tcatggcggg ctggtcgtag aagcttcacc aagcacttca 1080
actcttcaaa ccccggtata aaccaacctc gtgagttgcg atatgtgaaa cggttcctca 1140
agcagcttta cgagaagccc gacgacgttc tcgatcatgt acggaagtat gtttttcgac 1200
aggtctttcg atgagccata aacctcattt ctttgacagc ttggtcggct ctacaacgct 1260
ttcaatgacc tatgggctcg agactgaacc ttacaacgat ccctatgttg acctggtaga 1320
gaaagctgtc cttgcagcgt ctgagattat gacatctggc gcctttcttg ttgacatcat 1380
ccctgccatg aaacacattc ctccatgggt cccagggact atcttccatc aaaaggccgc 1440
cttaatgcga ggtcatgcgt actatgttcg tgaacagcca ttcaaagttg cccaggatat 1500
gattgtaagc aaccttgccc aactttgtcc attcccttgc ctaattcatt cgtacttaga 1560
aaactggtga ctatgagccc tcctttgtat ccgacgctct tcgagatctt gagaactcgg 1620
aaaaccagga tgaagattta gagcacctca aggatgttgc tggtcaagtc tacattggta 1680
tatcatgcct ttctcttcgg tcgtggatgc ctctaattgt tgactgttta gctggtgctg 1740
atacgactgc atccgccttg ggcaccttct tcctcgccat ggtctgtttc cccgaagtac 1800
agaaaaaagc acaacgagaa ttggatagtg ttctcaatgg aaggatgccc gagcacgtcg 1860
acttcccatc tttcccatac ctcaacgctg tgatcaaaga agtctaccgg tgtgttattt 1920
atgcgtcgag cgcggggttt aggtcggctg acgttcgtga tgcagctgga gacctgtgac 1980
tcctatgggc gtacctcatc aaaccatctc agatgacgtt tacagggact accacatccc 2040
taagggatcc atcgtgttcg ccaaccaatg gtatgtttgc gttcttgact tccgtattcc 2100
aatcttgact tgtcttaagg gcaatgtcca acgacgagac cgattacccc cagccggacg 2160
aattccagcc tgagcgatac ttgaccgaag atggcaagcc taataaggcg gttagagacc 2220
cctttgatat tgcgttcggc ttcggtagaa ggtcagcaac tattcattga gctgcgccga 2280
ggatactgac ctcgcctttt agaatttgcg ctggtcgtta ccttgctcat tccaccatca 2340
ccttggctgc agcctctgtt ctgtcgctat ttgatctctt aaaagcagtt gacgaaaatg 2400
gcaaggaaat cgagcctact agagagtatc atcaggctat gatttcgtga gtgatttaat 2460
tcgctgctga acacccggcc ctggctaaac gccgtctgca gacgtccact tgatttcccg 2520
tgtcgtatca agccaaggag caaggaagcc gaggaggtta tccgtgcttg cccgttaacg 2580
ttcaccaagc caactcttgg tgtctagaca catgtttaca tcttcgaacg tgtatatcag 2640
aatggaatct cttgtattcg ttgaacacgc gtttgatcag aaaatctgag ataacgactt 2700
caaggttcaa tctccaacct ccgcatgtag tgaggataat accttcagcg aagatggtct 2760
tgtctcgcca tggccttatt cagtctcggg aacctatgcg ttcaagttaa gagtttattt 2820
acaaacatcg aatgaaagac agctaatggg cgtgcctact gtactacacg aggaggattc 2880
cattctcccc ggtacagacc aagaatgaca ctactgtcca acgacatcga agccttgccc 2940
cttcctgaga cagtcgcccc cagccaattc ggacagctat agacgaacca tgcgacagtc 3000

caaagatgtc caagggcctt ccagtaccag gccggtttaa taccgagctt ccttccgacc 3060
cagagaaccc catcctccat ctgtatgatc gccgcttgga cgcagaagaa ctccatagtc 3120
ccggccttgt gccaatctgc gtgaaccttg aaatccccaa tcgcatgcaa aactcccgag 3180
aggaagaatg cggtgtacag ttgtacgtaa agcgccgggt tagagccaga cttcaagccc 3240
agcatcttcg tcgaaagatg ttttcctggg gctgataggc acttattagg tttaggtcag 3300
agagggtgat gatgattgaa cagaagaagc ttactctgcg aactaattga tgccaggacc 3360
gacttttcat tgatatgaga taaggtttac ccaggaatag attagatgga cttaccccca 3420
gaaccgccta acggtagaag catctctcca gcggccaaac acggggaccc agtcctatca 3480
gtagctctaa tcagacccta gacctttcag tctggcgcag ttgacaatta cacacctgtg 3540
gttctgagta gcccgagaga acttgcatga cactcaatgc cgtaagaaga attgatactt 3600
ggtttgtcgt gaacaggagc catgcggcaa tatctgcaca ccgccagatc aaagggcgag 3660
aagcaagagg ttcgatgaat gcgactgggc ttcggctata cagcgagacc agatcacaaa 3720
taaggtaatg ccgaaaacct gtgacgagtt gctggaggag aaatgaaagg cgtgaagtct 3780
ttggggtgaa cctgtggacg aaaatcttcg gctcccaatt ccagccaacg ccacgccgac 3840
tttgcaccag ccatgtcgcc cacttgatgc gttcaagcaa cgaggcttgc tcgacacctt 3900
tctggttgcg gaattgcaat tctctctgga cgtccgtcaa gagaatataa tccgtggccg 3960
tgagggtaat aacgaagagg ttgttggcaa tgtcataatc caaggtcgaa tcgccggtcg 4020
tggtcatggt gaggtaggtg ttaagcccaa caataataac ccagagaacc catcgtcctc 4080
tagcaggccg aatggcacaa gagagtacca atagaatgaa agatagaacc agaagttccg 4140
gcgagaaggg cttcatgctg gtatcagtcc aaggaagacg gcctcgctgg ggaggatcct 4200
tgtcggtgtc gcagcattct tcaagcagat cctcactttt gagaccgctc cgagcggaaa 4260
acacgagtca aggccaaaga tgataagata ttacaatgca acactcttgg tacagttctg 4320
tcaccgagca tgtggtgcac caggttgata cggtggccac ggtaaaacaa tgggaaaacc 4380
ggcgaccgat accaccaggg gtttccattc ggcgtggaca agagattgta ttacgacccg 4440
aagtcgccac ctttcgtctt agctgtcagt ttctgtccag tttgctcctc atggcaaatc 4500
gctagtccca gcctccttga atccacaccg ccggcccgga gctatgtatt aacctggttc 4560
aagtctgttt cgaaccagca atggagcaag tccagcttct gtgtaaggca ctgatgaatg 4620
tcaatctcca acctgtcgat cttcgaagct cgatcaggat tacccccaat cagtattgtt 4680
acaaagatag cacacgtcca ggtccatcgc gggtaaattt taatttcccc tcggccgtgg 4740
gtctttgcgt aagccattct gcgggtacgg tacccaccta agtccaaaga aaacctcata 4800
ccgagactga tagagatcca acgaacggag atactgttct tgggcctgcg tcatacatgg 4860
aagacaggag tgtttccttt tcattaaatg tcaaaccaat agctgtgttg aaccaagtcg 4920
atttcttttc cgagcttgag aacttgaagt atgaaataaa aatacatttg catctatcag 4980
aaaggcgata tcttagagct acgtgtatag tctaccgaac tagcgatatc aatggtggat 5040

tccattgctc ccatttgctg tgaccttggt cccatttgtc gatgacccat tgattttctt 5100
gaccccattg gtacctttgg acacctcctg gttggcgttg ctgatatctg cacgagtgag 5160
acgaccgagg tcatcgtaga gggccgtgtg caggtaggtg acgcggatta tattgatata 5220
atcccgtgcg cgtttggccc cgacatgtgg agtgagttgc tggaccaagt actcgaaacc 5280
atcgtcggta gccttgcgtt cgaatttggt caattcaagc agagcagctt cacgagcctt 5340
ctctgtatcg gtaccagact ttggtccggt gaattcggag aacatgatgg agttgagatt 5400
gacttcgttg aagtcgcggg agatactgtg aaggtcgttg gcgagccttg agaatgtacc 5460
aaagtgcatg acacagtcgt tgaacaagta cttcaggact ggggagggga aaacgtccac 5520
caaatcgcga gagcctcgtt cttcattgat ctgatgacca agaagacaaa gggcgaagac 5580
gagggcgatg gtcccagcga cgttatcagc gccaacgaca tgtgtccagc gatagtgaga 5640
ggttccgatg cgctccttgt cgagtccacg ttcacgaagg agaatgtttt cttcgcactg 5700
agcaatacct gccaggaaat agtgctcaat ttcggagcgg aggagaccct tatcgttatc 5760
gctggcgagc tgcgcacgag gatggttcaa cagggaatag gcaaagcgct caatgacctc 5820
gatgtgcgta ggcatccggt catccgggac ctcgctgagg gtcgagaacg acttcggatc 5880
cgcgaatagg tcgcggatct tcttcttgag atcgtctaag tcctcattgg tggccttgat 5940
gagggtcata tcgaggtagt cgtcggtgtt gtaaagaccg cggatgagaa cgagcacgtc 6000
cagcatccct tgtgaactga taggagtgcc ttccaagcta cttggagcga tggtcatgta 6060
tggcaagaac tcgaaccatt tgcccgccgc tcccttctct accttggcga acgtggacga 6120
tggaacacgg ttaagctcgg ggcccatgag agtggcctca atgccccacg taagcttgcg 6180
ccattcagga gcaggcttga acatgtcaag acgcccgaag aacttagaga gttcctttgg 6240
caaggtttgc gagatagcag gaagagtgct gatggaagat ggatcgaagc gggggatggg 6300
tacgttgaga gcagaaacaa ggtaggcatc gcggaatgac tcgacgctgt atgtaacctt 6360
gtcaatccag acacggtcct ccggtttggc agcagggcgg gcgtagaaga tgggggtgag 6420
gtatgccttc gcggattcaa tgactttgta caggtggtcg cggatgaggt cgcaagtggg 6480
aagagaagcg acgttggcga gtgtaatgag agcgtatgag gtttcttcag cgcatcccca 6540
agagccatcg ggcttctggc tctggagaat acgactgatc atcgtgaagc aggcgatgga 6600
caccctagac aggagcttct cggatatgga tttaaggttg ccctttccgt gctcgaaaag 6660
gagacggaca agcgcctgtg aggacagcat ggaggagtac cattctgata cattctgaaa 6720
ggatagttta gttgacagtc agatcaattg ataaggtatg gcttacccat ttgtctttga 6780
cgacacctgc tgatgtccac cagacatcgg cgacataagt ggcgatcttg acgatttggg 6840
attcgtactt gttgacatca ggggcgtgga ggagcgacat aaggcagttg gagttgacgg 6900
tcacgcttgc gttcctttcg aaggagtagc aacggaagta ggtaggtgcc tcaaactctg 6960
taacgaattc gtcatgggag tatgggtggt tgagaacttg taagagcatc agggttttcg 7020
agctcatgtc agcgtcgtga gtggttccgg gaactatcca ggtcgttcat cagccatgat 7080
acgtcaaaat gctcgaagaa ggactgacca aagcctaaga caccttttcc tgccacaagg 7140

aattcacgta atttgagggc aatgcgatcc aagcattccg gatccatttg tgcaaattcg 7200
aggttgttgt cataaaggga gctgagggac tggaaaccag gtagttagta ctgagttagt 7260
aaataaataa tgagagaaca tacccatacg atctcgaaga aggtcatcgg ccagagatta 7320
ggaacaacat ctcggccatg gggtgcgtag acctcgataa cgtggcgaag gtaatcttcc 7380
gctcggtcat cccacttagt ggccttcatg aggtacgcag cggtggtgga tggcgtagcc 7440
atgaagttac catcacgtag gagatgaggc atgcgatcga agtcgcagac accgacgaat 7500
gcctccatgc agtgaagcaa agagctgttc ttggcgtaga tagcctccca gttaagcttc 7560
gccagctttc cggcgtacat gttgtacagg aggtcatgat gggggaaggt gaaggatacg 7620
ccaaaggcgt cgagttgttt gaggaggcag ggtacgatca tctcgtacgc gacacgctca 7680
gtctccatga tgtcccagcg ctttagggca tcgtcgagat aattttgagc ggctctggca 7740
cgggcaggta tgtcgggttt tgaggcgttg ctctcgtgca tcttgagagc gacaaggcag 7800
gccagagtat tgacgatgga gtcaatgagg gatccatccc ctgaccaact gccgtcagcc 7860
tcctggtgct cgtagatgta ggtgaaggtc tctgggaaga cgaagacttg tttgccgtcg 7920
atctcacggg agaccatggc tacccaagca gtgtcgtaga tagtcggatt cgcggtgcca 7980
atacccctag aacctggcgt attgagcgca gactcgagag tctgcatgag ggttcgggcg 8040
cgtgcatgaa gatcttcgga tagacccata gtgaaggtga gagcgcagcg aagtaaaggg 8100
agtctgggtt ggaaaggtgg tgagtctggg ttcaagtact ggaacgaaga cgggatctta 8160
cttttaagca gtctctggac tttgcatatt gggcaaaagc tccagtcaac caaaccacaa 8220
gttgtttggt tgaagctgac gtcttggggt cacatcaccc gactggtttg gctcagagtt 8280
caagctcgtt atccgtgaaa caagtgtcca ctcgaaggag ttggacaggt tctagatgcg 8340
taccaacatg gttgcaccaa taatggttca gccgatatca gaaccgacag aaaatcccgc 8400
aggcatgtca agattccgat gttccacaat tatcagaacc cggctatgtc aattgtgcga 8460
cgcgttatgg ccaagattgg gccaagagct aaggttccaa cgagaaggtg tcactggaag 8520
tccaggaaag gcaaacgaac ccgttcgaga agatttaggc ttgtagaaga accagctgac 8580
taagcccatc actcttcatt ttggcgtact gtgtactcgg ggcaaccact ttcatagtgc 8640
caaccatctg ccgtcttcac cggcggccta tctcttaaga aatctaagat ttggagataa 8700
taagtacaag attcgtgcta agcggtttct gacgacctgg cttagctccg tggttcctgg 8760
atcttctttg cctattccat tttgcatccg gacattcgcc atatcctccc gattctgtca 8820
gagcttggcc ccgctcatga cccctcaaac acaagggtct tgcgaattgg ggcactctga 8880
ctacatctgt caactcgata cctttctagg ctatacgctg ttagaactca gaggatattt 8940
cttatttatc tattcactga gccgaagtgt accaattagc attgcccact ccttcagatt 9000
tacctcccta aattcatacg atgagaatac ctaacgtctt tctctcttac ctgcgacaag 9060
tcgccgtcga cggcactctg tcatcttgtt ctggagtgaa atcacgaaag ccggtcattg 9120
cctatggctt tgacgactca caagactctc tcgtcgatgt aagcaccttc ttctttatca 9180

tttcaactct ggctcaccgg cttggtaaaa acctaggaga atgacgaaaa aatattggag 9240
ccctttggct actatcgtca tcttttgaaa ggcaagagcg ccaggacagt gttgatgcac 9300
tgcttcaacg cgttccttgg actgcccgaa gattgggtca ttggcgtaac aaaggccatt 9360
gaagaccttc ataatgcatc cctactgtga gcataatgtc cacactattt ttttttcgtt 9420
cgatctctga catcgcacct ggcagaattg atgacatcga agacgagtcc gctctccgtc 9480
gtggttcacc agccgcccac atgaagtacg ggattgccct gaccatgaac gcggggaatc 9540
ttgtctactt cacggtcctt caagacgtct atgacctcgg aatgaagaca ggcggcactc 9600
aggtcgccaa cgcaatggct cgcatctaca ctgaagagat gattgagctc caccgtggtc 9660
aaggcattga aatctggtgg cgtgaccagc ggtcccctcc ttccgtcgat caatacattc 9720
acatgctcga gcagagtgag tttttccacc gactgctgtc atccacggac atatcctgac 9780
tattccctca ccagaaaccg gcggcctgct caggcttggc gtacggctct tgcaatgcca 9840
tcccggtgtc aataacaggg ccgacctctc cgacattgcg ctccgtattg gtgtctacta 9900
ccaacttcgc gacgactaca tcaacctcat gtccacaagc taccatgacg agcgtggatt 9960
cgctgaggac ataaccgaag gaaagtacac tttcccgatg ttacactcac tcaagaggtc 10020
acctgattct ggactgcgtg gtatgtgttc agcagtcgct tgctttcaat gatttactga 10080
cagcccggga tttcatttag aaatcttgga ccttaaaccg gcagacattg ccctgaagaa 10140
gaaagctatc gctatcatgc aagatactgg atcgcttgtt gcaacccgga accttctcgg 10200
tgcagttaag aatgatctca gtggattggt tgctgaacag cgtggagacg actacgctat 10260
gagcgcgggt cttgaacgat tcttggaaaa gttgtacatc gcagagtaga taagaatctc 10320
aatagaattc gtccatgaat ggaacaatat ttaaatccaa ttcatctcaa ggctcctcgc 10380
aattgaatcc aaatttaaaa ttaaatactc gatttttcca gatgttattg caaccaaaac 10440
atctcgacgt cacaggttcg ccacagcctc ggtaaaggga atttgagggt atgatgcata 10500
tgttgatcca cctttgagtg gtgcccctcg acagacaatc atccttcctt tatctccttt 10560
cagcagaccc agcctaaacc ggggtagcta ttgacgactg caagttcagc gtggaagttc 10620
ttgacgatgg cattatctca catcacatgg gaaacaagac attgagaact gaacattcgt 10680
acattacgaa actctctatc gccgaatact atggtgttag gaggctacaa cgcagcgaac 10740
gcttccttaa tcaagtcttc cttcatctta tctcgaggtt caattttgca tgcgaacgga 10800
agtggaagag tctcaagaga aacctgtcag aataaaagtg atcgttaacc tcggcagccg 10860
tatcttgaga aaatgataag cgactatacg caccgacttg tcgtaacagt cctccatgtt 10920
catattcttc acagtgtcct tgtttgaaga atctgggtaa aaattgaatg cccaacagag 10980
cctcatgatg aagagaccct gtgccagttc atatcagcaa atttcttcga cccggaagaa 11040
cacctaacgt acagttgatc gttttgccag cttatcgcct gggcagactc tctaaagaca 11100
gtaaatgatg aggtaccgag tgaaggatag gatgagactc acacgtccag caccgaacag 11160
gaaatcggga ttgacgtctt cagataagcc tggcttcgtg ccgtttggcg acaagaaata 11220
gcgttcaggc ttgaaggcct caggttcgtc gaagagctct ggaggttagt gtcgtcggaa 11280

acacgtagtt aatcgagttg ggatcaggga cttaccgggg tcatggccca ttccccctag 11340
agactggtgc gttcaatgct cagaaatgtg agagataatc aactcacaga tgttcatgaa 11400
gatcatactt ccctctggca gtacgtaacc gccatactag gaagcccgtc agtgtccgaa 11460
aatcacgata agataccacg ttcgtgaact cacagacaag ctctcccgcg agacgtgggg 11520
aagggctaca gggccgactg gccgaagccg aaggacctaa gccacgcgat tgagaacaat 11580
gaaactgaca gatgtcttcc catgggactc acctcctgta ggaacgcctt gagataaggc 11640
aaccgttcca aatcattgaa gcatggcatg gtttcggtcc ccaaaacatt gtccagctcg 11700
tcctgtatct tgcgctggca gtccgggtgg gcgataagag caagaataca cgattcgatg 11760
tacgatatcg tggtcttcgc gccggcgtcc aagaaaccac cgctaaggtt tctagaaaat 11820
attatcagat actcgctcaa cgtatcagca gaggatttac gtacgataac tcaagccagc 11880
tacgaccatc cggatggtca atcacggact ctgcaaaaga tccggtcctg accccggaat 11940
ccatcgcctt cttggcacct tccaagagag aattgtagac accattacgg aaatccttga 12000
attcatccac aatggtcttc cagccggccc cggggaaacc gcgagggatg tagtctaaga 12060
aggggaaagc gtcgaccgct gcaccgttgt gagcgatttg accaattctg gtggcagctt 12120
cgtatgcatt ctcgataatc gtgccatagt aactttcgca acgtggctgg ccatacacaa 12180
tgtgcaggag tagcgacatc atagcacgcc taatatggat cggccgattc tatcgttagt 12240
tcgtcagtta aggacatcac taaccagaaa gattcgtcga actgacagga gcgtccatca 12300
atagatcgtg catgaggttc acagattcct cttcttgtcg cggtatgtag ccactcaagg 12360
cacttggcgt taggtaattg tggatacctt tgcgaccagt cttccatacg gaagtgtctg 12420
aagaaaatgc gttatcagga agtgtttaaa cggtgtagag aaatgacata cccatgcttt 12480
ccaccgtgag attcaggcct tctgtatacc gggcaatcat gggcgaaaat ggccggtctg 12540
agacatactc cagtcaattc cggtacgatt aggctgaact agaagaaacc aacctcctgt 12600
gatattaccc tgcttgtcaa gaatagtccg aacagccttt ggactgttca gaacaatcac 12660
agtgcgattc atcaatttga gctagaaacc atgagaagtt tattatttct atcgttctgc 12720
acgactcaca gagtacactt cgccatactc cctggcccac tctgtcaatc tggggtaaat 12780
tagctttagg tggccgagtt gaggacgggc aaaacatact gcattggaag ccacatcttc 12840
gtcatgagat gagcatttcc gagaacaggc ttggtaggtg gcccgggtgg caagaagttc 12900
tccctggagc ctagctgaag gagcttatag acggcaacag cggaacctgc agcagcagcc 12960
acgatcacgg gatccacgtt cgcaacagac gggaggtcga cggacagcat tgcgtgaata 13020
aagctcgagc ggtgcaaaag gtgtatggtc ccttcaccaa tgtcgagctc cccgggctct 13080
taaagtggtg ggggccgaac tgcatggtgg agaatcttga gtctggatga gcccacacga 13140
ctgtttgagc caggttacga tcggggttga actccatcct gaattgaact agttttcaga 13200
agatcctaat cactggttcg ccccaatatc gccaagggga acgaggagca ttgtcaaatc 13260
tacccgagct tcaagcagac atcgacgttc gaaggaaaga aggggaaaat taatgatggt 13320

ccctcttggc aatatccgag atccgaaacc ccttccgttt cctggaaggt gcacgttgct 13380
ccaagtcgat acttgactga aagttcttca gctccctagt catcggacgt ctgaactttg 13440
cagggtgcgt ttcaattccg tcctcagcat gataagcgtc tccgatcgtg gacttgcgca 13500
gtctcaagga gctttcagct gcttttcgag ttagtcgaat gtcggcactc ggtcggacat 13560
tattgcggta gtatgagaga aactccaaca caccactgct tatcatgcgc ctggctcaca 13620
agagcactat gtgagacacg ttcatctgct gtcatgattg tcaaaaggag ctagaaaggc 13680
gtaaaaataa tgtgacaggc ttccgagtgt gccaacagct gggccctccg tatccatcat 13740
tctaatcgtt cacaccgttc acacccaggc tcaggatgag aaagaatcgc atgaactctg 13800
agtatctcag catctatact gtacacccca aactatgcac atccaaaaaa gagaatgcaa 13860
tttccagaaa tctagactat aacgtcggca atccccctat ctaatagtct gcaacatcgt 13920
ggatcacctg cacaactgac tgactacgtg gtaccatctc gcattcaaac ggttttggca 13980
tcgagaccgg accctattgt gaacaactaa gaattttgtt ctggcacgcg tcgtggggca 14040
ctcacgggta caacgacatc gtccttcatt gacttggggc tgttaggcag gggcttgatg 14100
tcgaatcccc agatgatgtt caaagataca gtgcgctatc agaacatcag ctatcagttg 14160
tcactgaggt cacgagcgta gcataccttg aaaatttcag ccatcttgag tccaggacag 14220
agcctgcgcc cagcgccgaa agtgaaggta tgacggtagc cagtcaggtc aacgcttggt 14280
tttgtgccaa attcagactc catgtaccgt tcggggcgga aatcgtctgg ggcctcgaaa 14340
acatctgcaa aaaggcttca gtatggcttc acaatcagca atacaagagg tcactcactt 14400
gggtctcgtt ggatgccatc tatcacaaat tcagaatcag catgtcagaa aagagacaga 14460
agggtagcac tcacaaaggt tcatcacgat gacggtaccc ttcgggatga agtagccatt 14520
gtactaaggt attgtcagct ccatcaccta tagcgaggaa aagggtcaat tacttcgaaa 14580
tcctctgtcg agtaatgagg cggtacgatg ggactcggag gccagatgcg agttacctgc 14640
gaggatggaa tctgttaacc cttcatacat cacgtcaagt caatccacaa acctctctga 14700
cgacgcaatt gaagtatttc atcttcaatg catcttgata agttggcaaa cgcgagtcgt 14760
attcatcgcc catgacctcc ttcagctcat cacgaatctt ctgctggcat tcggggtgca 14820
tcgtcatcat gagcacgaag acacgagtga acatagcgag ggtatcagtt cctccgtcaa 14880
tcatgacgcc tccgtgatag ctgcagaatg tacgtcagtt tccattcaac atagcggtga 14940
tgggaaagga gagactaacg caataagatc cctatccttg aatccaaact catccttcct 15000
ctgaagaatg gtctgcatgt gagacccgtc gaagacgcca gcttccattc tcttctcaac 15060
ccttccgagg aaatcattaa agataccaag ttgcttgtcc ttgatacctt gagccatgac 15120
cctccagccg gccagactat caggaagcca cttggcgagc caaggaatta gagcggtgaa 15180
gtgaacacct cggagaccca tcatgttttc gaagtcgtga agatattctt cgtggtaggg 15240
aatgaatggg tctgaggagg tgaggacgcg ttcaccataa gcgatagcaa caatactgga 15300
catgctggtg cggacgagat gcctaaagaa ttcctgtgaa agcctctgtc aaaacagtcg 15360
atagtgctaa tcgaaatccc ttaccttggg ctcagccaac agctccttca tcagcacgat 15420

ggtctccgtc tcaatgttct ctgcatatcg atcaatactg tcgttgctaa tgagcaactt 15480
aaaggccttg tggttgattc ggaattcgtc ggattctgaa acagagtcag tcagataaag 15540
gacccacgaa caaagagctt gtcgtactgt aggaggcgat aggaaggaaa cggtcgtctt 15600
tgataggagc agggaggaaa ccagtgggtc tttcagcagt cttggcattc agcttgtcaa 15660
gaatgccagt aacggaggct gagtctgtta ggacgataac gttcttgaag aagatcttca 15720
actatgtggc ataaacttca tgaatattga gaaatgcggt gtatcgtcaa tactaacgct 15780
gtatattcct ccatattctt gtgcccatcg gctaagccta gaaaacgtgg ttaggtacgt 15840
caatgaagga gtagctttgg gcaacctact gaaggtgcat gtcgtccatt gctggcatct 15900
ggtggagatt acccaacacc ggcttcgtag gtggcccagg aggtaacgtc ttctccctcg 15960
accccatacg aagcagcttg tagaccaagt agcatgccaa agggatggcc acaggtgcga 16020
tcatgttgct gtcaagcaga gcagccttga cttcagaaag attcatagtc gtccgaaagt 16080
ctagaccctg tggaagttgg gcccattcct tgagttgact gatatccatg gttcagcgca 16140
cttggcaaga tcttgaagtt cccgggttac acgccgttat catgctgccc tatacttatg 16200
agagctcaat aaggttcggt ccaaaacgcc aatggtttgc ccgtcagaat caacacggtt 16260
tgctaaccaa agggtcctgc ctaatggaga gtgccaattt ttgatccaac tctgcgccgc 16320
catcaaatga gccctcgctt cctcgaaagc atgggttttg gcattttgcc tccggtggcg 16380
acggttctgc ttatccatgg gccgccggga gatttacttg gcttggctcg gttagattac 16440
ttggctggtt cattgttatc catcagagcg gtagactgac agtggcaaga attaacagcc 16500
agaacaatgg tctttccacg gcggcttgtt ccatgggaga aaggcttgga gcggtgtggt 16560
acgtatcttc accataattt catagtccac gcgatcccat tcaggaaaaa tgaagcactt 16620
gggtcgtaaa tctccgctct ctacgtgttc ctgcgaagtc ataaggagcg ggggaacccc 16680
tgtactgaaa ccaagcaata cgcagcagtg accatggaag gcaaggtcgt gctccattgt 16740
tttagtcatt atatgaaaat cctgctaacc atctgagtca catagatcgc aatcgttaca 16800
ggcgcatcca atggcattgg actcgccacc gtcaatctcc tcctcgcagc aggagcgtct 16860
gtctttggcg tagacctcgc tctagcaccg ccctcggtga cctccggaaa attcaaattc 16920
ctacaactca acatctgcga caaggatgca cccgccagga ttgtgtccgg ctccaaggac 16980
gcctttggaa gcgagagaat cgacgccctc ttgaacgtcg ctggtatctc ggactacttc 17040
cagaccgcgt tgaccttcga agacgatgta tgggacagag tcatcgatgt caacctggct 17100
gcacaagtga ggttgatgag agaggtattg aaggttatga aggtccagaa gtcaggtagt 17160
atcgtgaacg tagtcagcaa gctggccctc agcggtgctt gtggaggtgt cgcatacgtt 17220
gcgagtaaac atgccttggt aagaggatgt cccgctgcta gcatcgtact tgctaatgca 17280
agcaatcggc ttctgtagct tggtgtgaca aagaacaccg cgtggatgtt caaggacgat 17340
ggtattcgat gcaatgccgt ggcgcctggc tcgaccgaca ccaacattcg aaacacgaca 17400
gacccgacca aaatagatta cgatgcattc tctcgagcca tgtgagtatc ttccgtggat 17460

tttcgggatg tcgttcgttc tctgatcaaa gaccttggga taaggcctgt tatcggcgta 17520
cactgcaact tgcagaccgg cgagggtatg atgagccctg aacctgcagc ccaagcgatc 17580
ttcttcttag cttcagactt gagtaacggg acaaatggcg tcgttattcc ggtcgataac 17640
gggtggagtg tcatttaggt cgccgtatat tataggtaca caggccagca ttaggcgact 17700
tcgaatttta tgtaattagc caatgttacc tctgcgaaag aatgagttct tgcgtagatg 17760
gatgagcctc tcaagacgag caaggaaaca tacccttctt ttcgtcgtcg ctatctcgcc 17820
ttcatcatcg gcataccttt tccctaccat atccacatcc atttcatctc cggatctcga 17880
cctaatcatc ccaaccagac tcccaagccc tgacctatcc acaacacgct cagaatcatc 17940
agctctccaa tcgacctcct caagcatcat ctccgggaca tattccatat ccataggtgc 18000
cttcgttacg ccatatcttg ctcccaacac cccatgtgtg agaggggaac gtaggcgata 18060
gatggtgcgt ataaagtacg cgtattcttg ggagtgtccg tcatttgacg aagagcgtag 18120
gatcccaggt tcaatttcag ccacgtaggt gatatttgag taaagcccga gggaggtata 18180
gtaccagatc ctttcaacag gagtgggtaa ctggaaagga agaagttgat acttgacttc 18240
gccgagcacg atttgctggg tttgggccct ctagaacatt ctggctgtca gaactccgag 18300
tcaaggttgg ttggacctga gagagatggg cataccgaaa cagcgattat aacatccttc 18360
cctccaacgc ctccttcaga tgaagtccaa gcgaaagcca tggtggttgt cggaggagat 18420
gcccaggcaa atgccatgat cgctcatggc aagaagttga tgttccgagc ctgtcaagca 18480
ttgttgagtt ttgatgattg cctaaaggga aaggagtaag cagagaggcg cccgtagaag 18540
cgcacgagtt ggtgtaggac gttggtcctt cctcgaggtg ctgtccgtac gatcaagcaa 18600
accaggaaac aaagaaaatg ttcccaagtg acaacatgat caagagatat tgatattgcg 18660
gtacgaaaac atgttatgag tccccgtact cttacacctt cgcctcctct ctctttagcc 18720
accaggatga ccgctgtgga agggtatcct accctcctca cgaaattccg aaaccaggtc 18780
gttccgacct tcaagatggt ttcttgttca ttctccgtca agattccgga gctcgcttgt 18840
aggctggtgc gggactgatc acgaagatca gtatcttggt ggacgtaagg ttgagatcta 18900
ctcctaggac gcgtaaaagg cactgttggg gtcacagtgc gcctacgcga gtggaatccc 18960
cccgagtcag tgccaccttg gtgctgatca gagtgtgaat gccttatccg gtcattttcc 19020
tgcctttttt tgtagaagta cattttgtct cgtgatcatg tattctctcg cgcgcatcgc 19080
gcgtcttgaa ataattaggt tcttgacacc tcaccaccac gatctaagta ttgaggatca 19140
cacggatcct caccctcagc acgctaagta tttcattcag ttttcattct ttttaaagtc 19200
tacattttcg tggttgtatc gagaagccta cggcttctct tactacgcca cgtttgcgac 19260
tgatctctca gttctcccca cttctctcgt atccaggttc ccggaccgag tgcggttgat 19320
tcggcacccc gcggctgcaa gggattttct tcctcaccgc cttacccttg ttctgcccct 19380
tgtctatata tcttcggtca acttgagttc accggaggca atctgagcgg ggacagtcgc 19440
gtagaattcc tctgcatatt tgtgttccaa ggagctgacg atgaatccgg acatggtaat 19500
ctctttgccg acaatcaaca taggattcta ggggggggca gtctaaggtc aggaagtcaa 19560

gacgctatgc aaggaggaca caccttaata ggcgttccgt cgccattata gcccgaaatc 19620
attccacatt cctggatgtg accgccgtta gcttagttca aatgtgcgag gacttactta 19680
cgatgaaacg cgcctttcgg ccggcagcgt cgagagcggc ttcgagcgtt tctccgccaa 19740
cattgtccca gtatctatag tcaacaggta tcgtaaactc gtgtttcttc catgcacaga 19800
gagagacgta cacatcaatg ggcccctcct gagccagaac tccagcggtt ttggtcgtct 19860
tataattgaa ggcaacgtca gcaccaatgg acttcatgaa ctcaactttg gcctccgagc 19920
cagtggatgc gatgactttt agcccatcgc gcatggctag ctggatgacc atgttgtatc 19980
gacaggcggg tgagataaag aagccttgac aagggaggac acccacgagc caacggggcc 20040
tgctcctgca gtcacgaatg cggtttcccc cttcaaaatc gttagacggt tccgtaagct 20100
ggaaatcatt ttgcaagcct tctttgcttt tgaaaactcc ttccatgcgt aaacagccgt 20160
tttaccttgc aagatttgtt agttattgac aaagttcaag tgcaaacaga aatagttacc 20220
tggcattccc gcagctccaa tgtaagtcga ccaagacaaa ctttccttgt tctcaagaac 20280
tttgtagcca tcagaagatg cgatgacgtt gtattcctga tgctccagta tcttacgatt 20340
agacatgttc ttagaccttg gttagataga gaaacggcag catacggaag acaccatata 20400
catgatctcc agctttgacg ttctcattct cagagcgaac tactccgtca ccatggctat 20460
acaatctgaa ggtgctagtg ttagaattat cacaacatcc ggaaaacaaa ggcaaaaatc 20520
acggtttgcc gacagggaag ggtggctaga cccaataaac cagtgagaat tatgacacct 20580
caaaaaccta tcacttaccg aatagctgga cttctcaggt gcgcgcattt ttcctcgcag 20640
gtaggaatca atggacagga ccaaggtctt taccaggaac cctccattga gcggcacatg 20700
atctgtgtcg atggtctgcg actcgtcgta tacgatcgtc tctccgggta caggatatcc 20760
tgtggtggaa atacttgtga gaaaaatggg tttgcaaagt cgaaccaaaa ccaaacctgt 20820
tgggacctta ttgaacttgg cgcttccgtt tctaatcact ggcatgtttg aaggcagtgg 20880
ctttgtgaga ggtcaattca ggctccttat aacattttca aggattgagg gctcagattt 20940
ataagaacca taacgccatc ggcctgcttg ccatatggca ctcgcgaata atgacctcat 21000
gacttccagt tgcaccggta agagtttcga agcaatcata tggcaggcgc tgcgaaagcc 21060
atttgtactg gaggtctctt tcaagcgata atgttcaagt caaagagcat ctttccgacc 21120
tcaaagcgtg atgtgaccgc tgtttgacag caccgttaaa gccaaacaac gcgtacgcgt 21180
caatttctga agcctgtgtg taaacagcat acgctcccaa acaggaccgg gttgggtttg 21240
gggttgagca aagccttctc cgcgcacgca tcaaacagaa aagaaaagaa tttagattgg 21300
gaaagaaata acaaccaaat acaaactaca atggcctcaa taatcaagag accaaggagc 21360
aacaggtttg gccaactcag gatttagttg ggtaaagtgg caacatatcg gcccgagaag 21420
agcccttctt ccttggcctt gatttctatt gaaaacgagg gatgagcaat gagtagcaga 21480
tgccacaaga tagaaactca cgcagggcca aatgtttcga gttgaaacga catatagcaa 21540
agttgcctgt agtgatgaat gtcagtatta gcaacatttc gaaaacggca tttatcaagc 21600

tcaccaactg cattccacag attatcgatc ccggaatcgg cccaaacgcc gttcaactcg 21660
ccctccacgg tattacccca gaatggtttc agctgggctg tgagttcctc tcccaagatt 21720
tctctattca cagtgcccgt agtaccgagt ctgagtggaa tcaatcagag gatgaaacag 21780
caagggtata aagtaagaga acctacccag tcgcaaaaag tataacttcc gcttgaagct 21840
cagagccgtc cgcgaacttg aggccagtcg gagtgatacg ttcaatcgag cttccagatt 21900
tgagcttgat cttgccatct gcgatcaatt gactagcacc agcatctgca ggcatgtcta 21960
atcaataact ctgtaaacga aatgaggagg ggaggacgta ccaaggtgga atccaccgat 22020
tctttcgcga acactgacca atgggccagc accattcata ccatcattga ggccaaatcc 22080
gaccttgcgt aggccatcca agagctctct gtcggcgtgt attatcaaca cagagtcatt 22140
gaagaatttg tggagactta cttgtccatt tcggcaataa ctttgacagc gcgttgagca 22200
agccttgctc ccacagcgaa cgggaaggca ttcaacagcc tatctgcgat gtctgtgggc 22260
ggagcattct cactgtacag agctgtaatc gcagtttaat tgagttcagc cgggaaagca 22320
gagctgaaat acctccaagc atgacttttc tagaattcgc cgttgtcatg atattggtcg 22380
agctcctttg atacatcgtc acatctacca attataaaac tttgaataag gatgcagaac 22440
aggtaagacc aaactactta ccgacaccgc tccaatagta gtcttctgca atatcatgag 22500
cagaggaacc tgcaccgaca atgaccacct tctttccaag atggtctgtt gctctgtcgt 22560
gctggataga atggaggatt tggcctttga atgtttcctg gtgtccgtat gagcgaaggt 22620
cagaggaagt tgaaaagact tggtttctta ccattccagg gatggagggg agctccgcct 22680
taccacttcc tgctcctgtc gcgaaaacca agtaggacat attcaaagtg attggtgatt 22740
gatctccacg cttgattttg atggtccatt ggccagttgc ttcctccttt ctggcgcttt 22800
caacgatgga agaagtccaa acattgagat caagagctgc tgcgtaactt tcgagccatt 22860
gtccaagctg atggagctgt aaggtcccag attcacaaaa ggaaaccatc tcaccttctt 22920
ggcaggagtg aagagtggcc aagttgaagg gaaactaaat taggtgagtc agtgcttgca 22980
ccagtcaacg aggtaatgaa catacggcat gtatggcatg tggtcaaaat ctgtaacgaa 23040
tgtgagagat gggcaacgag agtggagttt ggccttacaa atgggatcgt gtagacagag 23100
cgcatcgtag cgcgtacgcc agctatcacc aattcgggca ttcttctcga taatcaggga 23160
aggaacgccc aaagccttga gccttgcagc aacgcataag ccgctttggc ctcctccaac 23220
aatcaggact ttaggttgtg tgccctcgag ttcgacttcc ttcctcctct tctcttccca 23280
tttgccatgc cagggcgaag agtctcggag accattgatc tgctcgggga atcccttcaa 23340
gtcctcgaga ttggtgaaga cgcaatatcc cttccatcca tcggtagcag tggggaccag 23400
acgaataatg ccagaggcaa cgccaacatc ggtttcgaag tcgaagaaga acgagatgaa 23460
ggtgaagtcg gggaaggggc tttgtaggcc caagtagtgg tcttcacgca gcttgaatga 23520
cttcggcttg acagccttga gcctatcttc gaggaactcc gtgacctttg ggagcccgat 23580
gaatgtacga aagtcccacg tcaaggcgag gagatctcgc cagaaggaat ccgtgatgaa 23640
gagagatgca acttgtttgg catcaccggc ctcggctgca gaagcaaagg cagcaaacca 23700

atcctgggcg actttcttta catcaatgtc tgctggaacg ggcgctgcac ccaagcggtc 23760
aagggtggcc agaggaacac taagaagttg gtcgagttgt tcgggagtaa tcgacatggt 23820
agaaatggct gatatcgttt ggggaaagtc ggtgcgttct acataccgat tttaaatgaa 23880
ttgacgttca tagtggctgt aagtcgagac acggatatct cggtgggatt cgtaagaacg 23940
ggtcacccgg tcgctccgca gccgatagaa caagttacta cagggttcca agaggtcatc 24000
taagggcgtc gggtcacaac ccgaaggcgt ccattgatct gacattacgg aaagccagcc 24060
ggctccgtcg cgccaggact cttgcttcgc gagaacgttg gaagtgtgat cgacaacgtg 24120
ggcacttgtc gacaacgaat aggatggact ggaaggcgtg tgtaatcatg gtgaatatcc 24180
cgagttgaag ttcggttaac ttccgagggg ttgcctcttt tggcaagtct agctgttcac 24240
agtcttcaga attggggggt cagccgaagt aaattcgtgt tggcggctcc aagacgggag 24300
agagcaacgg gtatttacat ttcgcgatta gatgcatata gtgcccgcta tcaagaagat 24360
acaatgaaat gcaagctact agacgttcca gaggccatac tatgtcatta aatggccata 24420
atttcttcat ctttctcctg gaagggaatc atagtggagc tcgatgggtg atgcataaat 24480
ctctgcggga actcgtcagc gtttcatata gctctcaaaa taaacatgac ttacgagggg 24540
cgtttggtgc ctctcaagct cgtcatggtc actgacgaac tccttggtcg tgtcaatctt 24600
gatggagatc gtaccggttt ggcgctgatg aatgatgagt aactgtacta tacttttagg 24660
ttatgacaac ttaccctgga ggtgatactt gcagccttgg gatagcgaga aagggacagg 24720
ttgtcacttg tgctctggat accatcgctg gagccacgga tcattttgcg ggcgttgagt 24780
gtggcaagaa gagagttggt gtacactgtg ccaggttaat ttgagttcag gaagcaggat 24840
atctggaagc atgactcacg acgaccaatg cagaagaaaa acgcgatgta gagaaaggtt 24900
tgtccagcta cgacgatcta gggagggatc gcccgtgtaa acaattgtga agagagacga 24960
agtcttgcca cggaagatta ctcactgaga ctaacgaagc aattgcgcag agactatcat 25020
cagcgaggta tcagctttgt aacacttatc ggcggtgtta gcagattacg aacctcgtca 25080
gtaagcctgt gttgacggca aataggatct ggcatgaagt tagtgagaat ttcggtggtg 25140
cacgaaagct taagaagcat accagcttct taatgatggt gtcggaacta tgggagcgat 25200
cgtcatgcca cgtccagaag tagattgaat aatacgtacc ggcggaagcc agttcgagag 25260
ctgtacagca atgtgcaaag tataatcgcg atgaggacat cacccgccgc cgcaaaagca 25320
ttgaccatga tggacagaaa ctaatttgtg aaatgagcga tggaatcagg tctagagaga 25380
tgaggcccca ccttcagttg ttgcagttcg gcgaatgtgt gaaggtcgag gctgaagaaa 25440
taatcagtaa ccaaggaaga gccagacagc cgagccttta gaacgcacga caagacgctg 25500
aacgccagaa tgcatcctac ccgggcaagt gagtcaaccc aatatcataa ataaattagt 25560
gaaagcgcac cgaactcaag gagaacaagg agaatctgaa agaggggttt gcatcagaac 25620
aacacatccg gaatcggtgt tctaaatact gaccacaacg gcggtaagaa cgatgttgtg 25680
actgctcact gccgaggtat tagttaaaat gacctggtga aattgagcaa acgcacgaag 25740

ccaaaccctc aaagcgagaa atctgccgat gttgggatca gaaacgcaac aggacaatgg 25800
ctggatccgc gtacccttgg actagaaacg ccgtcaggcc ctgtgaagaa gtcagcagcg 25860
gtttcggcaa gtgatggaag gagcgtacat tgaaaagtac ctccaccttg tcgaggatga 25920
gcgattgttt cgggagctgg cctgcgaact taccagtaga ctcctggaag acatcgggtg 25980
acccggtaag acaattgagg gaccaaagga agtatgctat acgtaccaca ccagaacatt 26040
gagcgcatcc gggttgaagt agttcgtaat cgtgtatgtg tacactgcat aacattatca 26100
ggggggaacg taacgtagca tacagccaac ttaccagtat gagaaataag aatttggtga 26160
atcgtatcaa atatcataac ggcagcgacc tgtctaatat caggagaaaa attctccaga 26220
acaacgggaa agcgacgaac cagtgttttc agaggccacg catcctgctg gtgagtgaag 26280
taataccagg cctgaacaca ggaaactatt ccaagttaga gtacggcatg aaagagaagg 26340
aataattaat accaccgtga aggctgtgta gaaaagggtc agcggatgtc cggaaaggga 26400
taaaacaaaa gctaacttcg ctgaaacgac ggcgccaata aacgcagctc ctaagctaga 26460
accaccgccg tcagtgccac aagaccaata aagtcgggta agtgccactc acgttttgtc 26520
caaagcatga gacatattca aggtagacat gaaaaacgag agagatacag ggaatggtgg 26580
acaattatta gacaaggaag aggatttggg acgatgagtg ccagacagtc ggtgaataga 26640
ggtattataa gttattttgc tccgtgcttg aaattcttga agatttcagt cacaattcgt 26700
catgcaagga agacgcttgg ggagcctcga ggacgtgacc acgcagtcaa ggtacggaag 26760
ctgagaagca ccagatgttt tgccaggtcg tgaactactg cagaagctgg tcatccactc 26820
tcgggtctct aaactgcccg ttttatcgac agacctgttt gtaacttgaa ctgctaactt 26880
ttccctgttt tccttttttt tttagagcaa aagcctagca aacagacttt gagcgtccga 26940
agcctattta gccttacaag gtcagtcaga atcgtaaaca acccagtatt tcgaggattc 27000
aagaactctg cttcgacgac aatcatgaaa tggtaatgaa acacagatac 27050

## Description

### Field of the Invention

The present application, among others, relates to novel polypeptides having diterpene synthase activity, nucleic acid molecules encoding same, as well as to a gene cluster derived from *Clitopilus passeckerianus,* which cluster is considered to be involved in the biosynthetic pathway for producing a diterpene, more precisely pleuromutilin.

### Background of the Invention

Both terpenes and terpenoids are diverse and very large classes of mostly naturally-occurring organic chemicals that are derived from five-carbon isoprene units, which are assembled and then modified in numerous ways. Both terpenes and terpenoids may differ from one another in their carbon skeletons and in their functional groups. The majority of terpenes and terpenoids comprise one or more cyclic structures. Terpenoids are commonly also referred to as isoprenoids. Terpenes and terpenoids may be classified according to the number of terpene units (C5) which are part of their skeleton. Accordingly, monoterpenes are composed of two isoprene units (C10, skeleton of 10 carbon atoms), sesquiterpenes are composed of three isoprene units (C15, skeleton of 15 carbon atoms), diterpenes are composed of four isoprene units (C20, skeleton of 20 carbon atoms), and the like. Diterpenes and diterpenoids are commonly derived from geranylgeranyl pyrophosphate (GGPP).

Diterpene synthases are enzymes well-known to the skilled person, which usually catalyze a reaction using geranylgeranyl pyrophosphate (GGPP) as a substrate to form a diterpene or diterpenoid, respectively. Here, GGPP is usually transformed into a cyclic compound comprising one or more carbocycles. Accordingly, diterpene synthases are often referred to as diterpene cyclases. Diterpene synthases are commonly involved in a biosynthetic pathway for producing a diterpene or diterpenoid.

Gene clusters are commonly known as a group of neighbouring genes building a functional unit, e.g. by encoding polypeptides involved in one particular biosynthetic pathway, such as a pathway for producing a secondary metabolite. Secondary metabolites are substances that are usually produced by a certain organism under specific conditions. As opposed to primary metabolites, they are normally not essential for the organism that produces them.

The broad-spectrum antibiotic pleuromutilin, a terpenoid, more precisely a diterpenoid, is one example for a fungal secondary metabolite. It was first isolated in 1951 from *Pleurotus mutilus* (Fr.) Sacc. and *Pleurotus passeckerianus* Pil. Primarily, pleuromutilin inhibits the growth of Gram-positive bacteria and of *Mycoplasma.* Pleuromutilin binds to the peptidyl transferase component of the 50S subunit of ribosomes and inhibits protein synthesis in bacteria. Pleuromutilin is only one member of the group of pleuromutilin antibiotics, which further comprise a number of semisynthetic derivatives including tiamulin, which has been described to be effective for the treatment of dysentery and pneumonia in swine, valnemulin and retapamulin (cf. *Yao, 2007*):

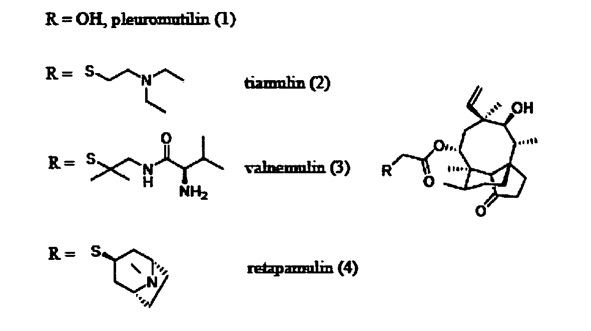

Other exemplary members of the group of pleuromutilins are azamulin and BC-3781. Further pleuromutilins are disclosed in Hunt, E., 2000, and in the references cited therein. Pleuromutilins have originally predominantly been used in veterinary medicine but they are gaining increasing interest as a human therapeutic (cf. *Hu et al., 2009).*

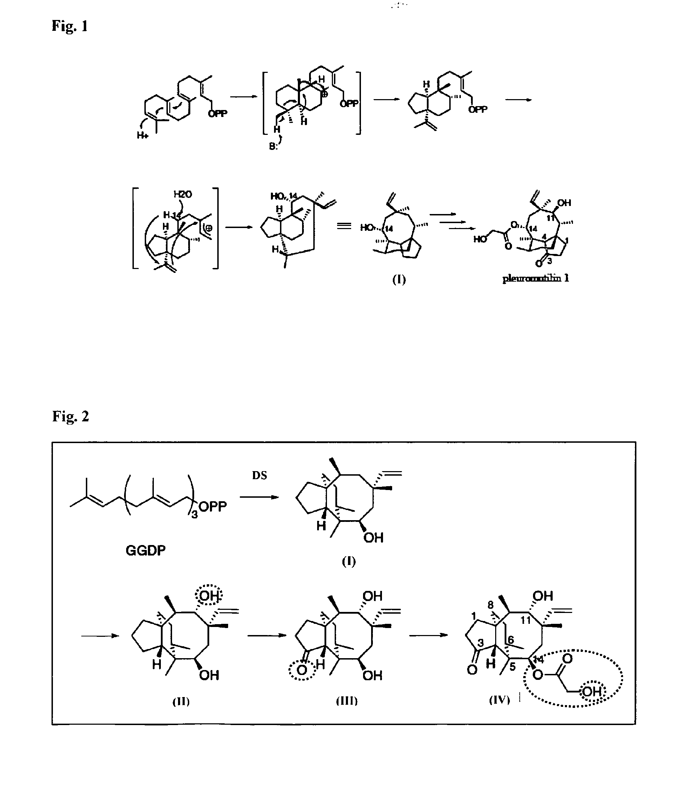

The general biosynthetic pathway for producing pleuromutilin was uncovered by isotope labeling experiments in the 1960s. An important reaction in the biosynthetic pathway for producing pleuromutilin is the reaction of geranylgeranyl pyrophosphate (GGPP), into a tricyclic pleuromutilin precursor, which reaction is thought to be catalyzed by a particular diterpene synthase (DS), a postulated enzyme commonly referred to as pleuromutilin synthase. Details of said proposed reaction (cf. *Yao, 2007*) are outlined in Figure 1.

In the subsequent reactions of the pathway for producing pleuromutilin, the actions of cytochrome P-450 enzymes (functions at C3 and C11) and an acyltransferase (functions at C14 hydroxyl) are considered necessary to complete formation of pleuromutilin (cf. *Yao, 2007*). The proposed later stages of the formation of pleuromutilin from GGPP (cf. *Tsukagoshi, et al., 2007*) are outlined in Figure 2.

Clustering of the genes responsible for biosynthesis of secondary metabolites is a common feature in most microorganisms, including Streptomycetes (*Ikeda at al., 2003; Oiynyk et al., 2007*) and fungi (*Keller et al., 2007*). Efforts have been made to identify a biosynthetic gene cluster for the formation of pleuromutilin. For example, Yao (2007) describes three distinct attempts to identify diterpene synthase genes for the formation of pleuromutilin. While Yao discovered several GGPP synthase genes (ggs genes), no diterpene synthase gene could be identified.

Accordingly, there is a need in the art for identifying nucleic acids such as a gene cluster and genes encoding a diterpene synthase, as well as for identifying polypeptides encoded thereby, as the identification of the pleuromutilin gene cluster would open the path towards a rational manipulation of pleuromutilin production. Due to increasing problems with antibiotic resistance there is a particular need in the art to provide tools for producing alternative antibiotics such as pleuromutilin or precursors or variants thereof for possible use in medicinal applications.

The present inventors have succeeded in identifying a nucleic acid sequence which is contemplated to comprise a gene cluster involved in the biosynthetic pathway for producing a diterpenoid, more precisely pleuromutilin. Said nucleic acid sequence is derived from the genome of *Clitopilus passeckerianus*, and is envisaged to comprise at least six transcriptionally co-regulated open reading frames encoding polypeptides which are thought to be involved in pleuromutilin biosynthesis. Moreover, the present inventors have further succeeded in identifying, as part of this gene cluster, a polypeptide which is a new diterpene synthase. It appears to be the only diterpene synthase in the genome of *Clitopilus passeckerianus* and is thus envisaged to be the long-sought pleuromutilin synthase. Said diterpene synthase gene is in close proximity, and in fact co-regulated, with a putative geranylgeranyl diphosphate synthase gene, more than one cytochrome p450 enzyme-encoding genes and a putative acyltransferase-encoding gene, making it plausible and credible that it is involved in the biosynthetic pathway for producing pleuromutilin of *Clitopilus passeckerianus.* With the discovery of the biosynthetic gene cluster for pleuromutilin synthesis, the present invention is moreover believed to provide valuable tools for producing diterpenoids, particularly pleuromutilin and pleuromutilin precursors, and to provide a basis for synthesizing novel pleuromutilin antibiotics or pleuromutilin analogues, e.g. by starting from the product of the diterpene synthases disclosed herein.

### Summary of the Invention

The present invention, among others, relates to a polypeptide having diterpene synthase activity as defined in the appended claims.

In another aspect, the invention relates to corresponding nucleic acid molecules as defined in the appended claims. A particular nucleic acid molecule is one which is envisaged to be a gene cluster involved in the biosynthetic pathway for producing a diterpene, more precisely pleuromutilin.

According to further aspects, the invention relates to subject-matter such as methods and uses as defined in the claims and described hereinbelow.

The aspects and particular embodiments of the invention are set forth in the claims and in the following disclosure.

### Detailed Description of the Invention

### DEFINITIONS

As used herein, technical terms generally have the common meaning as understood in the art, unless defined otherwise hereinbelow.

Generally, as used herein, the term "comprising" includes the meanings "having" and "consisting of'.

Polypeptides are usually linear amino acid polymers, wherein the individual amino acids are linked to one another via peptide bonds. Polypeptides which contain a low percentage, e.g. less than 10%, 5%, 3% or even less than 1%, such as from greater than 0% to 1% of modified or non-natural amino acids are also envisaged. Preferred polypeptides, however, do not contain non-natural amino acids and modifications are only naturally occurring modifications, such as glycosylation, ubiquitination or the like. As is well known to the skilled person, a polypeptide may, for example, be modified by the phosphorylation of serine, threonine or tyrosine residue(s) by phosphorylation, or by glycosylation of e.g. asparagine residue(s) or serine residue(s). Modified polypeptides are likewise envisaged herein as comprised by polypeptides.

The term "wild type" when used herein in connection with a polypeptide refers to a naturally occurring, non-mutated form of such polypeptide.

Polypeptides of the invention particularly include non-natural polypeptides. A "non-natural polypeptide" as referred to herein does not occur as such in nature, but can be, and in particular has been, produced by laboratory manipulations, such as genetic engineering techniques or chemical coupling of other molecules to a polypeptide. Examples of modified polypeptides are polypeptides carrying in particular additions, substitutions, deletions, truncations produced by genetic engineering techniques. Preferably, a "non-natural polypeptide" is a polypeptide which is not encoded as such by the genome of a naturally occurring species, in particular a polypeptide that is not identical to any one of those polypeptides of the gene bank database as of the filing date of this application with a naturally occurring species identified as its source. In certain preferred general embodiments, the polypeptides referred to herein are non-natural polypeptides.

A "polypeptide" as used herein particularly relates to a molecule comprising more than 30, and in particular at least 35, 40, 45 or at least 50 or 100 amino acids, but not more than 10,000, in particular not more than 9,000, 8,000, 7,000, 6,000 or 5,000 amino acids.

A preferred diterpene synthase of the present invention comprises from about 500 to about 1,500 amino acids, particularly from about 800 to about 1,200 amino acids, especially from about 900 to about 1,100 amino acids, more particularly from about 900 to about 1,020 amino acids. Preferably, the molecular weight of this polypeptide is between 90 kDa and 140 kDa, particularly between 100 kDa and 130 kDa, especially between 105 kDa und 120 kDa.

An "isolated" polypeptide is meant to be a polypeptide that is present in an environment which differs from the environment in which it is naturally present or in which it was produced. Preferably, an isolated polypeptide is at least 0.01%, particularly at least 0.1%, more particularly at least 1%, more particularly at least 10%, such as at least 20%, 30%, 40%, 50%, 60%, 70%, 80%, more particularly at least 90%, such as at least 95%, 96%, 97%, 98%, 99% pure as determined according to European Pharmacopoeia 6.6, hereby incorporated by reference, by denaturing, discontinuous SDS PAGE with a 12% resolving gel and Coomassie staining. Preferably, an isolated polypeptide is not contained in an organism or a cell. In a preferred embodiment, the isolated polypeptide is not associated with any polypeptide component of its natural environment or of the production environment.

In case of polypeptides, preferably, the nature of amino acid residue changes by which the polypeptide having at least X% identity to a reference sequence differs from said reference sequence is a semi-conservative and more preferably a conservative amino acid residue exchange.

| Amino acid | Conservative exchange | Semi-conservative exchange |
|---|---|---|
| A | G;S;T | N;V;C |
| C | A;V;L | M; I; F; G |
| D | E; N; Q | A; S; T; K; R; H |
| E | D; Q; N | A; S; T; K; R; H |
| F | W; Y; L; M; H | I;V;A |
| G | A | S; N; T; D; E; N; Q; |
| H | Y; F; K; R | L; M; A |
| I | V;L;M;A | F; Y; W; G |
| K | R; H | D; E; N; Q; S; T; A |
| L | M; I; V; A | F; Y; W; H; C |
| M | L;I;V;A | F; Y; W; C; |
| N | Q | D; E; S; T; A; G; K; R |
| P | V; I | L; A; M; W; Y; S; T; C; F |
| Q | N | D; E; A; S; T; L; M; K; R |
| R | K; H | N; Q; S; T; D; E; A |
| S | ; T; G; N | D;E;R;K |
| T | A; S; G; N; V | D; E; R; K; I |
| V | A; L; I | M; T; C; N |
| w | F; Y; H | L; M; I; V; C |
| Y | F; W; H | L; M; I; V; C |

Changing from A, F, H, I, L, M, P, V, W or Y to C is semi-conservative if the new cysteine remains as a free thiol. Changing from M to E, R or K is semi-conservative if the ionic tip of the new side group can reach the protein surface while the methylene groups make hydrophobic contacts. Changing from P to one of K, R, E or D is semi-conservative, if the side group is on the surface of the protein. Furthermore, the skilled person will appreciate that Glycines at sterically demanding positions should not be substituted and that P should not be introduced into parts of the protein which have an alpha-helical or a beta sheet structure.

Nucleic acid molecules are well known to the skilled person. Preferably, a "nucleic acid molecule" as used herein relates to a nucleic acid polymer consisting of nucleotide monomers, such as a DNA or RNA.

An "isolated" nucleic acid molecule is meant to be a nucleic acid molecule that is present in an environment which differs from the environment in which it is naturally present or in which it was produced. Particularly, it is separated from at least one nucleic acid molecule with which it is ordinarily associated its natural environment or the production environment, respectively. Preferably, an isolated nucleic acid molecule is not contained in an organism or a cell. In a preferred embodiment, the isolated nucleic acid molecule is not associated with any nucleic acid molecule associated with its natural environment or the production environment.

The term "wild type" when used herein in connection with a nucleic acid molecule refers to a naturally occurring, non-mutated form of such nucleic acid molecule.

Nucleic acid molecules of the invention particularly include non-natural nucleic acid molecules. A "non-natural nucleic acid molecule" as referred to herein does not occur as such in nature, but can be, and in particular has been, produced by laboratory manipulations, such as genetic engineering techniques or chemical coupling of other molecules to a polypeptide. Examples of modified nucleic acid molecule are nucleic acid molecules carrying in particular additions, substitutions, deletions, truncations produced by genetic engineering techniques. Preferably, a "non-natural nucleic acid molecule" is a nucleic acid molecule which is not identical to one of those nucleic acid molecules of the gene bank database as of the filing date of this application with a naturally occurring species identified as its source. In certain preferred general embodiments, the nucleic acid molecules referred to herein are non-natural nucleic acid molecules.

A "partial sequence of a sequence x" generally refers to a sequence comprising one or more contiguous sections of the sequence x. In certain preferred embodiments, it comprises one contiguous section of the sequence x. Preferably, in case of a nucleotide sequence, the partial sequence comprises one or more, particularly one open reading frame. More preferably, the partial sequence is a sequence encoding a polypeptide, particularly a coding sequence not comprising introns (cds).

As used herein, a nucleotide sequence is said to be "of *species* origin" if it is contained anywhere within the genome of said *species.* Said nucleotide sequence may or may not be part of a gene.

The determination of corresponding positions in related sequences as well as the calculation of percentages of identity can be performed with the help of well known alignment algorithms and optionally computer programs using these algorithms. The identities in this patent application have been calculated by aligning sequences with the freeware program ClustalX (Version 1.83) with default parameters and subsequent counting of identical residues by hand. Percentage identity (PID) was then calculated by dividing the number of identities by length of the shortest sequence. Default settings for, e.g., pairwise alignment (slow-accurate) are: gap opening parameter: 10.00; gap extension parameter 0.10; Protein weight matrix: Gonnet 250; DNA weight matrix IUB. The ClustalX program is described in detail in *Thompson et al., 1997.*

Accordingly, as used herein, a nucleotide or amino acid sequence is said to have "X % sequence identity" to a given sequence if an alignment with the freeware program ClustalX (Version 1.83) with default parameters, a subsequent determination of identical residues, such as by counting by hand, and a subsequent calculation of the percentage identity (PID) by dividing the number of identities by the length of the shortest sequence gives "X % sequence identity".

A "vector" as used herein particularly relates to DNA elements that may be used for transferring and introducing foreign DNA sequence into a host. Vectors include, but are not limited to plasmids, viruses, phages, and cosmids. In a preferred embodiment, the vector is an "expression vector". As is known to the skilled person, an expression vector is designed such that a coding sequence inserted at a particular site can be transcribed and translated into a polypeptide.

A "host" as used herein is preferably selected from a cell, tissue and non-human organism. In preferred embodiments, the host is a fungal host, more particularly a fungus from the division basidomycota, even more particularly from the order agaricales, even more particularly from the family entolomataceae. In a preferred embodiment, the host is from the genus *Clitopilus* and is particularly selected from the group consisting of *Clitopilus scyphoides, Clitopilus prunulus, Clitopilus hobsonii, Clitopilus pseudo-pinsitus, Clitopilus pinsitus* and *Clitopilus passeckerianus,* more particularly selected from *Clitopilus pinsitus* and *Clitopilus passeckerianus.* In another preferred embodiment, the host is from the genus *Pleurotus.* Preferably, the host is selected from a cell, tissue and non-human organism which is known to be capable of producing pleuromutilin, such as from the group consisting of *Omphalina mutila, Clitopilus scyphoides, Clitopilus prunulus, Clitopilus hobsonii, Clitopilus pseudo-pinsitus, Clitopilus pinsitus* and *Clitopilus passeckerianus.* Particularly, the host is *Clitopilus pinsitus* or *Clitopilus passeckerianus.*

A "cell" as used herein is not particularly limited. Preferably, said cell is one in which a vector of the invention can replicate. Preferably, said cell is one in which a coding sequence inserted in a vector is transcribed and translated into a polypeptide. Preferably, said cell is not a totipotent stem cell. In some embodiments, the tissue is a non-animal cell. In preferred embodiments, said cell is from a fungus as described above.

A "tissue" as used herein is not particularly limited. Preferably, said tissue is one in which a vector of the invention can replicate. Preferably, said tissue is one in which a coding sequence inserted in a vector is transcribed and translated into a polypeptide. In some embodiments, the tissue is a non-animal tissue. In preferred embodiments, said tissue is from a fungus as described above. A tissue may be an organ. One preferred fungal tissue is a mycelium.

A "non-human organism" as used herein is not particularly limited. Preferably, said non-human organism is one in which a vector of the invention can replicate. Preferably, said non-human organism is one in which a coding sequence inserted in a vector is transcribed and translated into a polypeptide. In preferred embodiments, the non-human organism is a non-animal organism. In preferred embodiments, the non-human organism is a fungal organism, more particularly a fungus from the division basidomycota, even more particularly from the order agaricales, even more particularly from the family entolomataceae. In a preferred embodiment, the fungal organism is a fungus from the genus *Clitopilus* or from the genus *Pleurotus.*

Both the term "wild type host" and the term "naturally occurring host" as used herein refer to a host that occurs in nature. Particularly, such wild type host is any cell, tissue or non-human organism that is or is part of a naturally occurring species that is part of database as of the filing date of this application.

The term "non-naturally occurring host" refer to a host that does not occur in nature. In preferred embodiments, said host is non-naturally occurring due to the introduction therein of e.g. a nucleic acid molecule or vector of the present invention. In preferred embodiments, said host is non-naturally occurring due to the modification therein of a nucleic acid molecule having a sequence of a nucleic acid molecule described herein.

A "corresponding naturally occurring host" of a non-naturally occurring host refers to a corresponding host that occurs in nature and does not show the non-natural feature of the corresponding non-naturally occurring host. Generally, the corresponding naturally occurring host may be a host capable or incapable of producing pleuromutilin. Generally, the corresponding naturally occurring host may be a host capable or incapable of producing a pleuromutilin precursor, particularly the compound according to formula (I). As used herein, a "host incapable of producing pleuromutilin" refers to a host which produces no detectable amounts of pleuromutilin. Accordingly, as used herein, a "host capable of producing pleuromutilin" refers to a host which produces detectable amounts of pleuromutilin in accordance with one of the above methods.

Whether or not a given host produces detectable amounts of pleuromutilin may easily be determined by extracting a homogenized sample of cells of said host with a suitable solvent and assaying for the presence or absence of pleuromutilin in said extract e.g. by a method involving HPLC (e.g. as described in *Hartley el al., 2009),* MS (e.g. as described in *Tsukagoshi, el al., 2007*) or NMR or MS (e.g. as described in *Yao, 2007*), preferably while also using a sample containing pleuromutilin as a positive control.

As to a fungal host, a "host incapable of producing pleuromutilin" preferably means a host showing a negative result for pleuromutilin production as determined by the "assessment of pleuromutilin production" described on page 26 of *Hartley et al., 2009.* As to a non-fungal host, a "host incapable of producing pleuromutilin" preferably means a host showing a negative result for pleuromutilin production when subjecting an extract of a homogenized sample of cells of said host to the HPLC analysis described in the "assessment of pleuromutilin production" on page 26 of *Hartley et al., 2009.*

Accordingly, a fungal "host capable of producing pleuromutilin" preferably means a host showing a positive result for pleuromutilin production as determined by the "assessment of pleuromutilin production" described on page 26 of *Hartley et al., 2009,* i.e. the observation of a "pleuromutilin peak". As to a non-fungal host, a "host capable of producing pleuromutilin" preferably means a host showing a positive result for pleuromutilin production when subjecting an extract of a homogenized sample of cells of said host to the HPLC analysis described in the "assessment of pleuromutilin production" on page 26 of *Hartley et al., 2009.*

Non-limiting exemplary fungal strains that are capable of producing pleuromutilin are e.g. disclosed in *Hartley et al., 2009.* These include, but are not limited to strains of *Omphalina mutila*, *Clitopilus hobsonii*, *Clitopiluspinsitus* and *Clitopilus passeckerianus*.

A gene cluster may commonly refer to a group of genes building a functional unit. As used herein, a "gene cluster" is a nucleic acid comprising sequences encoding for polypeptides that are involved together in at least one biosynthetic pathway, preferably in one biosynthetic pathway. Particularly, said sequences are adjacent. Preferably, said sequences directly follow each other, wherein they are separated by varying amounts of non-coding DNA. Preferably, a gene cluster of the invention has a size from 10kb to 50kb, more preferably from 14kb to 40kb, even more preferably from 15kb to 35kb, even more preferably from 20kb to 30kb, particularly from 23kb to 28kb.

Terpenes and terpenoids are well-known to the skilled person as already described in the introduction herein. As used herein, no distinction is made between a "terpene" and a "terpenoid". Accordingly, as used herein, no distinction is made between a "diterpene" and a "diterpenoid". A diterpene may comprise 15 carbon atoms. Preferably, a diterpene comprises one or more cyclic structural elements. A diterpene may also comprise more or less than 15 carbon atoms. Exemplary diterpenes / diterpenoids include but are not limited to aconitine, cafestol, cembrene, kahweol, phytane, retinol, stevioside, and taxadiene. Preferred examples of diterpenes / diterpenoids include, but are not limited to pleuromutilin, tiamulin, valnemulin, retapamulin, wherein pleuromutilin is particularly preferred.

Pleuromutilin is well-known to the skilled person as a fused 5-6-8 tricyclic diterpenoid. It may be depicted as "pleuromutilin 1" in Figure 1.

A "pleuromutilin antibiotic" as used herein refers to any antibacterial agent of the pleuromutilin family of antibiotics. Pleuromutilin antibiotics include, but are not limited to pleuromutilin, tiamulin, valnemulin, retapamulin, azamulin, BC-3781 and the ones disclosed in *Hunt, E., 2000.* Herein, a pleuromutilin antibiotic is preferably pleuromutilin or a pleuromutilin derivative. Pleuromutilin derivatives are not particularly limited and include any conceivable pleuromutilin derivative.

A "pleuromutilin precursor" as used herein refers to any intermediate compound of the biosynthetic pathway for producing pleuromutilin. A pleuromutilin precursor may or may not be the product of a geranylgeranyl pyrophosphate synthase and hence may or may not be geranylgeranyl pyrophosphate. Preferably, a "pleuromutilin precursor" refers to any intermediate compound of the biosynthetic pathway for producing pleuromutilin downstream of geranylgeranyl pyrophosphate. A preferred "pleuromutilin precursor" is the product of a diterpene synthase, particularly a pleuromutilin synthase, as disclosed herein. A preferred pleuromutilin precursor herein is a pleuromutilin precursor depicted in Figure 1. An especially preferred pleuromutilin precursor herein is the compound according to formula (I) as depicted in Figure 1.

A "pleuromutilin precursor" may also be the product of any other reaction catalyzed by a polypeptide involved in the biosynthetic pathway for producing pleuromutilin. Other preferred pleuromutilin precursors particularly include the compounds (II) and (III) depicted in Figure 2 herein.

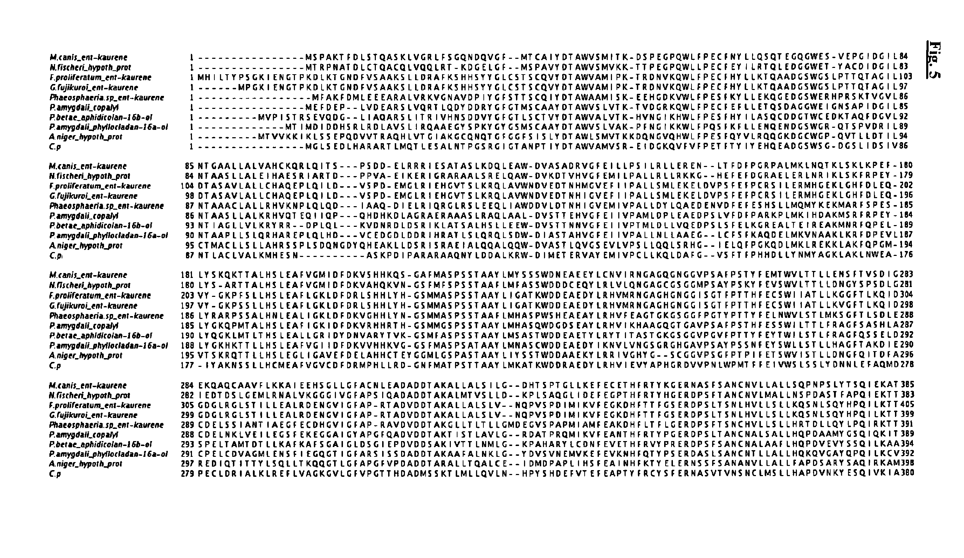

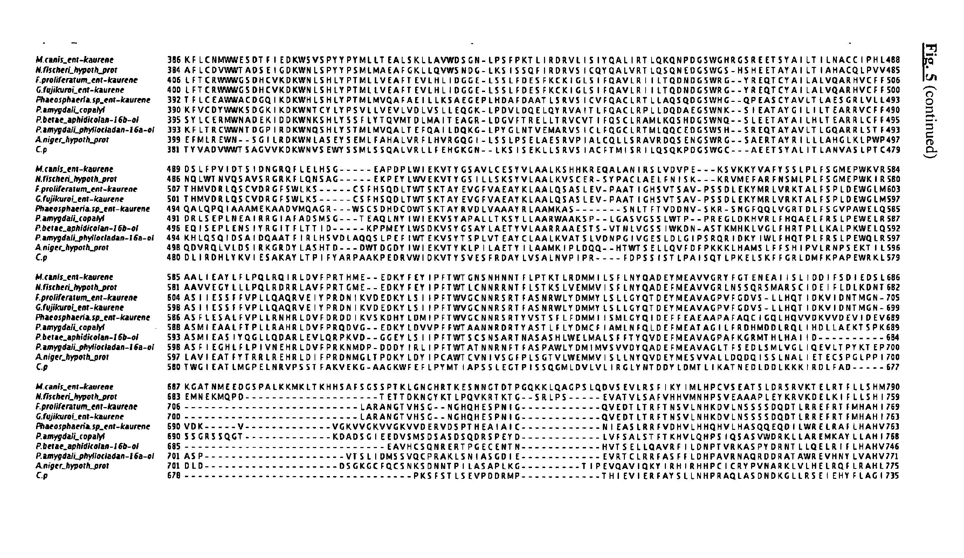

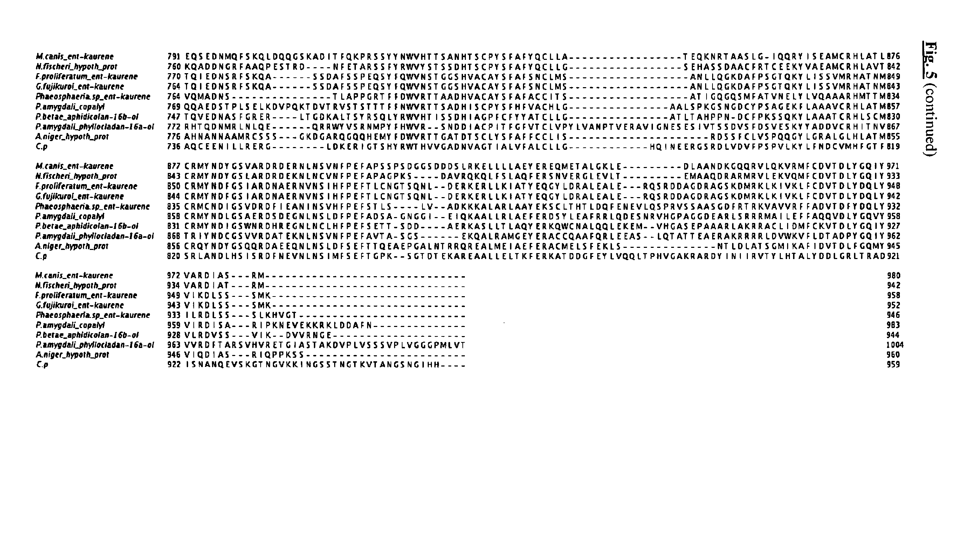

In addition, "diterpene synthase" and "diterpenoid synthase" are used interchangeably herein. In addition, a "diterpene synthase" may also be referred to as a "diterpene cyclase". Diterpene synthases are well-known to the skilled person as also described hereinabove. That is, the skilled person will readily be in the position to recognize a diterpene synthase, such as by way of its homology to known diterpene synthases. To this end, the skilled person may suitably employ computational methods such as an alignment, e.g. an alignment similar to the one disclosed herein in Figure 5. Herein, a "diterpene synthase" particularly refers to a polypeptide capable of catalyzing a conversion of geranylgeranyl pyrophosphate into a molecule containing one or more cyclic structures. Preferably, said molecule is a pleuromutilin precursor. Accordingly, in preferred embodiments, the diterpene synthase is a pleuromutilin synthase. Herein, a "pleuromutilin synthase" particularly refers to a polypeptide capable of catalyzing the conversion of geranylgeranyl pyrophosphate into a molecule containing one or more cyclic structures which molecule is a pleuromutilin precursor, particularly a pleuromutilin precursor depicted in Figure 1, especially a compound according to formula (I).

As used herein, a "polypeptide having diterpene synthase activity" preferably refers to a polypeptide that is capable of catalyzing a conversion of geranylgeranyl pyrophosphate into a molecule containing one or more cyclic structures. Said polypeptide may or may not have further activities and diterpene synthase activity may not be the main activity of said polypeptide. Diterpene synthase activity may, for example, be detected and/or measured by incubating a sample containing the polypeptide, preferably the purified polypeptide, with GGDP in a suitable buffer and detecting the production of a product of a diterpene synthase (e.g. a diterpene molecule containing one or more cyclic structures), optionally in connection with the consumption of GGDP, by a suitable method e.g. involving MS or GC/MS. One particular exemplary enzyme assay for the activity of a diterpene synthase is disclosed in *Toyomasu et al., 2000.* Said assay basically involves the detection of the product of a diterpene synthase by means of GC-MS. Exemplary assays for diterpene synthase activity are also disclosed in *Kawaide et al., 1997,* one particular assay involving detection of GGDP consumption and product formation by employing [³H]GGDP, another one being suitable to identify metabolites by GC-MS. If necessary for a given assay, a diterpene synthase may be expressed e.g. in a fungal host or in *E. coli* and may optionally be purified therefrom.

As used herein, a "polypeptide having pleuromutilin synthase activity" preferably refers to a polypeptide that is capable of catalyzing the conversion of geranylgeranyl pyrophosphate into a pleuromutilin precursor, particularly into a pleuromutilin precursor depicted in Figure 1, especially into a compound according to formula (I). Said conversion may comprise one or more steps. Said polypeptide may or may not have further activities and pleuromutilin synthase activity may not be the main activity of said polypeptide. Pleuromutilin synthase activity may, for example, be detected and/or measured by incubating a sample containing the polypeptide, preferably the purified polypeptide, with GGDP in a suitable buffer and assaying for the production of a pleuromutilin precursor, particularly a compound according to formula (I). The detection of the production of a pleuromutilin precursor, particularly of a compound depicted in Figure 1, especially of a compound according to formula (I) will easily be achievable by the skilled person on basis of the disclosure herein including the teachings of the references disclosed herein; e.g. by a method involving MS or NMR. In preferred embodiments, a pleuromutilin synthase of the invention has at least 5%, 10% or 20%, preferably at least 30% or 40%, such as at least 50%, or at least 60%, 70%, 80%, particularly at least 90% or 95%, especially at least 100%, such as at least 125%, 150% or 175% of the activity of the pleuromutilin synthase of SEQ ID NO: 9, particularly at least 2-fold or 5-fold, at least 10-fold or 25-fold, such as from 50- to 100-fold the activity of the pleuromutilin synthase of SEQ ID NO: 9.

The terms "geranylgeranyl pyrophosphate", which is abbreviated as GGPP, and "geranylgeranyl diphosphate", which is abbreviated as GGDP, are used interchangeably herein.

As used herein, the "biosynthetic pathway for producing a diterpene" refers to the biosynthetic pathway leading to a diterpene such as pleuromutilin. Said pathway at least comprises a step of converting GGPP into a diterpene or diterpene precursor and may further comprise a step of the formation of GGPP and/or one or more later steps of the synthesis of the diterpene.

As used herein, the "biosynthetic pathway for producing pleuromutilin" refers to the biosynthetic pathway leading to a pleuromutilin antibiotic such as pleuromutilin. In preferred embodiments, it refers to the biosynthetic pathway commencing with a step of the formation of geranylgeranyl pyrophosphate (GGPP), which is preferably formed from farnesyl pyrophosphate (FPP), and leading to pleuromutilin. In other preferred embodiments, it refers to the biosynthetic pathway leading from geranylgeranyl pyrophosphate (GGPP) to pleuromutilin. Said pathway has been described to include a reaction from GGPP into a pleuromutilin precursor, particularly a compound according to formula (I), as well as further reaction steps, such as those depicted in Figure 2 (cf. *Yao*, 2007), which may also be referred to herein as later stages of the biosynthetic pathway. Common precursors of GGPP are isopentenyl diphosphate (IPP) and dimethylallyl diphosphate (DMAPP). As is known to the skilled person geranyl pyrophosphate (GPP) may be synthesized from IPP and DMAPP, and FPP may be synthesized from IPP and GPP.

In fungi and animals, isopentenyl diphosphate (IPP) and dimethylallyl diphosphate (DMAPP), have been described to be synthesized via the so-called the mevalonate (MVA) pathway (cf. *Dewick, 2002*)*.* In the latter pathway, two molecules of acetyl-coenzyme A (acetyl-CoA) are reacted to give acetoacetyl-CoA. Acetoacetyl-CoA is reacted with a further molecule of acetyl-CoA to form 3-hydroxy-3-methylglutary-CoA (HMG-CoA). This reaction is catalyzed by HMG-CoA synthase. HMG-CoA reductase then converts HMG-CoA to mevalonic acid (MVA). The six-carbon atoms containing mevalonic acid is then transformed into the five-carbon atoms containing isopentenyl-5-pyrophosphate (IPP) by two phosphorylation reactions to yield mevalonate-5-phosphate and mevalonate-5-pyrophosphate, respectively, followed by a decarboxylation reaction to yield IPP. Isopentenyl diphosphate is isomerized by IPP isomerase to generate dimethylallyl diphosphate (DMAPP).

An alternative metabolic pathway leading to the formation of isopentenyl pyrophosphate (IPP) and dimethylallyl pyrophosphate (DMAPP) is the so-called non-mevalonate pathway or methyl erythritol phosphate pathway (MEP pathway), which starts from pyruvate and glycerinaldehyde-3-phosphate, and which is e.g. used by most bacteria and by green algae (cf. *Kuzuyama*, *2002*). Higher plants and red algae may employ either the MVA or the MEP pathway.

In fungi, the full biosynthetic pathway for producing pleuromutilin has been described to include the reaction from FPP to geranylgeranyl pyrophosphate, which reaction is catalyzed by a particular prenyltransferase, a so-called GGPP synthase (ggs), which extends FPP with one IPP molecule. Subsequently, GGPP is reacted into a cyclic pleuromutilin precursor by a particular diterpene synthase which is also called pleuromutilin synthase. The thus obtained pleuromutilin precursor is then converted into pleuromutilin in the so-called later stages of pleuromutilin synthesis. Later stages of pleuromutilin synthesis are believed to include the actions of an acyltransferase and cytochrome P-450 enzymes.

As used herein, the expression of a polypeptide being "involved in the biosynthetic pathway for producing pleuromutilin" particularly refers to a polypeptide which is capable of catalyzing at least one of the reactions of the biosynthetic pathway for producing a pleuromutilin antibiotic, particularly pleuromutilin, and especially the pathway as defined hereinabove. Preferably, said polypeptide catalyzes at least one reaction in the conversion of FPP to a pleuromutilin antibiotic such as pleuromutilin. Preferably, said polypeptide catalyzes at least one reaction in the conversion of GGPP to a pleuromutilin antibiotic such as pleuromutilin. In preferred embodiments, the expression particularly refers to a polypeptide which is capable of catalyzing at least one of the reactions depicted in Figure 2. Most preferably, said polypeptide is essential for at least one reaction in the overall conversion of GGPP to pleuromutilin.

A preferred polypeptide involved in the biosynthetic pathway for producing pleuromutilin is a diterpene synthase, such as those of the present invention. A particularly preferred polypeptide involved in the biosynthetic pathway for producing pleuromutilin is a pleuromutilin synthase, which is capable of catalyzing the conversion from GGPP to the tricyclic intermediate of formula (I). Most preferably, said pleuromutilin synthase is essential for the conversion from GGPP to the tricyclic intermediate of formula (I).

As used herein, the expression of a nucleic acid, gene or gene cluster being "involved in the biosynthetic pathway for producing pleuromutilin" particularly refers to a nucleic acid, gene or gene cluster encoding one or more polypeptides that is/are involved in the biosynthetic pathway for producing a pleuromutilin antibiotic such as pleuromutilin. The latter nucleic acid, gene or gene cluster are also said to be directly involved in the biosynthetic pathway for producing pleuromutilin. Nucleic acids, genes or gene clusters involved in the biosynthetic pathway for producing pleuromutilin also include ones, such as promoters or enhancers, that are indirectly involved in the biosynthetic pathway for producing pleuromutilin by acting on directly involved nucleic acids, genes or gene clusters. Preferably, a nucleic acid, gene or gene cluster involved in the biosynthetic pathway for producing pleuromutilin encodes a preferred polypeptide involved in the biosynthetic pathway for producing pleuromutilin as described herein.

As used herein "stringent hybridization conditions" and "stringent conditions" refers to conditions under which a nucleic acid molecule will hybridize to its target and to a minimal number of other sequences only.

Stringent conditions within the meaning of this invention include pre-washing in a solution of 6x SSC, 0.2% SDS at 22°C; hybridizing at 65° C, in 6x SSC, 0.2% SDS overnight; followed by four washes at 65°C of 30 minutes each, two in 1x SSC, 0.1% SDS and two in 0.2x SSC, 0.1% SDS.

A "method for the fermentative production" of a polypeptide is well-known to a skilled person. As used herein, it is a method involving a living system, e.g. an organism such as bacterial or a fungal organism, or a cell (culture), or a tissue (culture). The living system may be any of the hosts disclosed herein.

A "method for the fermentative production" may be characterized by only including production steps involving a living system. A "method for the fermentative production" may also be characterized by both including production steps involving a living system and production steps not involving a living system. The latter method may also be referred to herein as a "method for semisynthetic production".

As used herein, a "method for the synthetic production" does not involve a living system, e.g. an organism such as bacterial or a fungal organism, or a cell (culture), or a tissue (culture). Preferably, such method employs chemical means or an isolated polypeptide, such as the polypeptides disclosed herein.

A "method of the production of a polypeptide" as used herein is not particularly limited. It comprises a method for the fermentative production of a polypeptide, a method for the synthetic production of a polypeptide, and any combination of the latter methods.

### SHORT DESCRIPTION OF THE FIGURES

FIG. 1 illustrates the proposed details of the reaction catalyzed by pleuromutilin synthase leading from GGDP to a compound according to formula (I) (cf. *Yao, 2007*).
FIG. 2 illustrates the stages of the biosynthetic pathway for producing pleuromutilin which lead from GGDP to pleuromutilin, i.e. the reaction catalyzed by the diterpene synthase as well as later stages of pleuromutilin synthesis (modified from *Tsukagoshi, et al., 2007*).
FIG. 3 shows the polypeptide (SEQ ID NO: 9) as well as the coding nucleic acid sequence (SEQ ID NO: 8) of the diterpene synthase (gene) involved in the biosynthetic pathway for producing pleuromutilin. Also shown is the corresponding genomic nucleic acid sequence (SEQ ID NO: 13), wherein introns are depicted in lower case as well as a sequence comprising the putative gene cluster from *Clitopilus passeckerianus* (SEQ ID NO: 15).
FIG. 4 depicts a proposed outline of a part of the gene cluster identified by the present inventors.
FIG. 5 depicts an amino acid alignment of the preferred diterpene synthase sequence of the invention (designated as "*C.p*") with known and putative diterpene synthase sequences (*Phomopsis amygdali* copalyl diphosphate synthase (BAG30962), *Phomopsis amygdali* phyllocladan-16a-ol synthase (BAG30961), *Phoma betae* aphidicolan-16b-ol synthase (BAB62102), *Phaeosphaeria* sp. ent-kaurene synthase (BAA22426), *Fusarium proliferatum* ent-kaurene synthase (ABC46413), *Gibberella fujikuroi* ent-kaurene synthase (BAA84917), *Microsporum canis* ent-kaurene synthase (EEQ29644), *Aspergillus niger* hypothetical protein An18g02710 (XP_001398730), *Neosartorya fischeri* hypothetical protein NFIA_009790 (XP_001264196))

### DESCRIPTION OF THE SEQUENCES

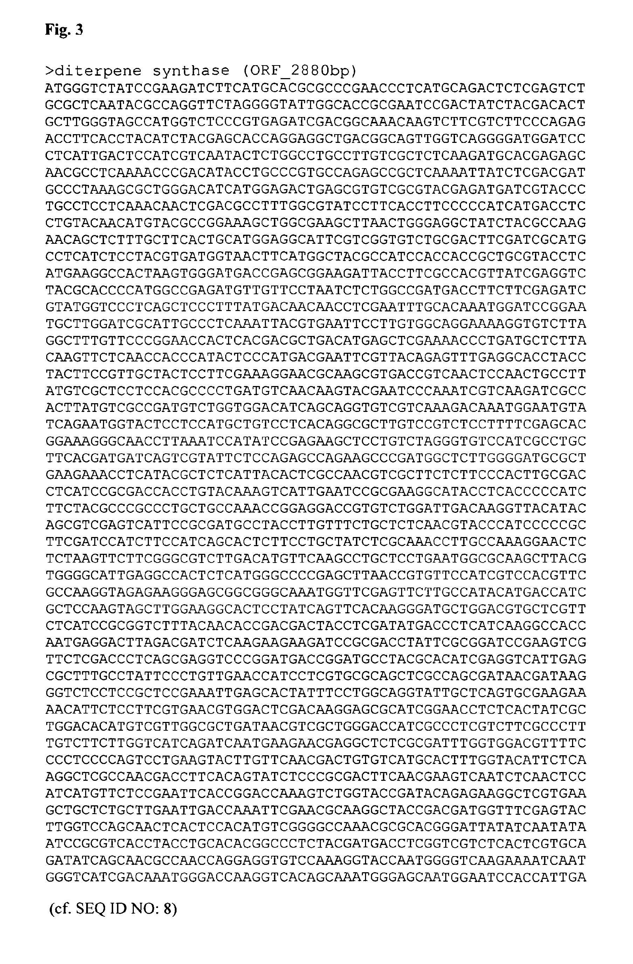

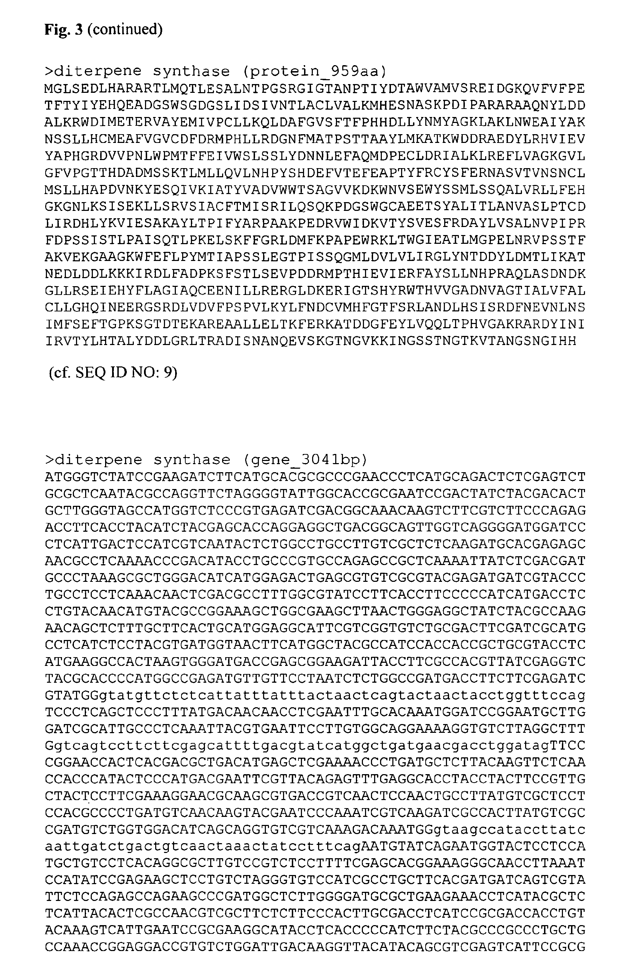

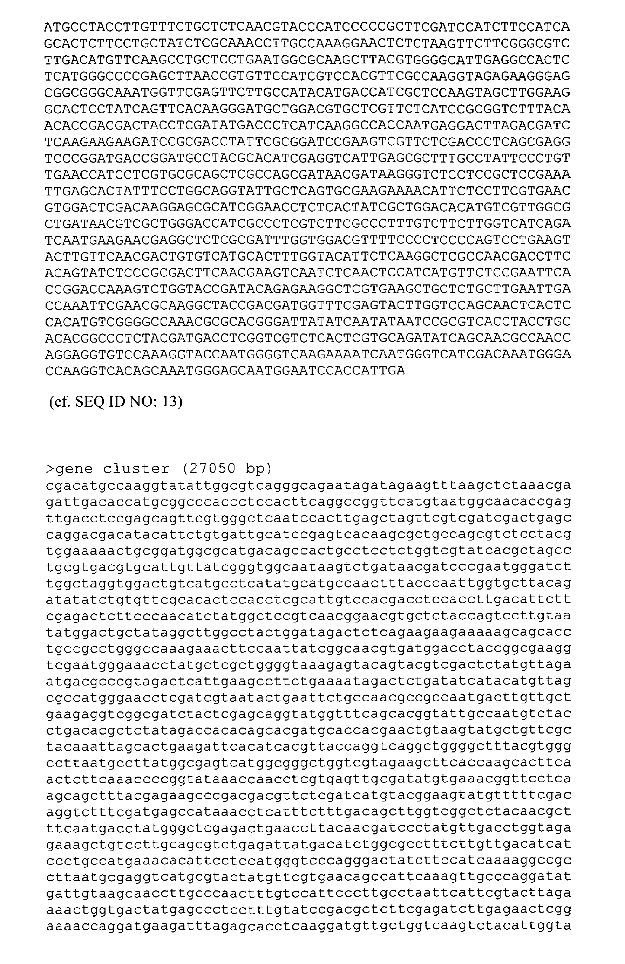

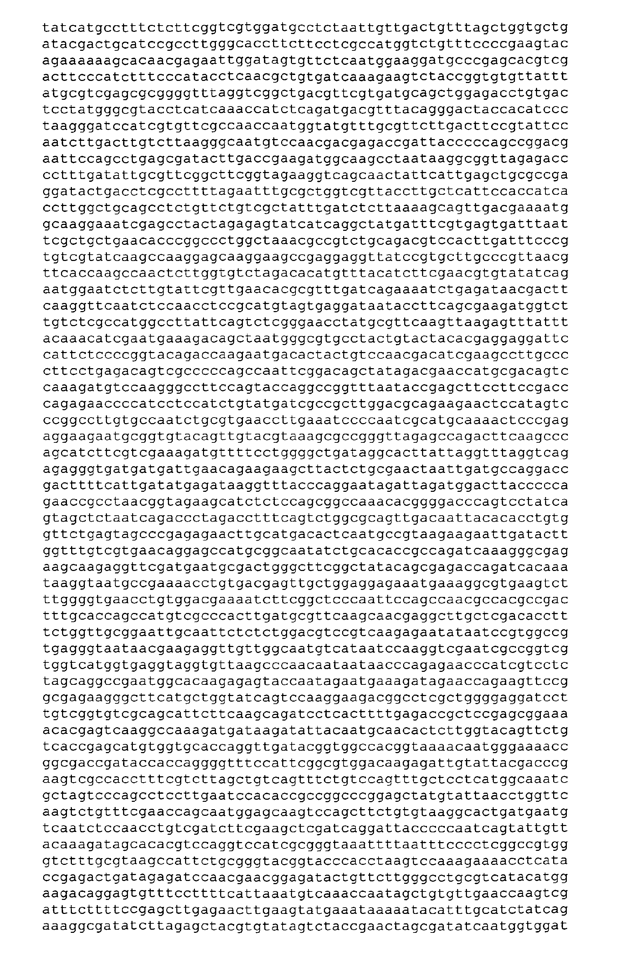

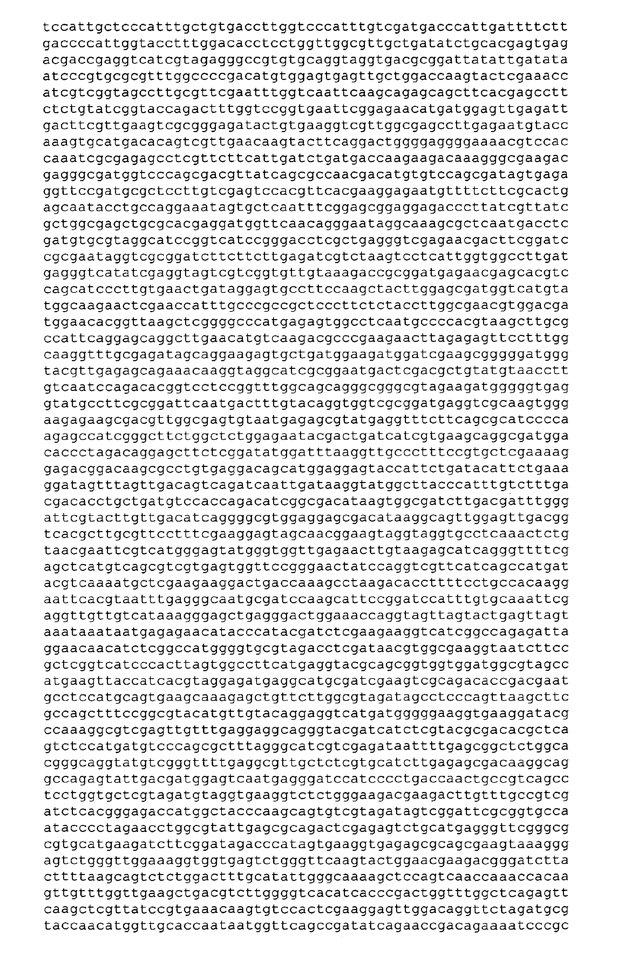

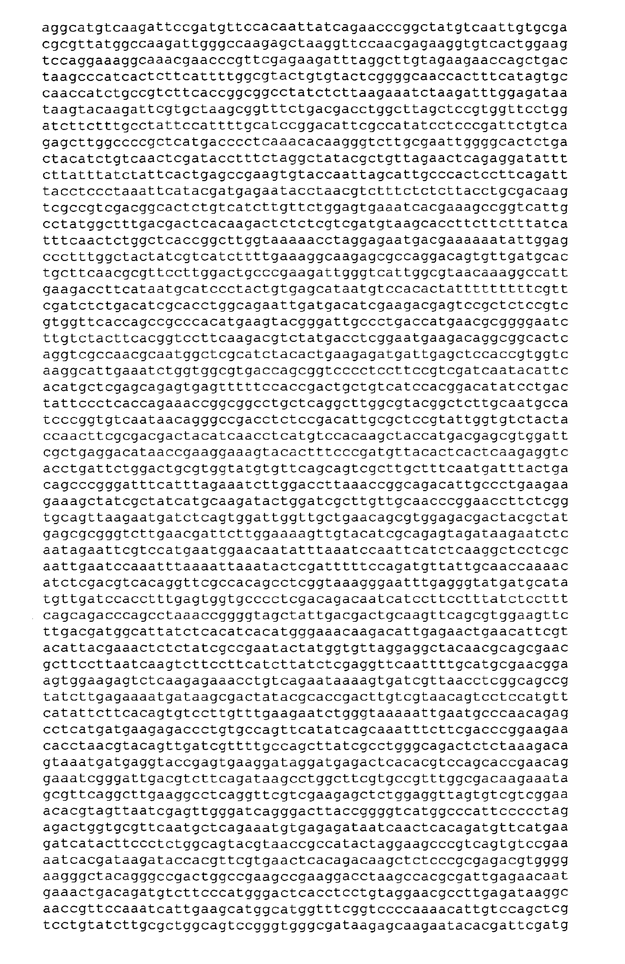

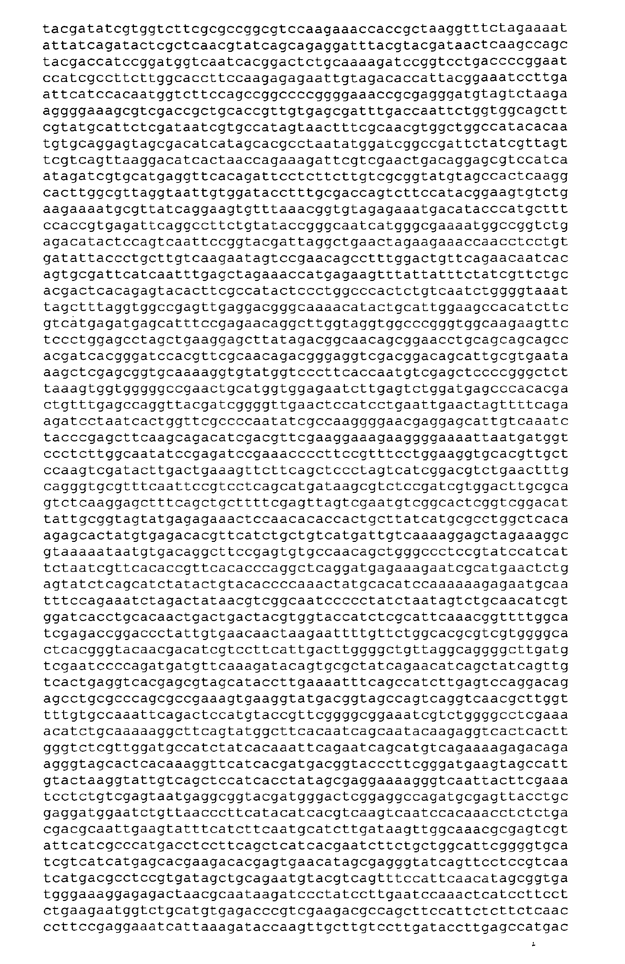

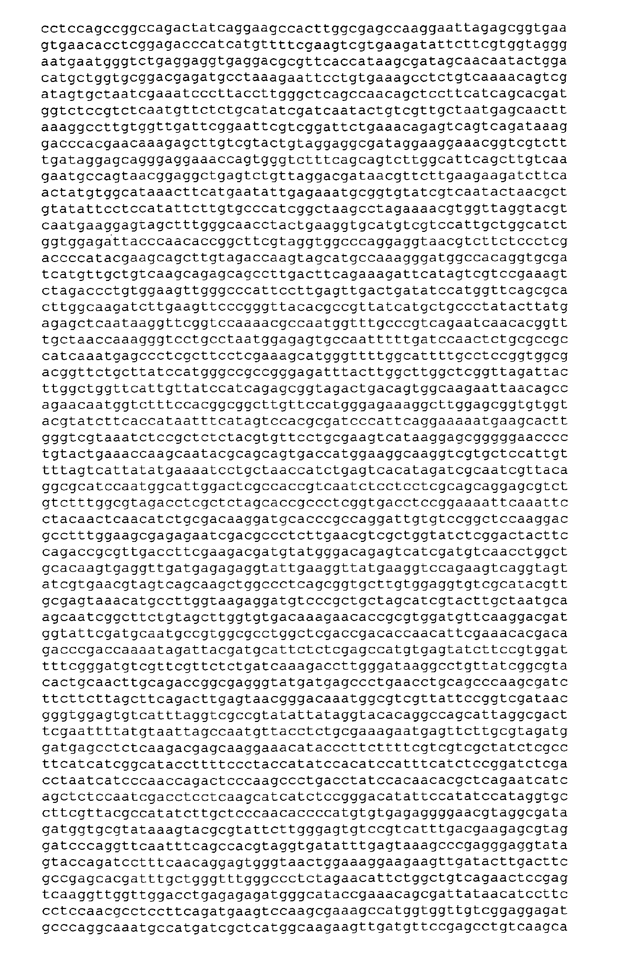

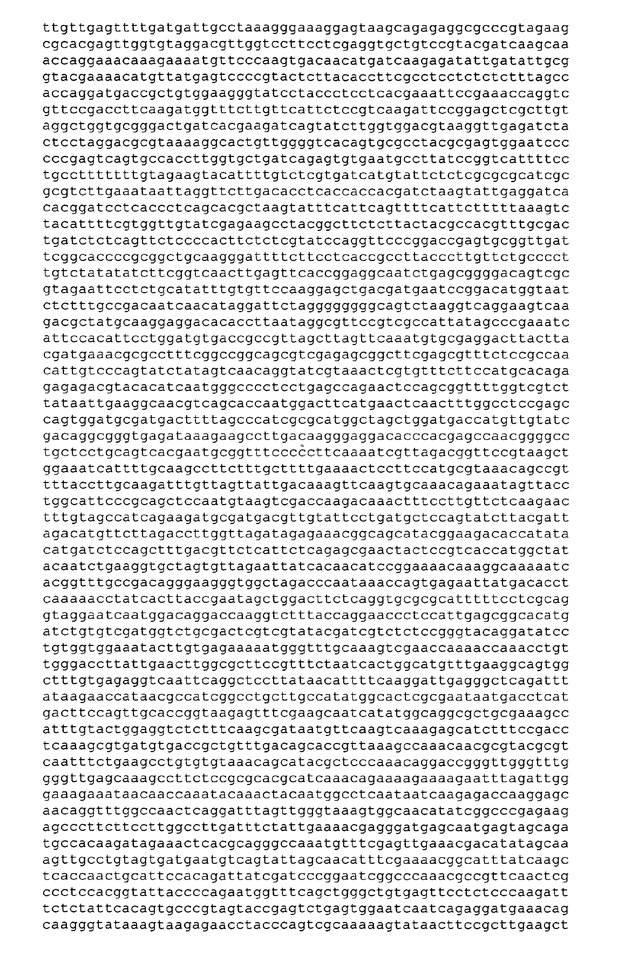

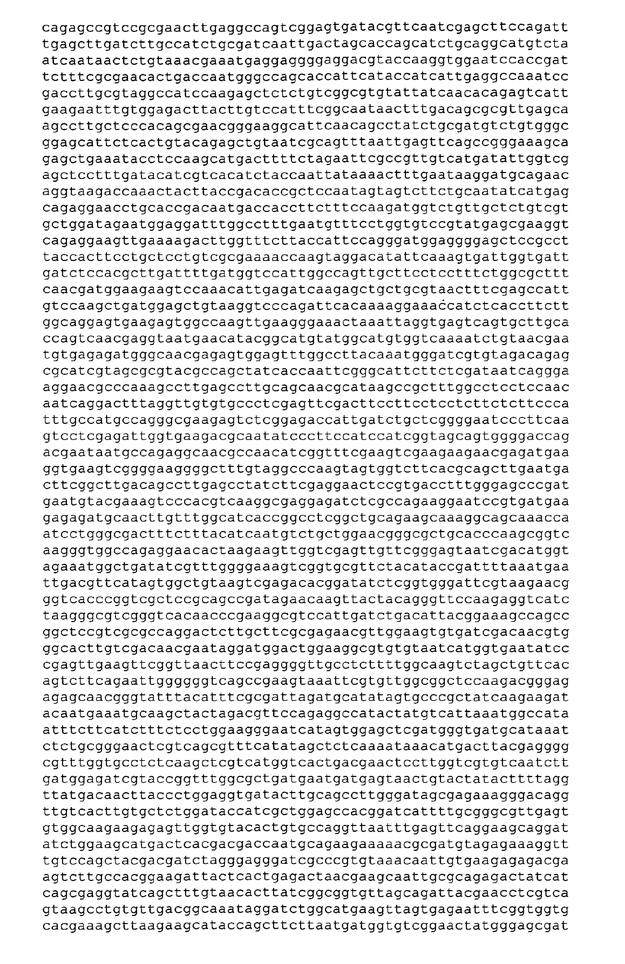

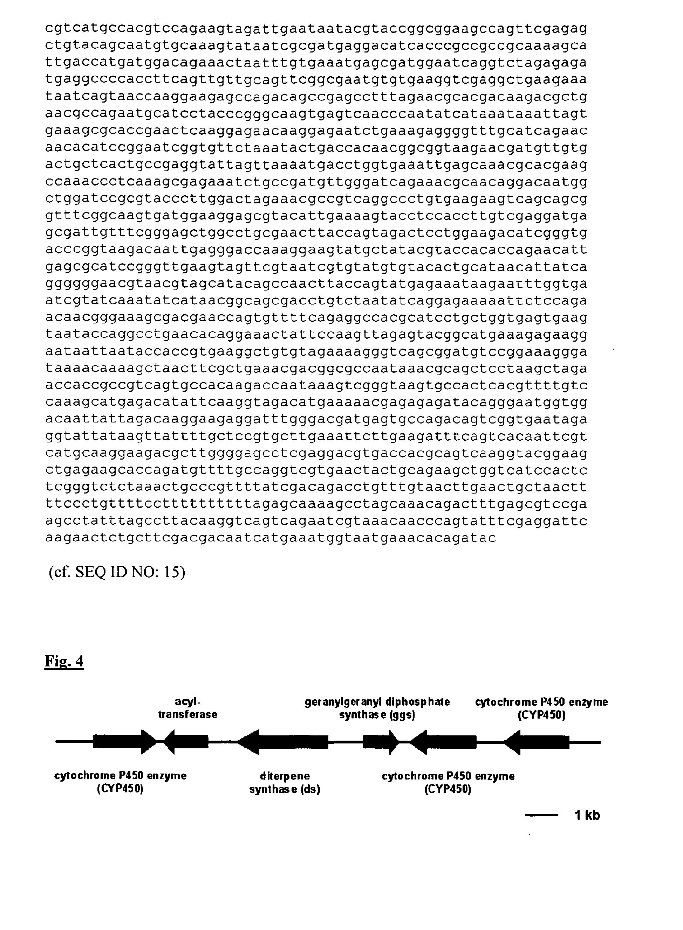

SEQ ID NO: 1: GNFMATPSTTAAYLMKATKWDDRAEDYLRHV
SEQ ID NO: 2: FEAPTYFRCYSFERNASVTVNSNCLMSLL
SEQ ID NO: 3: RLANDLHSISRDFNEVNLNSIMFSEF
SEQ ID NO: 4: DYINIIRVTYLHTALYDDLGRLTRADISNA
SEQ ID NO: 5: YSLLNHPRAQLASDNDKGLLRSEIEHYFLAG
SEQ ID NO: 6: SHYRWTHVVGADNVAGTIALVFALCLLG
SEQ ID NO: 7: PSSTFAKVEKGAAGKWFEFLPYMTIAPSSLEGTPI
SEQ ID NO: 8: is shown in Figure 3
SEQ ID NO: 9: is shown in Figure 3
SEQ ID NO: 10: 
ggtaacttcatggctacgccatccaccaccgctgcgtacctcatgaaggccactaagtgggatgaccgagcggaagattaccttcg
ccacgtt
SEQ ID NO: 11: tttgaggcacctacctacttccgttgctactccttcgaaaggaacgcaagcgtgaccgtcaactccaactgccttatgtcgctcctc
SEQ ID NO: 12: aggctcgccaacgaccttcacagtatctcccgcgacttcaacgaagtcaatctcaactccatcatgttctccgaattc
SEQ ID NO: 13 and 15: are shown in Figure 3
SEQ ID NOs: 1 to 3 are amino acid sequences of conserved regions of a preferred polypeptide, more precisely a preferred diterpene synthase, of the invention, whereas SEQ ID NOs: 4 to 7 relate to signature regions of said polypeptide.
SEQ ID NO: 9 (cf. also SEQ ID NO: 14) shows the supposed polypeptide sequence of the diterpene synthase involved in the production of pleuromutilin of *Clitopilus passseckerianus.*
SEQ ID NO: 8 shows the corresponding nucleotide sequence of the diterpene synthase cDNA involved in the production of pleuromutilin of *Clitopilus passseckerianus.*
SEQ ID NOs: 10 to 12 are nucleic acid sequences encoding the conserved regions of SEQ ID NOs: 1 to 3.
SEQ ID NO: 13 shows the supposed gene sequence of the diterpene synthase involved in the production of pleuromutilin of *Clitopilus passseckerianus.*
SEQ ID NO: 15 shows a nucleic acid sequence which is contemplated to comprise a gene cluster involved in the biosynthetic pathway for producing a diterpenoid, more precisely pleuromutilin. Said nucleic acid sequence is derived from the genome of *Clitopilus passseckerianus.*

### DETAILED DESCRIPTION OF PREFERRED ASPECTS AND EMBODIMENTS

The present invention particularly relates to compounds such as polypeptides and nucleic acid molecules, as well as methods and uses as defined in the claims.

Generally, in a first aspect, the present invention relates to novel isolated polypeptides, particularly to novel diterpene synthases, in particular pleuromutilin synthases.

In one embodiment, the polypeptide comprises an amino acid sequence which amino acid sequence comprises a sequence having at least 50% sequence identity to SEQ ID NO: 1, a sequence having at least 40% sequence identity to SEQ ID NO: 2, and at least one sequence selected from the group consisting of i) a sequence having at least 15% sequence identity to SEQ ID NO: 7; ii) a sequence having at least 25% sequence identity to SEQ ID NO: 4; iii) a sequence having at least 45% sequence identity to SEQ ID NO: 5; and iv) a sequence having at least 45% sequence identity to SEQ ID NO: 6, wherein SEQ ID NOs: 1-2 and 4-7 are of *Clitopilus passeckerianus* origin. The amino acid sequence of said isolated polypeptide may further comprise a sequence having at least 50% sequence identity to SEQ ID NO: 3. SEQ ID NOs: 1 to 7 are of *Clitopilus passseckerianus* origin.

In another embodiment, the polypeptide comprises an amino acid sequence which amino acid sequence comprises a sequence having at least 50% sequence identity to SEQ ID NO: 3, a sequence having at least 40% sequence identity to SEQ ID NO: 2, and at least one sequence selected from the group consisting of i) to iv) as defined above.

In another embodiment, the polypeptide comprises an amino acid sequence which amino acid sequence comprises a sequence having at least 50% sequence identity to SEQ ID NO: 1, a sequence having at least 50% sequence identity to SEQ ID NO: 3, and at least one sequence selected from the group consisting of i) to iv) as defined above.

In another embodiment, the polypeptide comprises an amino acid sequence which amino acid sequence comprises a sequence having at least 60%, particularly at least 70% sequence identity to SEQ ID NO: 9, more preferably at least 80%, even more preferably at least 85%, or even at least 90%, such as even more preferably at least 95% sequence identity to SEQ ID NO: 9.

The molecular weight of the isolated polypeptide define herein is preferably between 90 kDa and 140 kDa, particularly between 100 kDa and 130 kDa, especially between 105 kDa und 120 kDa.

In particular embodiments, the polypeptide comprises an amino acid sequence which amino acid sequence comprises SEQ ID NO: 1 and SEQ ID NO: 2, and at least one sequence selected from the group consisting of i) to iv) as defined above, especially at least one sequence selected from the group consisting of i') SEQ ID NO: 7; ii') SEQ ID NO: 4; iii') SEQ ID NO: 5; and iv') SEQ ID NO: 6. The amino acid sequence may further comprise SEQ ID NO: 3.

The isolated polypeptide of the invention preferably has diterpene synthase activity, especially pleuromutilin synthase activity. Accordingly, the isolated polypeptide is preferably involved in the biosynthetic pathway for producing pleuromutilin and is preferably capable of catalyzing the conversion of geranylgeranyl pyrophosphate into a pleuromutilin precursor, particularly into a compound according to formula (I). More preferably it is essential in pleuromutilin producing organisms for the production of pleuromutilin.

In certain preferred embodiments, the polypeptides of the invention are non-natural polypeptides.

According to a second aspect, there is provided an isolated nucleic acid molecule comprising
A) a nucleotide sequence encoding a polypeptide according to any one of claims 1 to 4 or a polypeptide of SEQ ID NO: 9,
B) a nucleotide sequence which is
   a) the sequence of SEQ ID NO: 8; or
   a') the sequence of SEQ ID NO: 15 or the sequence complementary thereto ; or
   b) a partial sequence of a sequence defined in a'), which partial sequence encodes a diterpene synthase; or
   c) a sequence which encodes a diterpene synthase and has at least 40% sequence identity to a sequence defined in a') or has at least 60% sequence identity to the sequence defined in a) or the partial sequence defined in b); or
   d) a sequence which encodes a diterpene synthase and which is degenerate as a result of the genetic code to a sequence defined in any one of a), a'), b) and c); or
   e) a sequence which encodes a diterpene synthase and which is capable of hybridizing to SEQ ID NO: 8 and/or SEQ ID NO: 13 under stringent conditions,
C) at least 18 consecutive nucleotides of a nucleotide sequence as defined in item B, and/or
D) at least 18 consecutive nucleotides and capable of hybridizing to a nucleic acid molecule having a nucleotide sequence as defined in item A or item B under stringent conditions.

In one preferred embodiment, the isolated nucleic acid molecule comprises A) as defined above. In another preferred embodiment, the isolated nucleic acid molecule comprises any one of B), such as Ba, Ba', Bb, Bc, Bd, Be, as defined above.

Generally, preferred nucleic acid molecules of the invention encode a diterpene synthase, more preferably a pleuromutilin synthase; and/or encode a polypeptide having diterpene synthase activity, more preferably a polypeptide having pleuromutilin synthase activity. As used herein, a "sequence which encodes a polypeptide having diterpene synthase activity" is preferably a sequence which encodes a diterpene synthase. Preferably, a sequence which encodes a diterpene synthase is a sequence which encodes a pleuromutilin synthase.

Also, as used herein, a "sequence which encodes a polypeptide having diterpene synthase activity" is preferably a sequence which encodes a polypeptide having pleuromutilin synthase activity. Preferably, a sequence which encodes a polypeptide having pleuromutilin synthase activity is a sequence which encodes a pleuromutilin synthase.

In one additional embodiment, the isolated nucleic acid molecule comprises C) as defined above. In one additional embodiment, the isolated nucleic acid molecule comprises D) as defined above. Said at least 18 consecutive nucleotides of C) or D) are preferably at least 19, 20, 25, particularly at least 30, 35, 40, 45, particularly at least 50, 55, 60, 65, particularly at least 70, 75, 80, 85, 90, 95, particularly at least 100, 150, 200, 250, particularly at least 300, 350, 400, 450, or 500 consecutive nucleotides.

The sequence according to Ba' was obtained by isolating and sequencing genomic DNA of *Clitopilus passeckerianus.*

In preferred embodiments, the nucleic acid molecule of the invention comprises a nucleotide sequence which nucleotide sequence comprises a sequence having at least 70% sequence identity to SEQ ID NO: 8, more preferably at least 80%, even more preferably at least 85%, or even at least 90%, such as even more preferably at least 95% sequence identity to SEQ ID NO: 8.

In other preferred embodiments, the nucleic acid molecule of the invention comprises a nucleotide sequence which nucleotide sequence comprises a sequence having at least 50%, particularly at least 60%, especially at least 70% sequence identity to SEQ ID NO: 15, more preferably at least 80%, even more preferably at least 85%, or even at least 90%, such as even more preferably at least 95% sequence identity to SEQ ID NO: 15. In certain preferred embodiments, the nucleic acid molecule of the invention comprises a gene cluster involved in a biosynthetic pathway for producing a diterpene, particularly for producing pleuromutilin.

The isolated polypeptides and the isolated nucleic acid molecules are preferably derivable from a fungal host, particularly a fungus from the division basidomycota, more particularly from the order agaricales, even more particularly from the family entolomataceae, especially from the genus *Clitopilus,* particularly from the group consisting of *Clitopilus scyphoides, Clitopilus prunulus, Clitopilus hobsonii, Clitopilus pseudo-pinsitus, Clitopilus pinsitus* and *Clitopilus passeckerianus,* in particular from *Clitopilus pinsitus* or *Clitopilus passeckerianus,* or from the genus *Pleurotus.*

In certain preferred embodiments, the nucleic acid molecules of the invention are non-natural nucleic acid molecules.

The nucleic acid molecule of the invention may further comprise one or more regulatory sequences e.g. selected from promoters, particularly strong promoters, enhancers, repressor binding sites, and terminators, or may further comprise any combination thereof.

A third aspect concerns a vector comprising a nucleic acid molecule of the present invention. Vectors are generally known in the art. Commonly vectors include phenotypical markes, preferably selectable markers, non-limiting examples of which include markers providing antibiotic resistance or prototrophy for certain amino acids. Alternatively, the markers may be screenable markers,

A fourth aspect relates to a host, particularly a non-naturally-occurring host selected from a cell, tissue and non-human organism, said host comprising at least one nucleic acid molecule of the invention and/or at least one vector of the invention. Particularly, said host is a fungal host, more particularly a fungus from the division basidomycota, even more particularly from the order agaricales, even more particularly from the family entolomataceae, especially from the genus *Clitopilus* or from the genus *Pleurotus.*

In some preferred embodiments, the host comprises a nucleotide sequence which nucleotide sequence comprises a sequence having at least 70% sequence identity to SEQ ID NO: 8, more preferably at least 80%, even more preferably at least 85%, or even at least 90%, such as even more preferably at least 95% sequence identity to SEQ ID NO: 8.

In other preferred embodiments, the host comprises a nucleotide sequence which nucleotide sequence comprises a sequence having at least 70% sequence identity to SEQ ID NO: 13, more preferably at least 80%, even more preferably at least 85%, or even at least 90%, such as even more preferably at least 95% sequence identity to SEQ ID NO: 13. In other preferred embodiments, the host comprises a nucleotide sequence which nucleotide sequence comprises a sequence having at least 50%, particularly at least 60%, especially at least 70% sequence identity to SEQ ID NO: 15, more preferably at least 80%, even more preferably at least 85%, or even at least 90%, such as even more preferably at least 95% sequence identity to SEQ ID NO: 15.

It is envisaged that based on the disclosure herein, and particularly on the disclosure of the putative ds cds and gene according to SEQ ID NOs: 8 and 13, as well as on SEQ ID NO: 15 including the supposed gene cluster involved in a biosynthetic pathway for producing a diterpene, a nucleic acid molecule of the invention encoding a diterpene synthase may be easily obtained by the skilled person. That is, employing bioinformatics techniques / computational techniques such as gene prediction software such as "GeneScan" a computational tool for the genome-wide prediction of protein coding genes from eukaryotic DNA sequences, may suitably be employed to identify the identity and location of said nucleic acid molecules as well as of further nucleic acid sequences encoding proteins involved in the biosynthetic pathway for producing a diterpene which sequences are part of the gene cluster described herein.

In addition, SEQ ID NO: 15, which sequence is envisaged to encode a gene cluster involved in a biosynthetic pathway for producing a diterpene, may suitably be employed in this respect. Said nucleic acid molecule may e.g. be removed from a *Clitopilus passeckerianus*, and/or may be modified by routine techniques such as site directed mutagenesis in order to arrive at further nucleotide sequences of the invention. Particularly in light of the partial nucleic acid sequences SEQ ID NOs: 10 to 12, which encode conserved regions of a preferred polypeptide of the invention, and in particular SEQ ID NO: 8, which encodes the preferred polypeptide of the invention, and in particular SEQ ID NO: 15, which sequence is envisaged to encode a gene cluster involved in a biosynthetic pathway for producing a diterpene, the skilled person is considered to be readily in a position to identify and obtain nucleic acids of the invention.

It is envisaged that based on the disclosure herein nucleic acid molecules of the invention as well as a vector of the invention may also be obtained by a procedure which is outlined below: First steps are, for example, preparing a messenger RNA population isolated from a specific cell culture, e.g. a cell culture from *Clitopilus passeckerianus* such as mycelium from *Clitopilus passeckerianus*, preparing DNA probes suitable for hybridizing with at least a part of the desired messenger RNA. Exemplary DNA probes that are considered suitable for this purpose may be based on DNA sequences such as SEQ ID NOs 8 and 10 to 13, of which DNA sequence SEQ ID NO: 8 is a nucleic acid sequences encoding a preferred polypeptide of the invention and SEQ ID NOs: 10 to 12 encode conserved regions of said preferred polypeptide of the invention.

Subsequent steps are screening the messenger RNA population by said DNA probes, preparing cDNA molecules from the messenger RNAs via a reverse transcriptase, cloning the cDNA molecules into vectors, analyzing these vectors, e.g. by means of restriction enzymes or DNA sequencing, and selecting vectors which carry or are likely to carry the cDNA fragments. The cDNA fragment may subsequently be transferred into a suitable expression vector, equipped with well-known genetic elements that allow expression of the polypeptide. In case the cDNA fragment does not represent the desired gene, cDNA fragments of different clones may be combined and subsequently inserted in a suitable expression vector, equipped with well-known genetic elements that allow expression of the polypeptide. Adjacent regions of a region of interest may be further analyzed by means of screening a cosmid library containing larger sequence portions, or by techniques such as genome walking, e.g. within a gene bank comprising mutually overlapping DNA regions.

It is envisaged that the nucleic acid molecules may be prepared from *Clitopilus passeckerianus* or another organism which is known or suspected to be capable of producing a diterpene, particularly a pleuromutilin antibiotic, especially pleuromutilin. Preferably, the organism may be a fungal host, more particularly a fungus from the division basidomycota, even more particularly from the order agaricales, even more particularly from the family entolomataceae, in particular from the genus *Clitopilus* or *Pleurotus,* especially one of the pleuromutilin producers disclosed herein. Positive clones may be analyzed e.g. by restriction enzyme analysis, sequencing, sequence comparisons and others.

One exemplary suitable technique to obtain a full length sequence of an RNA transcript is known as "RACE" (rapid amplification of cDNA ends), which results in the production of a cDNA copy of the RNA sequence of interest, produced through reverse transcription of the cDNA copies. PCR-amplified cDNA copies are then sequenced. RACE may provide the sequence of an RNA transcript from a small known sequence within the transcript to the 5' end (5' RACE-PCR) or 3' end (3' RACE-PCR) of the RNA.

Tools to more closely define the borders of the gene cluster and uncover the regulation of the cluster, which are known to the skilled person, include transcription profiling by qRT-PCR (quantitative real time polymerase chain reaction) where amplified cDNA is detected in real time as the reaction progresses. In particular this technique is suitable to identify co-regulation and to quantify the expression level of genes which belong to the gene cluster and which are functional related. In addition transcription profiling can also be applied to identify functional related genes which are located elsewhere in the genome.

Suitable details for assisting the obtaining of a nucleic acid sequence of the invention on the basis of the disclosure may e.g. be taken from *Kawaide et al., 1997*, or *Kilaru et al., 2009b.*

Sequences of polypeptides of the invention may easily be deduced from the thus obtained nucleic acid sequences. Moreover, a polypeptide of the invention may be prepared by using the thus obtained cDNA (nucleic acid molecule) or expression vector in a suitable method to achieve expression of polypeptides, such as by introduction into a host. Accordingly, a fifth aspect concerns a method of producing a polypeptide of the invention, the method comprising (i) introducing into a host selected from a cell, tissue and non-human organism at least one nucleic acid molecule of the invention and/or at least one vector of the invention, and (ii) cultivating the host under conditions suitable for the production of the polypeptide. Particularly, the method comprises a further step of (iii) recovering the polypeptide from the host.

Methods for introducing genetic material into a host are well known to the skilled person, preferred ways depending on the respective host. For example in case that the host is a fungus such as from the genus *Clitopilus,* non-limiting examples are considered to include *Agrobacterium-mediated* transformation systems and PEG-mediated transformation systems (cf. *Kilaru et al., 2009b*)*.*

Methods of recovering or purifying, respectively, polypeptides from a host are known to a person skilled in the art. These methods may employ any known chromatographic techniques such as ion exchange chromatography or HPLC, centrifugation techniques such as ultracentrifugation, precipitation techniques such as ammonium sulfate precipitation, differential solubilization techniques, and the like. Conveniently, a polypeptide may be purified by any known technique involving the use of an N-terminal or a C-terminal tag, such as a His-tag, and a corresponding purification technique, such as involving Ni²⁺-affinity chromatography. Step (iii) may also be dispensable when employing means that allow secretion of the polypeptide from the host such as by using signal peptides. In the latter case, the growth medium may be used as is or may be subjected to one or more purification steps.

A sixth aspect concerns a method of producing pleuromutilin, the method comprising (i) introducing into a host selected from a cell, tissue and non-human organism a gene cluster of the invention such as a nucleic acid molecule having a sequence as defined in Ba' above, and (ii) cultivating the host under conditions suitable for the production of pleuromutilin.

In one embodiment, said method comprises introducing a vector comprising a sequence as defined in Ba' above in addition to or instead of a nucleic acid molecule having a sequence as defined in Ba' above.

The host may be a host capable or incapable of producing pleuromutilin and preferably is a host incapable of producing pleuromutilin. The host may be incapable of producing pleuromutilin due to the absence of a whole gene cluster for producing pleuromutilin or due to the absence of parts thereof, which is why said method is considered feasible by providing the whole cluster, such as a sequence as defined in Ba' above. Alternatively, only (the missing) parts of the cluster may be provided to complete the cluster and allow production of pleuromutilin. Particularly, the host may be incapable of producing pleuromutilin due to the absence of a nucleic acid encoding a pleuromutilin synthase or a polypeptide having pleuromutilin synthase activity as disclosed herein. In this case, a nucleic acid molecule or polypeptide of the invention may be introduced. Generally, more than one copy of the nucleic acid molecule may be introduced to increase production of pleuromutilin. The nucleic acid molecule(s) may be part of a vector. Upon introduction, the introduced nucleic acid molecule(s) may or may not integrate into a chromosome.

A seventh aspect generally concerns a method of altering the production of pleuromutilin in a host selected from a cell, tissue and non-human organism. In preferred embodiments, it concerns a method of altering the production of pleuromutilin in a host selected from a cell, tissue and non-human organism, wherein said host is capable of producing pleuromutilin and comprises at least one nucleic acid molecule comprising a nucleotide sequence as defined in A) or B) above, the method comprising manipulating i) the expression, ii) the identity, or iii) both the expression and the identity of said at least one nucleic acid molecule.

It is contemplated that this method is a method of increasing the production of pleuromutilin. Increasing the production of pleuromutilin may be achieved by directly or indirectly manipulating the expression of said at least one nucleic acid molecule. Direct manipulation in this respect may for example be achieved by the provision of further copies of said at least one nucleic acid molecule such as by the introduction of vectors disclosed herein and/or or by incorporation into a chromosome. In a preferred embodiment, overexpression of one or more nucleic acid molecules of the invention is achieved by means of one or more vectors. In one embodiment, the sequence of SEQ ID NO: 15 obtainable from *Clitopilus passeckerianus,* which sequence is envisaged to encode a gene cluster involved in a biosynthetic pathway for producing a diterpene and which sequence is obtainable by molecular biological methods known in the art, if the skilled man is provided with the information given herein, in particular the nucleic acid sequences SEQ ID NO: 8 and 10-12, is introduced into the host. Suitable vectors and expression systems are known in the art, and include those vector/selection systems described in the following publications, which are hereby incorporated by reference, as examples for Basidomycetes: *Binninger et al., 1987; Kilaru et al., 2009a;* and *Kilaru et al., 2009b,* and those vector/selection systems described in the following publications, which are hereby incorporated by reference, as examples for Streptomycetes: *Lacalle et al., 1992; Jones and Hopwood, 1984* and *Motamedi and Hutchison, 1987.*

Indirect manipulation in this respect may for example be achieved by the provision of elements such as suitable promoters or enhancers, or by the removal of repressor binding sites or terminators, or by combinations thereof. Promoters, enhancers, terminators, and the like are known to the skilled person. The production of pleuromutilin may also be increased by manipulating the identity of said at least one nucleic acid molecule such as by mutagenesis thereof and selection for increased pleuromutilin production. Alternatively, the production of pleuromutilin may be increased by a combination thereof. The expression may also be indirectly increased by optimizing the expression and/or transcriptional regulation of at least one nucleic acid molecule during fermentation of the host through the adjusting of physiological parameters and/or fermentation conditions. In one embodiment, regulatory genes and/or DNA binding sites of regulatory proteins of the at least one nucleic acid molecule are influenced.

It is also contemplated that the method of the seventh aspect is a method of decreasing the production of pleuromutilin. Particularly, it may comprise disrupting or down-regulating said at least one nucleic acid molecule. Methods for direct manipulation in this respect include the targeted disruption of genes that is well-known within the art. Therefore, e.g. a mutated host such as a mutated *Clitopilus* strain, such as a mutated *Clitopilus passeckerianus* strain may be constructed, from which one or more natural nucleic acids encoding polypeptides involved in the biosynthetic pathway for producing pleuromutilin have been partly or completely deleted from the genome. Alternatively, the identity of said at least one nucleic acid molecule may be manipulated e.g. by mutagenesis thereof and selection for decreased pleuromutilin production. Generally, other suitable such methods to decrease the production of pleuromutilin may involve the use of RNA interference (RNAi). The feasibility for an RNAi mediated gene silencing as a means of knocking down expression of specific genes has been demonstrated just recently in the dikaryotic *Clitopilus passeckerianus* by *Kilaru et al., 2009b*)*.* Here, it is believed to be more convenient than targeted gene disruption due to the dikaryotic nature of this fungus. The methods described in this reference are contemplated to be likewise applicable in other basidiomycetes, particularly in other members of the genus *Clitopilus,* such as the preferred ones disclosed herein including *Clitopilus passeckerianus.* Alternatively or additionally, production of pleuromutilin may be decreased by indirectly manipulating the expression by means of removal of elements such as suitable promoters or enhancers, or by the provision of repressor binding sites or terminators, or by combinations thereof. Generally, e.g. (limited) mutagenesis of the pleuromutilin gene cluster in, for example, promoter regions or coding sequences, may be used for altering the production of pleuromutilin. For example, it can lead to a beneficial increase in the capabilities of a pleuromutilin producing organism to produce a pleuromutilin precursor or can even change the final product of pleuromutilin biosynthesis.

The production of pleuromutilin in a host may also be altered by replacing individual sections in the at least one nucleic acid molecule by other sections, such as sections from other gene clusters. The production of pleuromutilin in a host may also be altered by inactivating individual steps in the biosynthetic pathway for producing pleuromutilin, such as by deleting or disrupting other polypeptides of the gene cluster. Also in this aspect, the host is preferably as defined hereinabove.

The method of altering the production of pleuromutilin in a host selected from a cell, tissue and non-human organism may particularly involve the use of an isolated nucleic acid molecule, a vector or of a host cell of the invention a) for overexpressing at least one nucleic acid molecule encoding a polypeptide involved in the biosynthetic pathway for producing pleuromutilin; or b) for inactivating or modifying one or more genes involved in the biosynthetic pathway for producing pleuromutilin; or c) for constructing a non-naturally occurring host from which one or more genes involved in the biosynthetic pathway for producing pleuromutilin have been deleted; or c) for constructing a mutated host, such as *Clitopilus* strains, from which one or more genes involved in the biosynthetic pathway for producing pleuromutilin have been deleted or disrupted.

The method of altering the production of pleuromutilin in a host selected from a cell, tissue and non-human organism may also involve the redirection of metabolic fluxes towards the educt of the MVA pathway, i.e. acetyl-CoA, and/or to increase the amount of substrate for the geranylgeranyl disphosphate synthase, e.g. by cutting off non-essential reactions competing for the precursors IPP and DMAPP.

An eighth aspect concerns the use of a nucleic acid molecule of the invention in the production of pleuromutilin, wherein 2 to 50 nucleotides of the sequence of said nucleic acid molecule are divergent from a sequence of a gene cluster involved in the biosynthetic pathway for producing pleuromutilin comprised by a wild type organism capable of producing pleuromutilin Preferably, said 2 to 50 nucleotides are at least 3, 4, 5, 6, 7, 8, 9, 10, 11, 12, 13, 14, 15, 16, 17, 18, 19, 20, 25, 30, 35, 40, or 45 nucleotides, preferably from 3 to 100, more preferably from 5 to 80 nucleotides, even more preferably from 8 to 60 nucleotides. Such limited mutagenesis of the pleuromutilin gene cluster in, for example, promoter regions or coding sequences, can lead to a beneficial increase in the capabilities of a pleuromutilin producing organism to produce pleuromutilin or can even change the final product of pleuromutilin biosynthesis, thereby yielding a higher yield of pleuromutilin precursors. Yet, said nucleic acid molecule must not be identical to a sequence of a gene cluster involved in the biosynthetic pathway for producing pleuromutilin comprised by a wild type organism capable of producing pleuromutilin or any one of the organisms selected for pleuromutilin production as of the priority date of this application. The use is not particularly limited and may comprise any of the methods for producing pleuromutilin disclosed herein. The use may involve the complete gene cluster as disclosed above, or any partial sequence thereof as long as the sequence employed is divergent from a sequence of a gene cluster involved in the biosynthetic pathway for producing pleuromutilin comprised by a wild type organism, particularly a wild type organism capable of producing pleuromutilin. The use may e.g. be an *in vivo, ex vivo* or *in vitro* use or a combination thereof.

A ninth aspect concerns the use of an isolated nucleic acid molecule of the invention in the production of a pleuromutilin precursor, wherein 2 to 50 nucleotides of the sequence of said nucleic acid molecule are divergent from a sequence encoding a diterpene synthase comprised by a wild type organism capable of producing pleuromutilin. Preferably, said 2 to 50 nucleotides are at least 3, 4, 5, 6, 7, 8, 9, 10, 11, 12, 13, 14, 15, 16, 17, 18, 19, 20, 25, 30, 35, 40, or 45 nucleotides, preferably from 3 to 100, more preferably from 5 to 80 nucleotides, even more preferably from 8 to 60 nucleotides. Such limited mutagenesis of the pleuromutilin gene cluster in, for example, promoter regions or coding sequences, can lead to a beneficial increase in the capabilities of a pleuromutilin producing organism to produce a pleuromutilin precursor or can even change the final product of pleuromutilin biosynthesis, which may e.g. result in a higher yield of pleuromutilin precursors. Particularly, the pleuromutilin precursor is a compound according to formula (I). The use is not particularly limited and may comprise any of the methods for producing a pleuromutilin precursor disclosed herein. The use may involve the complete gene cluster as disclosed above, or any partial sequence thereof as long as the sequence employed is divergent from a sequence encoding a diterpene synthase comprised by a wild type organism, particularly a wild type organism capable of producing pleuromutilin. The use may e.g. be an *in vivo, ex vivo* or *in vitro* use or a combination thereof.

A tenth aspect concerns the use of a host according to claim 8, in the production of pleuromutilin or of a pleuromutilin precursor, particularly wherein said pleuromutilin precursor is a compound according to formula (I). The use is not particularly limited. It may involve any of the hosts disclosed herein and may e.g. be an *in vivo, ex vivo* or *in vitro* use or a combination thereof.

Similarly, the invention concerns methods for the production of pleuromutilin or pleuromutilin precursor that correspond to the uses of the eight to tenth aspect and include at least one step of using an isolated nucleic acid molecule, a vector, or a host of the invention.

An eleventh aspect concerns the use of an isolated nucleic acid molecule of the invention for identifying one or more nucleic acids encoding a polypeptide having diterpene synthase activity. Preferably, such nucleic acids encoding a polypeptide having diterpene synthase activity encode a polypeptide having pleuromutilin synthase activity.

In addition, this aspect concerns the use of an isolated nucleic acid molecule of the invention for identifying one or more nucleic acids encoding a diterpene synthase, preferably encoding a pleuromutilin synthase.

In certain embodiments, said aspect concerns the use of a nucleic acid molecule of the invention for identifying a gene cluster of the invention involved in a biosynthetic pathway for producing a diterpene, particularly for producing pleuromutilin.

For example, the nucleic acid molecule of the invention may be used in a method which is characterized by the steps of establishing a cDNA or genomic library, screening said library by using a nucleic acid molecule disclosed herein, and isolating positive clones. That is to say, the nucleic acid molecules disclosed herein or partial sequences thereof may be employed as probes for screening a cDNA or genomic library. Various standard methods are available for identifying positive clones. In some embodiments, to improve detectability, an isolated nucleic acid molecule described herein may be labeled with a detectable label, such as any label that is easily identifiable e.g. by known physical or chemical methods. Detectable labels are well-known within the art and include, but are not limited to radiolabels, chromophores, fluorescent agents, enzymes, coenzymes, substrates, enzyme inhibitors, antibodies and the like.

The cDNA or genomic library may be prepared from any candidate organism, particularly from an organism which is known or suspected to be capable of producing a diterpene, particularly a pleuromutilin antibiotic, especially pleuromutilin. Preferably, the organism is selected from a fungal host, more particularly a fungus from the division basidomycota, even more particularly from the order agaricales, even more particularly from the family entolomataceae, in particular from the genus *Clitopilus* or *Pleurotus,* such as from the group consisting of *Clitopilus scyphoides, Clitopilus prunulus, Clitopilus hobsonii, Clitopilus pseudo-pinsitus, Clitopilus pinsitus* and *Clitopilus passeckerianus,* especially wherein said organism is *Clitopilus pinsitus* or *Clitopilus passeckerianus.* Positive clones may be analyzed e.g. by restriction enzyme analysis, sequencing, sequence comparisons and others. Adjacent regions of a region of interest may be further analyzed by means of screening a cosmid library containing larger sequence portions, or by techniques such as genome walking, e.g. within a genomic library comprising mutually overlapping DNA regions.

A method for identifying a nucleic acid i) encoding a polypeptide having diterpene synthase activity, ii) encoding a polypeptide having pleuromutilin synthase activity, iii) encoding a diterpene synthase, and/or iv) encoding a pleuromutilin synthase, may comprise a step of performing a Southern blot with chromosomal DNA of a candidate organism to detect the presence of nucleic acids having homology with nucleic acid molecules disclosed herein.

Optionally, a method for identifying a nucleic acid i) encoding a polypeptide having diterpene synthase activity, ii) encoding a polypeptide having pleuromutilin synthase activity, iii) encoding a diterpene synthase, and/or iv) encoding a pleuromutilin synthase, may comprise a preceding step of determining whether the candidate organism is capable of producing pleuromutilin by assaying for pleuromutilin production by any known method.

The invention further concerns the use of an isolated nucleic acid molecule of the invention for isolating one or more nucleic acids encoding a polypeptide having diterpene synthase activity and/or encoding a polypeptide having pleuromutilin synthase activity from an organism, preferably wherein the organism is as defined above.

In addition, this aspect concerns the use of an isolated nucleic acid molecule of the invention for isolating one or more nucleic acids encoding a diterpene synthase, preferably encoding a pleuromutilin synthase from an organism, preferably wherein the organism is as defined above.

A twelfth aspect concerns a method of the production of a pleuromutilin precursor, particularly of a compound according to formula (I), wherein the method is a method for the fermentative production of said precursor and comprises the steps of (i) introducing into a host selected from a cell, tissue and non-human organism at least one nucleic acid molecule of the invention and/or at least one vector of the invention, and (ii) cultivating the host under conditions suitable for the fermentative production of said precursor.

A thirteenth aspect concerns a method of the production of a pleuromutilin precursor, particularly of a compound according to formula (I), wherein the method is a method for the synthetic production of said precursor and comprises reacting geranylgeranylpyrophosphate with a polypeptide according of the invention or a polypeptide obtainable by a method described above. In the latter two aspects, the host is preferably as disclosed hereinabove.

In an even further aspect, the invention concerns an isolated compound according to formula (I). In some embodiments, the isolated compound according to formula (I) is obtainable by a method disclosed herein, particularly by a method of the twelfth or thirteenth aspect.

In a still further aspect, the diterpene, particularly the pleuromutilin precursor, produced in accordance with the invention may be converted into a pleuromutilin antibiotic such as a new pleuromutilin antibiotic. This may be done by synthetic organic chemistry, e.g. in analogous ways to how pleuromutilin is converted to tiamulin (analogous to the process described in IN 2005CH00521), valnemulin (analogous to the process described in CN 101318921), retapamulin (analogous to the process described in WO 2009075776) and the like. The present invention thus also relates to the use of intermediate I (i.e. the compound according to formula (I)) in the semisynthetic production of a pleuromutilin antibiotic. "Semisynthetic production" relates to a combination of fermentative production of a pleuromutilin precursor followed by at least one synthetic chemical step to yield a covalent modification of the pleuromutilin precursor.

Thus the invention also concerns a method for the production of a pleuromutilin antibiotic, wherein the method comprises at least one step of reacting a diterpene or pleuromutilin precursor obtained by means of a polypeptide or method of the invention.

In an even further aspect, the new pleuromutilin antibiotic, especially the new pleuromutilin obtained in accordance with the invention is used as a medicament. In one embodiment, it is used in a method of treating a bacterial infection. Also envisaged is a method of treating a bacterial infection involving a step of administering a diterpene, particularly the new pleuromutilin antibiotic, especially the new semisynthetic pleuromutilin obtained in accordance with the invention, to a subject in need thereof, particularly a subject suffering from a bacterial infection or a disorder or disease involving a bacterium

The bacterium (causing the bacterial infection or involved in said disorder or disease) may be selected from the group consisting of Gram-positive bacteria particularly staphylococci, streptococci, pneumococci and enterococci; Gram-negative bacteria particularly selected from the genera *Neisseria, Haemophilus, Moraxella, Bordetella, Legionella, Leptospira;* mycoplasmas; chlamydia; Gram-positive anaerobes and Gram-negative anaerobes.

Generally, all documents cited herein are incorporated by reference herein in their entirety. Also, while certain aspects and embodiments of this invention are described or exemplified herein, it will be understood by a person skilled in the art that various modifications can be made without departing from the spirit and scope of the invention.

### EXEMPLIFYING SECTION

The following examples are meant to further illustrate, but not limit, the invention. The examples comprise technical features and it will be appreciated that the invention relates also to combinations of the technical features presented in this exemplifying section.

### Example 1: Identification of a diterpene synthase (DS)

The present inventors have identified a ds gene in *Clitopilus passeckerianus.* Partial protein sequences of the polypeptide encoded by the ds gene that have been identified and are included herein as SEQ ID NOs: 1 - 7. The putative protein sequence of the diterpene synthase encoded by the ds gene is herein included as SEQ ID NO: 9 and was obtained by computational methods. Alignment of the sequence with known terpene synthase sequences has revealed numerous shared conserved regions rendering it likely that the polypeptide has diterpene synthase activity. Particularly since *Clitopilus passeckerianus* is known to the present inventors as a pleuromutilin producing strain and in view of the fact that this is the only diterpene synthase in *Clitopilus passeckerianus,* it is envisaged that the identified diterpene synthase has pleuromutilin synthase activity. An alignment of the putative protein sequence of the diterpene synthase encoded by the ds gene (designated as "*C.p*") and several known diterpene synthase sequences is shown in Figure 5.

### Example 2: Identification of the putative pleuromutilin gene cluster in Clitopilus passeckerianus

To arrive at the present invention, the inventors have analyzed the genomic region around the putative diterpene synthase described above and have employed bioinformatics tools using genomic sequences of *Clitopilus passeckerianus.*

The present inventors have, in close proximity to the putative ds gene, identified a previously unknown putative geranylgeranyl diphosphate synthase (ggs) gene of *Clitopilus passeckerianus.* The new ggs gene from *Clitopilus passeckerianus* shows a close relationship to known ggs genes. An alignment with a known ggs gene of *Phomopsis amygdali* (BAG30959) gives 31% identity. The identities were calculated by aligning sequences with the freeware program ClustalX (Version 1.83) with default parameters and subsequent counting of identical residues by hand. Percentage identity (PID) was then calculated by dividing the number of identities by length of the shortest sequence. Default settings for, e.g., pairwise alignment (slow-accurate) are: gap opening parameter: 10.00; gap extension parameter 0.10; Protein weight matrix: Gonnet 250; DNA weight matrix IUB. The ClustalX program is described in detail in *Thompson et al., 1997*

As illustrated in Figure 4, further analysis of a genomic region comprising SEQ ID NO: 8 and having a size of about 27 kb further revealed three putative CYP450 genes, a putative acyltransferase gene as well as further putative genes in proximity to the ggs and ds genes, which suggests that this cluster is the pleuromutilin biosynthesis gene cluster. Moreover, the presence of these enzymatic activities is in line with the predicted biosynthetic pathway. Furthermore, expression analysis reveals that those genes show co-regulation under various conditions known to influence gene expression patterns, further corroborating the above conclusion.

### Particular embodiments

In the following section, the present invention is illustrated by several embodiments which are, however, not intended to limit the scope of the present invention.
1. An isolated polypeptide comprising an amino acid sequence,
   which amino acid sequence comprises one, particularly two, especially three sequences selected from the group consisting of
   a1) a sequence having at least 50% sequence identity to SEQ ID NO: 1;
   a2) a sequence having at least 40% sequence identity to SEQ ID NO: 2; and
   a3) a sequence having at least 50% sequence identity to SEQ ID NO: 3, and which amino acid sequence comprises at least one sequence selected from the group consisting of
   b1) a sequence having at least 25% sequence identity to SEQ ID NO: 4;
   b2) a sequence having at least 15% sequence identity to SEQ ID NO: 7;
   b3) a sequence having at least 45% sequence identity to SEQ ID NO: 5; and
   b4) a sequence having at least 45% sequence identity to SEQ ID NO: 6.
2. The isolated polypeptide of embodiment 1, wherein said amino acid sequence comprises any group of sequences selected from the following groups of sequences as defined in embodiment 1
   a1, b1; a1, b2; a1, b3; a1, b4; a1, b1, b2; a1, b1, b3; a1, b1, b4; a1, b2, b3; a1, b2, b4; a1, b3, b4; a1, b1, b2, b3; a1, b1, b2, b4; a1, b1, b3, b4; a1, b2, b3, b4; a1, b1, b2, b3, b4;
   a2, b1; a2, b2; a2, b3; a2, b4; a2, b1, b2; a2, b1, b3; a2, b1, b4; a2, b2, b3; a2, b2, b4; a2, b3, b4; a2, b1, b2, b3; a2, b1, b2, b4; a2, b1, b3, b4; a2, b2, b3, b4; a2, b1, b2, b3, b4;
   a3, b1; a3, b2; a3, b3; a3, b4; a3, b1, b2; a3, b1, b3; a3, b1, b4; a3, b2, b3; a3, b2, b4; a3, b3, b4; a3, b1, b2, b3; a3, b1, b2, b4; a3, b1, b3, b4; a3, b2, b3, b4; a3, b1, b2, b3, b4.
3. An isolated polypeptide comprising an amino acid sequence,
   which amino acid sequence comprises
   a1) a sequence having at least 50% sequence identity to SEQ ID NO: 1; and
   a2) a sequence having at least 40% sequence identity to SEQ ID NO: 2; and, optionally,
   a3) a sequence having at least 50% sequence identity to SEQ ID NO: 3,
   and which amino acid sequence comprises at least one sequence selected from the group consisting of
   b1) a sequence having at least 25% sequence identity to SEQ ID NO: 4;
   b2) a sequence having at least 15% sequence identity to SEQ ID NO: 7;
   b3) a sequence having at least 45% sequence identity to SEQ ID NO: 5; and
   b4) a sequence having at least 45% sequence identity to SEQ ID NO: 6.
4. The isolated polypeptide according to any one of embodiments 1 or 3,
   wherein said amino acid sequence comprises any group of sequences selected from the following groups of sequences as defined in embodiment 1
   a1, a2, b1; a1, a2, b2; a1, a2, b3; a1, a2, b4;
   a1, a2, b1, b2; a1, a2, b1, b3; a1, a2, b1, b4; a1, a2, b2, b3; a1, a2, b2, b4; a1, a2, b3, b4;
   a1, a2, b1, b2, b3; a1, a2, b1, b2, b4; a1, a2, b1, b3, b4; a1, a2, b2, b3, b4; a1, a2, b1, b2, b3, b4.
5. The isolated polypeptide according to any one of embodiments 1 or 3,
   wherein said amino acid sequence comprises any group of sequences selected from the following groups of sequences as defined in embodiment 1
   a1, a2, a3, b1, a1, a2, a3, b2; a1, a2, a3, b3; a1, a2, a3, b4;
   a1, a2, a3, b1, b2; a1, a2, a3, b1, b3; a1, a2, a3, b1, b4; a1, a2, a3, b2, b3; a1, a2, a3, b2, b4, a1, a2, a3, b3, b4;
   a1, a2, a3, b1, b2, b3; a1, a2, a3, b1, b2, b4; a1, a2, a3, b1, b3, b4; a1, a2, a3, b2, b3, b4;
   a1, a2, a3, b1, b2, b3, b4.
6. The polypeptide according to any one of embodiments 1-5, which comprises two sequences, particularly three sequences, especially four sequences of b1, b2, b3, and b4.
7. The polypeptide according to any one of embodiments 1-6, wherein the sequence defined in a1 has at least 55%, 60%, 65%, 70%, 75%, 80%, 85%, 90%, 92%, 95%, 97%, or 99%, particularly 100% sequence identity to SEQ ID NO: 1.
8. The polypeptide according to any one of embodiments 1-7, wherein the sequence defined in a2 has at least 45%, 50%, 55%, 60%, 65%, 70%, 75%, 80%, 85%, 90%, 92%, 95%, 97%, or 99%, particularly 100% sequence identity to SEQ ID NO: 2.
9. The polypeptide according to any one of embodiments 1-8, wherein the sequence defined in a3 has at least 55%, 60%, 65%, 70%, 75%, 80%, 85%, 90%, 92%, 95%, 97%, or 99%, particularly 100% sequence identity to SEQ ID NO: 3.
10. The polypeptide according to any one of embodiments 1-9, wherein the sequence defined in b1 has at least 30%, 35%, 40%, 45%, 50%, 55%, 60%, 65%, 70%, 75%, 80%, 85%, 90%, 92%, 95%, 97%, or 99%, particularly 100% sequence identity to SEQ ID NO: 4.
11. The polypeptide according to any one of embodiments 1-10, wherein the sequence defined in b2 has at least 20%, 25%, 30%, 35%, 40%, 45%, 50%, 55%, 60%, 65%, 70%, 75%, 80%, 85%, 90%, 92%, 95%, 97%, or 99%, particularly 100% sequence identity to SEQ ID NO: 7.
12. The polypeptide according to any one of embodiments 1-11, wherein the sequence defined in b3 has at least 50%, 55%, 60%, 65%, 70%, 75%, 80%, 85%, 90%, 92%, 95%, 97%, or 99%, particularly 100% sequence identity to SEQ ID NO: 5.
13. The polypeptide according to any one of embodiments 1-12, wherein the sequence defined in b4 has at least 50%, 55%, 60%, 65%, 70%, 75%, 80%, 85%, 90%, 92%, 95%, 97%, or 99%, particularly 100% sequence identity to SEQ ID NO: 6.
14. The isolated polypeptide according to embodiment 5 comprising the sequences a1, a2, a3, b1, b2, b3 and b4, wherein the sequences a1, a2, a3, b1, b2, b3 and b4 have the following sequence identities:
   a1 has at least 60% sequence identity to SEQ ID NO: 1, a2 has at least 50% sequence identity to SEQ ID NO: 2, a3 has at least 60% sequence identity to SEQ ID NO: 3,
   b1 has at least 35% sequence identity to SEQ ID NO: 4, b2 has at least 25% sequence identity to SEQ ID NO: 7, b3 has at least 55% sequence identity to SEQ ID NO: 5, and b4 has at least 55% sequence identity to SEQ ID NO: 6, more preferably
   wherein
   a1 has at least 70% sequence identity to SEQ ID NO: 1, a2 has at least 60% sequence identity to SEQ ID NO: 2, a3 has at least 70% sequence identity to SEQ ID NO: 3, b1 has at least 45% sequence identity to SEQ ID NO: 4, b2 has at least 35% sequence identity to SEQ ID NO: 7, b3 has at least 65% sequence identity to SEQ ID NO: 5, and b4 has at least 65% sequence identity to SEQ ID NO:6, and even more preferably wherein
   a1 has at least 80% sequence identity to SEQ ID NO: 1, a2 has at least 70% sequence identity to SEQ ID NO: 2, a3 has at least 80% sequence identity to SEQ ID NO: 3, b1 has at least 55% sequence identity to SEQ ID NO: 4, b2 has at least 45% sequence identity to SEQ ID NO: 7, b3 has at least 75% sequence identity to SEQ ID NO: 5, and b4 has at least 75% sequence identity to SEQ ID NO: 6.
15. The isolated polypeptide according to any one of embodiments 1-14, wherein at least five, particularly at least six, especially all seven of SEQ ID NOs: 1-7 are of *Clitopilus passeckerianus* origin.
16. The isolated polypeptide according to any one of embodiments 1-15, wherein the molecular weight of the polypeptide is between 90 kDa and 140 kDa, particularly between 100 kDa and 130 kDa, especially between 105 kDa und 120 kDa, and/or
   wherein the polypeptide comprises an amino acid sequence which amino acid sequence comprises a sequence having at least 70% sequence identity to SEQ ID NO: 9, more preferably at least 80%, even more preferably at least 85%, or even at least 90%, such as even more preferably at least 95% sequence identity to SEQ ID NO:9.
17. The isolated polypeptide according to any one of embodiments 1-16, wherein said polypeptide is a diterpene synthase, particularly a pleuromutilin synthase, and/or wherein said polypeptide has diterpene synthase activity, particularly pleuromutilin synthase activity.
18. The isolated polypeptide according to any one of embodiments 1-17, wherein the polypeptide is involved in the biosynthetic pathway for producing pleuromutilin.
19. The isolated polypeptide according to any one of embodiments 1-18, wherein the polypeptide is capable of catalyzing the conversion of geranylgeranyl pyrophosphate into a pleuromutilin precursor, particularly into a compound according to formula (I).
20. The isolated polypeptide according to any one of embodiments 1-19, wherein said polypeptide is derivable from a fungal host, particularly a fungus from the division basidomycota, more particularly from the order agaricales, even more particularly from the family entolomataceae;
   in particular wherein said polypeptide is derivable from the genus *Clitopilus* or from the genus *Pleurotus*;
   especially wherein said polypeptide is derivable from any one of *Clitopilus scyphoides, Clitopilus prunulus, Clitopilus hobsonii, Clitopilus pseudo-pinsitus, Clitopilus pinsitus* and *Clitopilus passeckerianus,* in particular from *Clitopilus pinsitus* or *Clitopilus passeckerianus.*
21. An isolated nucleic acid molecule comprising
   A) a nucleotide sequence encoding a polypeptide according to any one of embodiments 1 to 20 or a polypeptide of SEQ ID NO: 9,
   B) a nucleotide sequence which is
      a) the sequence of SEQ ID NO: 8; or
      a') the sequence of SEQ ID NO: 15 or the sequence complementary thereto; or
      b) a partial sequence of a sequence defined in a'), which partial sequence encodes a diterpene synthase; or
      c) a sequence which encodes a diterpene synthase and has at least 40% sequence identity to a sequence defined in a') or has at least 60% sequence identity to the sequence defined in a) or the partial sequence defined in b); or
      d) a sequence which encodes a diterpene synthase and which is degenerate as a result of the genetic code to a sequence defined in any one of a), a'), b) and c); or
      e) a sequence which encodes a diterpene synthase and which is capable of hybridizing to SEQ ID NO: 8 and/or SEQ ID NO: 13 under stringent conditions,
   C) at least 18 consecutive nucleotides of a nucleotide sequence as defined in item B, and/or
   D) at least 18 consecutive nucleotides and capable of hybridizing to a nucleic acid molecule having a nucleotide sequence as defined in item A or item B under stringent conditions.
22. An isolated nucleic acid molecule comprising a nucleotide sequence encoding a polypeptide according to any one of embodiments 1 to 20 or a polypeptide of SEQ ID NO: 9.
23. An isolated nucleic acid molecule comprising a nucleotide sequence which is
   a) the sequence of SEQ ID NO: 8 or the sequence complementary thereto; or a') the sequence of SEQ ID NO: 15 or the sequence complementary thereto; or
   b) a partial sequence of the sequence defined in a) or a'), which partial sequence encodes a polypeptide having diterpene synthase activity; or
   c) a sequence which encodes a polypeptide having diterpene synthase activity and has at least 40% sequence identity to a sequence defined in a) or a') or has at least 60% sequence identity to the partial sequence defined in b); or
   d) a sequence which encodes a polypeptide having diterpene synthase activity and which is degenerate as a result of the genetic code to a sequence defined in any one of a), a'), b) and c); or
   e) a sequence which encodes a polypeptide having diterpene synthase activity and which is capable of hybridizing to a sequence defined in any one of a), a'), b) and c), particularly to SEQ ID NO: 8 and/or SEQ ID NO: 13, under stringent conditions,
24. An isolated nucleic acid molecule comprising a nucleotide sequence which is
   a) the sequence of SEQ ID NO: 15; or
   b) the sequence complementary thereto; or
   c) a sequence which encodes a polypeptide having diterpene synthase activity and has at least 40% sequence identity to the sequence defined in a) or b); or
   d) a sequence which encodes a polypeptide having diterpene synthase activity and which is degenerate as a result of the genetic code to a sequence defined in any one of a), b) and c); or
   e) a sequence which encodes a polypeptide having diterpene synthase activity and which is capable of hybridizing to a sequence defined in any one of a), b), c) and d), particularly to SEQ ID NO: 15, under stringent conditions.
25. An isolated nucleic acid molecule comprising a nucleotide sequence which is
   a) the sequence of SEQ ID NO: 15, which sequence encodes a gene cluster involved in a biosynthetic pathway for producing a diterpene; or
   b) the sequence of SEQ ID NO: 15, which sequence encodes a gene cluster involved in a biosynthetic pathway for producing pleuromutilin; or
   c) a sequence which encodes a polypeptide having diterpene synthase activity and has at least 40% sequence identity to the sequence defined in a) or b); or
   d) a sequence which encodes a polypeptide having diterpene synthase activity and which is degenerate as a result of the genetic code to a sequence defined in any one of a), b) and c); or
   e) a sequence which encodes a polypeptide having diterpene synthase activity and which is capable of hybridizing to SEQ ID NO: 15.
26. The isolated nucleic acid molecule according any one of embodiments 24 and 25, wherein said nucleic acid molecule comprises a gene cluster comprising nucleic acid sequences that encode for polypeptides which are capable of catalyzing the conversion of geranylgeranyl pyrophosphate into pleuromutilin, particularly that are capable of catalyzing the conversion of farnesyl diphosphate into pleuromutilin.
27. An isolated nucleic acid molecule comprising a nucleotide sequence which is
   a) the sequence of SEQ ID NO: 8; or
   b) the sequence of SEQ ID NO: 13; or
   c) a sequence which encodes a polypeptide having diterpene synthase activity and has at least 60% sequence identity to the sequence defined in a) or b); or
   d) a sequence which encodes a polypeptide having diterpene synthase activity and which is degenerate as a result of the genetic code to a sequence defined in any one of a), b) and c); or
   e) a sequence which encodes a polypeptide having diterpene synthase activity and which is capable of hybridizing to a sequence defined in any one of a), b), c) and d) under stringent conditions.
28. An isolated nucleic acid molecule comprising a nucleotide sequence which is
   a) a partial sequence of SEQ ID NO: 15, which sequence encodes a polypeptide having diterpene synthase activity; or
   b) a partial sequence of SEQ ID NO: 15, which sequence encodes a polypeptide having pleuromutilin synthase activity; or
   c) a sequence which encodes a polypeptide having diterpene synthase activity and has at least 60% sequence identity to the partial sequence defined in a) or b); or
   d) a sequence which encodes a polypeptide having diterpene synthase activity and which is degenerate as a result of the genetic code to a partial sequence defined in any one of a), b) and c); or
   e) a sequence which encodes a polypeptide having diterpene synthase activity and which is capable of hybridizing to a sequence defined in any one of a), b), c) and d) under stringent conditions,
29. The isolated nucleic acid molecule according any one of embodiments 21, 23, 24, 25 27 and 28, wherein said at least 40% sequence identity in item c) is at least 45%, 50%, 55%, particularly at least 60%, 65%, 70%, especially at least 75%, 80%, 85%, more particularly at least 90%, 92%, 95%, 97%, 99%, particularly 100% sequence identity; and/ or
   wherein said at least 60% sequence identity in item c) is at least 65%, 70%, 75%, particularly at least 80%, 85%, especially at least 90%, 92%, more particularly at least 95%, 97%, 99%, particularly 100% sequence identity.
30. The nucleic acid molecule according to any one of embodiments 21-29, wherein said nucleic acid molecule is derivable from a fungal host, particularly a fungus from the division basidomycota, more particularly from the order agaricales, even more particularly from the family entolomataceae;
   in particular wherein said nucleic acid molecule is derivable from *Clitopilus* or from *Pleurotus;*
   especially wherein said nucleic acid molecule is derivable from any one of *Clitopilus scyphoides, Clitopilus prunulus, Clitopilus hobsonii, Clitopilus pseudo-pinsitus, Clitopilus pinsitus* and *Clitopilus passeckerianus,* in particular from *Clitopilus pinsitus* or *Clitopilus passeckerianus.*
31. The nucleic acid molecule according to any one of embodiments 21 to 30, wherein the sequence which encodes a polypeptide having pleuromutilin synthase activity is
   i) a sequence which encodes a diterpene synthase; and/or
   ii) a sequence which encodes a polypeptide having pleuromutilin synthase activity; and/or
   ii) a sequence which encodes a pleuromutilin synthase,
   particularly wherein said polypeptide having diterpene synthase activity is capable of catalyzing the conversion of geranylgeranyl pyrophosphate into a pleuromutilin precursor, especially into a compound according to formula (I).
32. An isolated nucleic acid molecule comprising at least 18, 19, 20, 25, particularly at least 30, 35, 40, 45, particularly at least 50, 55, 60, 65, particularly at least 70, 75, 80, 85, 90, 95, particularly at least 100, 150, 200, 250, particularly at least 300, 350, 400, 450, or 500 consecutive nucleotides of a sequence as defined in any one of embodiments 22 to 31; especially wherein the nucleic acid molecule further comprises a detectable label.
33. An isolated nucleic acid molecule comprising at least 18, 19, 20, 25, particularly at least 30, 35, 40, 45, particularly at least 50, 55, 60, 65, particularly at least 70, 75, 80, 85, 90, 95, particularly at least 100, 150, 200, 250, particularly at least 300, 350, 400, 450, or 500 consecutive nucleotides, wherein said nucleic acid molecule is capable of hybridizing to a nucleic acid molecule as defined in any one of embodiments 22 to 31 under stringent conditions; especially wherein the nucleic acid molecule further comprises a detectable label.
34. A polypeptide encoded by a nucleic acid molecule according to any one of embodiments 21 and 23 to 31.
35. The polypeptide according to embodiment 34, further defined as in any one of embodiments 1 to 20.
36. A vector comprising a nucleic acid molecule as defined in any one of embodiments 21 to 31, or a vector comprising a nucleic acid sequence as defined in any one of embodiments 21 to 31.
37. A non-naturally-occurring host selected from a cell, tissue and non-human organism, said host comprising at least one nucleic acid molecule comprising a nucleotide sequence as defined in any one of embodiments 21 to 31, particularly wherein said nucleotide sequence encodes a polypeptide having diterpene synthase activity, particularly a diterpene synthase or pleuromutilin synthase, especially wherein said polypeptide having diterpene synthase activity is capable of catalyzing the conversion of geranylgeranyl pyrophosphate into a pleuromutilin precursor, in particular into a compound according to formula (I).
38. A non-naturally-occurring host selected from a cell, tissue and non-human organism, said host comprising at least one vector according to embodiment 36.
39. A non-naturally-occurring host selected from a cell, tissue and non-human organism, said host comprising at least one nucleic acid molecule comprising a nucleotide sequence as defined in any one of embodiments 21 to 31,
   particularly wherein said nucleotide sequence encodes a polypeptide having diterpene synthase activity, particularly a diterpene synthase or pleuromutilin synthase, especially wherein said polypeptide having diterpene synthase activity is capable of catalyzing the conversion of geranylgeranyl pyrophosphate into a pleuromutilin precursor, in particular into a compound according to formula (I),
   and said host comprising at least one vector according to embodiment 36.
40. The host according to any one of embodiments 37 to 39, wherein said host is capable of producing a pleuromutilin precursor, in particular a compound according to formula (I).
41. The host according to any one of embodiments 37 to 40, wherein said host is capable of producing a diterpene or diterpenoid, particularly pleuromutilin.
42. The host according to any one of embodiments 37 to 41, wherein a corresponding naturally-occurring host selected from a cell, tissue and non-human organism not comprising said at least one said nucleic acid molecule and/or vector is capable of
   (i) producing a compound according to formula (I), and/ or
   (ii) producing pleuromutilin.
43. The host according to any one of embodiments 37 to 41, wherein a corresponding naturally-occurring host selected from a cell, tissue and non-human organism not comprising said at least one said nucleic acid molecule and/or vector is incapable of
   (i) producing a compound according to formula (I), and/ or
   (ii) producing pleuromutilin.
44. The host of according to any one of embodiments wherein said host is a fungal host, more particularly a fungus from the division basidomycota, even more particularly from the order agaricales, even more particularly from the family entolomataceae, in particular wherein said host is from the genus *Clitopilus* or from the genus *Pleurotus*;
   especially wherein said host is selected from the group consisting of *Clitopilus scyphoides, Clitopilus prunulus, Clitopilus hobsonii, Clitopilus pseudo-pinsitus, Clitopilus pinsitus* and *Clitopilus passeckerianus,* in particular from *Clitopilus pinsitus* or *Clitopilus passeckerianus.*
45. A method of producing a polypeptide according to any one of embodiments 1 to 20, 34 and 35, the method comprising
   (i) introducing into a host selected from a cell, tissue and non-human organism at least one nucleic acid molecule according to any one of embodiments 21 to 31 and/or at least one vector according to embodiment 36, and
   (ii) cultivating the host under conditions suitable for the production of the polypeptide,
   particularly wherein the method comprises a further step of (iii) recovering the polypeptide from the host.
46. A method of producing a polypeptide according to any one of embodiments 1 to 20, 34 and 35, the method comprising
   (i) introducing into a host selected from a cell, tissue and non-human organism at least one nucleic acid molecule according to any one of embodiments 21 to 31, and
   (ii) cultivating the host under conditions suitable for the production of the polypeptide,
   particularly wherein the method comprises a further step of (iii) recovering the polypeptide from the host.
47. A method of producing a polypeptide according to any one of embodiments 1 to 20, 34 and 35, the method comprising
   (i) introducing into a host selected from a cell, tissue and non-human organism at least one vector according to embodiment 36, and
   (ii) cultivating the host under conditions suitable for the production of the polypeptide,
   particularly wherein the method comprises a further step of (iii) recovering the polypeptide from the host.
48. A method of producing a polypeptide according to any one of embodiments 1 to 20, 34 and 35, the method comprising
   (i) introducing into a host selected from a cell, tissue and non-human organism at least one nucleic acid molecule according to any one of embodiments 21 to 31 and at least one vector according to embodiment 36, and
   (ii) cultivating the host under conditions suitable for the production of the polypeptide,
   particularly wherein the method comprises a further step of (iii) recovering the polypeptide from the host.
49. A method of producing pleuromutilin, the method comprising
   (i) introducing into a host selected from a cell, tissue and non-human organism a nucleic acid molecule according to any one of embodiments 21Ba', 23, 24, and 25 and/or a vector comprising a nucleic acid molecule according to any one of embodiments 21Ba', 23, 24, 25 and 26, and
   (ii) cultivating the host under conditions suitable for the production of pleuromutilin.
50. A method of producing pleuromutilin, the method comprising
   (i) introducing into a host selected from a cell, tissue and non-human organism a nucleic acid molecule according to any one of embodiments21 Ba', 23, 24, 25 and 26, and
   (ii) cultivating the host under conditions suitable for the production of pleuromutilin.
51. A method of producing pleuromutilin, the method comprising
   (i) introducing into a host selected from a cell, tissue and non-human organism a vector comprising a nucleic acid molecule according to any one of embodiments 21Ba', 23, 24, 25 and 26, and
   (ii) cultivating the host under conditions suitable for the production of pleuromutilin.
52. A method of producing pleuromutilin, the method comprising
   (i) introducing into a host selected from a cell, tissue and non-human organism a nucleic acid molecule according to any one of embodiments 21Ba', 23, 24, and 25 and a vector comprising a nucleic acid molecule according to any one of embodiments 21Ba', 23, 24, 25 and 26, and
   (ii) cultivating the host under conditions suitable for the production of pleuromutilin.
53. A method of producing a pleuromutilin precursor, in particular a compound according to formula (I), the method comprising
   (i) introducing into a host selected from a cell, tissue and non-human organism a nucleic acid molecule according to any one of embodiments 21 to 31 and/or a vector according to embodiment 36, and
   (ii) cultivating the host under conditions suitable for the production of said pleuromutilin precursor.
54. A method of producing a pleuromutilin precursor, in particular a compound according to formula (I), the method comprising
   (i) introducing into a host selected from a cell, tissue and non-human organism a nucleic acid molecule according to any one of embodiments 21 to 31, and
   (ii) cultivating the host under conditions suitable for the production of said pleuromutilin precursor.
55. A method of producing a pleuromutilin precursor, in particular a compound according to formula (I), the method comprising
   (i) introducing into a host selected from a cell, tissue and non-human organism a vector according to embodiment 36, and
   (ii) cultivating the host under conditions suitable for the production of said pleuromutilin precursor.
56. A method of producing a pleuromutilin precursor, in particular a compound according to formula (I), the method comprising
   (i) introducing into a host selected from a cell, tissue and non-human organism a nucleic acid molecule according to any one of embodiments 21 to 31 and a vector according to embodiment 36, and
   (ii) cultivating the host under conditions suitable for the production of said pleuromutilin precursor.
57. A method of altering the production of pleuromutilin in a host selected from a cell, tissue and non-human organism, wherein said host is capable of producing pleuromutilin and comprises at least one nucleic acid molecule comprising a nucleotide sequence as defined in any one of embodiments 21 to 31, the method comprising manipulating i) the expression, ii) the identity, or iii) both the expression and the identity of said at least one nucleic acid molecule;
   particularly wherein said method is
   a) a method of increasing the production of pleuromutilin, or
   b) a method of decreasing the production of pleuromutilin, in particular comprising disrupting or down-regulating said at least one nucleic acid molecule;
   especially wherein said host is a fungal host, more particularly a fungus from the division basidomycota, even more particularly from the order agaricales, even more particularly from the family entolomataceae.
58. A method of altering the production of pleuromutilin in a host selected from a cell, tissue and non-human organism, wherein said host is capable of producing pleuromutilin and comprises at least one nucleic acid molecule comprising a nucleotide sequence as defined in any one of embodiments 21 to 31, the method comprising manipulating the identity of said at least one nucleic acid molecule.
59. A method of altering the production of pleuromutilin in a host selected from a cell, tissue and non-human organism, wherein said host is capable of producing pleuromutilin and comprises at least one nucleic acid molecule comprising a nucleotide sequence as defined in any one of embodiments 21 to 31, the method comprising manipulating the expression of said at least one nucleic acid molecule.
60. The method according to any one of embodiments 57 to 59, wherein said method is a method of increasing the production of pleuromutilin.
61. The method according to any one of embodiments 57 to 59, wherein said method is a method of decreasing the production of pleuromutilin,
   in particular comprising disrupting or down-regulating said at least one nucleic acid molecule.
62. The method according to any one of embodiments 57 to 61, wherein said host is a fungal host, more particularly a fungus from the division basidomycota, even more particularly from the order agaricales, even more particularly from the family entolomataceae,
   in particular wherein said host is from the genus *Clitopilus* or from the genus *Pleurotus;*
   especially wherein said host is selected from group consisting of *Clitopilus scyphoides, Clitopilus prunulus, Clitopilus hobsonii, Clitopilus pseudo-pinsitus, Clitopilus pinsitus* and *Clitopilus passeckerianus,* in particular from *Clitopilus pinsitus* or *Clitopilus passeckerianus.*
63. Use of an isolated nucleic acid molecule according to any one of embodiments 21 to 31 in the production of pleuromutilin, wherein 2 to 50 nucleotides of the sequence of said nucleic acid molecule are divergent from a sequence of a gene cluster involved in the biosynthetic pathway for producing pleuromutilin comprised by a wild type organism capable of producing pleuromutilin; or wherein said nucleic acid molecule is a non-natural nucleic acid molecule.
64. Use of an isolated nucleic acid molecule according to any one of embodiments 21 to 31 in the production of a pleuromutilin precursor, wherein 2 to 50 nucleotides of the sequence of said nucleic acid molecule are divergent from a sequence encoding a diterpene synthase comprised by a wild type organism capable of producing pleuromutilin; or wherein said nucleic acid molecule is a non-natural nucleic acid molecule;
   particularly wherein said pleuromutilin precursor is a compound according to formula (I).
65. The use according to embodiment 63 or 64, wherein from 2 to 50 nucleotides are at least 3, 4, 5, 6, 7, 8, 9, 10, 11, 12, 13, 14, 15, 16, 17, 18, 19, 20, 25, 30, 35, 40, or 45 nucleotides,
   particularly from 3 to 100, more particularly from 5 to 80 nucleotides, even more particularly from 8 to 60 nucleotides.
66. Use of a host according to any one of embodiments 37 to 44, in the production of pleuromutilin or of a pleuromutilin precursor, particularly wherein said pleuromutilin precursor is a compound according to formula (I).
67. Use of an isolated nucleic acid molecule according to any one of embodiments 21 to 33, particularly of embodiments 32 or 33, for identifying one or more nucleic acids encoding a polypeptide having diterpene synthase activity, particularly pleuromutilin synthase activity and/or encoding a diterpene synthase, particularly a pleuromutilin synthase.
68. Use of an isolated nucleic acid molecule according to any one of embodiments 21 to 33, particularly of embodiments 32 or 33, in a method of isolating one or more nucleic acids encoding a polypeptide having diterpene synthase activity particularly pleuromutilin synthase activity and/or encoding a diterpene synthase, particularly a pleuromutilin synthase.
69. Use of an isolated nucleic acid molecule according to any one of embodiments 21 to 33, particularly of embodiments 32 or 33, in a method of isolating a polypeptide having diterpene synthase activity, particularly pleuromutilin synthase activity, and/or of isolating a diterpene synthase, particularly a pleuromutilin synthase.
70. The use of any one of embodiments 67 to 69, wherein said polypeptide having diterpene synthase activity or diterpene synthase is further defined as is the polypeptide of any one of embodiments 1 to 20.
71. The use of any one of embodiments 67 to 68, wherein said one or more nucleic acids are further defined as is the nucleic acid of any one of embodiments 21 to 33.
72. A method of the production of a pleuromutilin precursor, particularly of a compound according to formula (I), wherein the method
   A) is a method for the fermentative production of said precursor and particularly comprises the steps of
      (i) introducing into a host selected from a cell, tissue and non-human organism at least one nucleic acid molecule according to any one of embodiments 21 to 31 and/or at least one vector according to embodiment 36, and
      (ii) cultivating the host under conditions suitable for the fermentative production of said precursor;
   or
   B) is a method for the synthetic production of said precursor and comprises reacting geranylgeranylpyrophosphate with a polypeptide according to any one of claims 1 to 20, 34, and 35, or a polypeptide obtainable by a method of any one of embodiments 45 to 48.
73. A method of the production of a pleuromutilin precursor, particularly of a compound according to formula (I), wherein the method is a method for the fermentative production of said precursor and particularly comprises the steps of
   (i) introducing into a host selected from a cell, tissue and non-human organism at least one nucleic acid molecule according to any one of embodiments 21 to 31 and/or at least one vector according to embodiment 36, and
   (ii) cultivating the host under conditions suitable for the fermentative production of said precursor;
   particularly wherein the method comprises a further step of (iii) recovering said precursor from the host.
74. The method according to embodiment 72 or 73, wherein said host is a fungal host, more particularly a fungus from the division basidomycota, even more particularly from the order agaricales, even more particularly from the family entolomataceae,
   in particular wherein said host is from the genus *Clitopilus* or from the genus *Pleurotus*;
   especially wherein said host is selected from group consisting of *Clitopilus scyphoides, Clitopilus prunulus, Clitopilus hobsonii, Clitopilus pseudo-pinsitus, Clitopilus pinsitus* and *Clitopilus passeckerianus,* in particular from *Clitopilus pinsitus* or *Clitopilus passeckerianus.*
75. A method of the production of a pleuromutilin precursor, particularly of a compound according to formula (I), wherein the method is a method for the synthetic production of said precursor and comprises reacting geranylgeranylpyrophosphate with a polypeptide according to any one of claims 1 to 20, 34, and 35, or with a polypeptide obtainable by a method of any one of embodiments 45 to 48.
76. An isolated compound according to formula (I).
77. An isolated compound according to formula (I), wherein said compound is obtainable by a method of any one of embodiments 53 to 56 or 72 to 75.
78. A method for the production of a pleuromutilin antibiotic, wherein the method comprises a step of reacting
   i) a pleuromutilin precursor obtained by a method according to any one of embodiments 53 to 56 and 72 to 75;
   ii) a pleuromutilin precursor obtained by a use of embodiment 64; or
   iii) an isolated compound according to embodiment 76 or 77,
   the method optionally comprising further reaction steps to produce said pleuromutilin antibiotic.
79. Use of
   i) a pleuromutilin precursor obtained by a method according to any one of embodiments 53 to 56 and 72 to 75;
   ii) a pleuromutilin precursor obtained by a use of embodiment 64; or
   iii) an isolated compound according to embodiment 76 or 77;
   in the production of a pleuromutilin antibiotic, particularly a pleuromutilin derivative.
80. A pleuromutilin obtained according to any one of embodiments 49 to 52, 63 and 66
   i) for use as a medicament; or
   ii) for use in a method of treating a bacterial infection, particularly an infection caused by a bacterium selected from the group consisting of Gram-positive bacteria particularly selected from staphylococci, streptococci, pneumococci and enterococci; Gram-negative bacteria particularly selected from the genera *Neisseria, Haemophilus, Moraxella, Bordetella, Legionella, Leptospira*; mycoplasmas; chlamydia; Gram-positive anaerobes and Gram-negative anaerobes; or
   iii) for use in treating a disorder or disease involving a bacterium selected from the group consisting of Gram-positive bacteria particularly selected from staphylococci, streptococci, pneumococci and enterococci; Gram-negative bacteria particularly selected from the genera *Neisseria, Haemophilus, Moraxella, Bordetella, Legionella, Leptospira;* mycoplasmas; chlamydia; Gram-positive anaerobes and Gram-negative anaerobes.
81. A pleuromutilin antibiotic obtained by a method of embodiment 78
   i) for use as a medicament; or
   ii) for use in a method of treating a bacterial infection, particularly an infection caused by a bacterium selected from the group consisting of Gram-positive bacteria particularly selected from staphylococci, streptococci, pneumococci and enterococci; Gram-negative bacteria particularly selected from the genera *Neisseria, Haemophilus, Moraxella, Bordetella, Legionella, Leptospira*; mycoplasmas; chlamydia; Gram-positive anaerobes and Gram-negative anaerobes; or
   iii) for use in treating a disorder or disease involving a bacterium selected from the group consisting of Gram-positive bacteria particularly selected from staphylococci, streptococci, pneumococci and enterococci; Gram-negative bacteria particularly selected from the genera *Neisseria, Haemophilus, Moraxella, Bordetella, Legionella, Leptospira*; mycoplasmas; chlamydia; Gram-positive anaerobes and Gram-negative anaerobes.

### References

- D.M. Binninger, C. Skrzynia, P.J. Pukkila and L.A. Casselton (1987) DNA-mediated transformation of the Basidiomycete Coprinus cinereus, The EMBO Journal, p. 835-840, Vol 6, No. 4
- Dewick PM. (2002) The biosynthesis of C-5-C-25 terpenoid compounds. Nat Prod Rep; 19(2):181-222
- Kawaide H, Imai R, Sassa T, Kamiya Y. (1997) Ent-kaurene synthase from the fungus Phaeosphaeria sp. L487. cDNA isolation, characterization, and bacterial expression of a bifunctional diterpene cyclase in fungal gibberellin biosynthesis, J Biol Chem., 29; 272(35): 21706-12
- Kilaru S., Catherine M. Collins, Amanda J. Hartley, Claire Bums, Gary D. Foster (2009a). Investigating dominant selection markers for Coprinopsis cinerea: a carboxin resistance system and re-evaluation of hygromycin and phleomycin resistance vectors, Current Genetics, p. 543-550, Vol. 55
- Kilaru S, Collins CM, Hartley AJ, Bailey AM, Foster GD. (2009b) Establishing molecular tools for genetic manipulation of the pleuromutilin-producing fungus Clitopilus passeckerianus. Appl Environ Microbiol, 75(22): 7196-204.
- Hartley AJ, de Mattos-Shipley K, Collins CM, Kilaru S, Foster GD, Bailey AM. (2009) Investigating pleuromutilin-producing Clitopilus species and related basidiomycetes. FEMS Microbiol Lett; 297(1):24-30.
- Hu C, Zou Y. (2009) Mutilins Derivatives: From Veterinary to Human-used Antibiotics. Mini-Reviews in Medicinal Chemistry ; 9(12):1397-1406
- Hunt E. (2000) Pleuromutilin antibiotics. Drugs of the Future, 55(11): 1163-1168.
- Ikeda H, Ishikawa J, Hanamoto A, Shinose M, Kikuchi H, Shiba T et al. (2003) Complete genome sequence and comparative analysis of the industrial microorganism Streptomyces avermitilis. Nature Biotechnology; 21 (5):526-531
- Keller NP, Turner G, Bennett JW. (2005) Fungal secondary metabolism - From biochemistry to genomics. Nature Reviews Microbiology; 3(12):937-947
- Kuzuyama T. (2002) Mevalonate and nonmevalonate pathways for the biosynthesis of isoprene units. Bioscience Biotechnology and Biochemistry; 66(8):1619-1627
- Lacalle RA, J A Tercero, and A Jiménez (1992), Cloning of the complete biosynthetic gene cluster for an aminonucleoside antibiotic, EMBO J.; 11 (2): 785-792.
- G H Jones and D A Hopwood (1984), Molecular cloning and expression of the phenoxazinone synthase gene from Streptomyces, J. Biol. Chem. 259: 14151-14157.
- H Motamedi and C R Hutchinson (1987), Cloning and heterologous expression of a gene cluster for the biosynthesis of tetracenom, Proc Natl Acad Sci U S A.; 84(13): 4445―4449.
- Oliynyk M, Samborskyy M, Lester JB, Mironenko T, Scott N, Dickens S et al. (2007) Complete genome sequence of the erythromycin-producing bacterium Saccharopolyspora erythraea NRRL23338. Nature Biotechnology; 25(4):447-453
- Thompson.J.D., Gibson.T.J., Plewniak.F., Jeanmougin.F. and Higgins.D.G. (1997) The ClustalX windows interface: flexible strategies for multiple sequence alignment aided by quality analysis tools. Nucleic Acids Research 24:4876-4882
- Toyomasu T, Kawaide H, Ishizaki A, Shinoda S, Otsuka M, Mitsuhashi W et al. (2000) Cloning of a full-length cDNA encoding ent-kaurene synthase from Gibberella fujikuroi: Functional analysis of a bifunctional diterpene cyclase. Bioscience Biotechnology and Biochemistry; 64(3):660-664.
- Toyomasu T, Nakaminami K, Toshima H, Mie T, Watanabe K, Ito H, Matsui H, Mitsuhashi W, Sassa T, Oikawa H. (2004), Cloning of a gene cluster responsible for the biosynthesis of diterpene aphidicolin, a specific inhibitor of DNA polymerase alpha, Biosci Biotechnol Biochem., 68(1): 146-52.
- Tsukagoshi T, Tokiwano T, Oikawa H. (2007) Studies on the later stage of the biosynthesis of pleuromutilin. Bioscience Biotechnology and Biochemistry; 71(12):3116-3121
- Yao, Qingmei (2007) Biosynthetic Studies of Fungal Diterpene Antibiotics. Master Thesis, Oregon State University
- CN 101318921
- IN 2005CH00521
- WO 2009075776

## Claims

1. An isolated polypeptide,
the polypeptide comprising an amino acid sequence which comprises a sequence having at least 50% sequence identity to SEQ ID NO: 1,
a sequence having at least 40% sequence identity to SEQ ID NO: 2, and
at least one sequence selected from the group consisting of
i) a sequence having at least 15% sequence identity to SEQ ID NO: 7;
ii) a sequence having at least 25% sequence identity to SEQ ID NO: 4;
iii) a sequence having at least 45% sequence identity to SEQ ID NO: 5; and
iv) a sequence having at least 45% sequence identity to SEQ ID NO: 6,
wherein SEQ ID NOs: 1-2 and 4-7 are of *Clitopilus passeckerianus* origin and
wherein said polypeptide is a diterpene synthase.

2. The isolated polypeptide according to claim 1, wherein said amino acid sequence further comprises a sequence having at least 50% sequence identity to SEQ ID NO: 3, wherein SEQ ID NO: 3 is of *Clitopilus passeckerianus* origin.

3. The isolated polypeptide according to any one of claims 1 and 2, wherein the molecular weight of the polypeptide is between 90 kDa and 140 kDa, particularly between 100 kDa and 130 kDa, especially between 105 kDa und 120 kDa;
and/or
wherein the polypeptide comprises an amino acid sequence which amino acid sequence comprises a sequence having at least 70% sequence identity to SEQ ID NO: 9, more preferably at least 80%, even more preferably at least 85%, or even at least 90%, such as even more preferably at least 95% sequence identity to SEQ ID NO:9.

4. The isolated polypeptide according to any one of claims 1 to 3, wherein the polypeptide is capable of catalyzing the conversion of geranylgeranyl pyrophosphate into a pleuromutilin precursor, particularly into a compound according to formula (I).

5. An isolated nucleic acid molecule comprising
A) a nucleotide sequence encoding a polypeptide according to any one of claims 1 to 4 or a polypeptide of SEQ ID NO: 9,
B) a nucleotide sequence which is
a) the sequence of SEQ ID NO: 8 ; or
a') the sequence of SEQ ID NO: 15 or the sequence complementary thereto; or
b) a partial sequence of a sequence defined in a'), which partial sequence encodes a diterpene synthase; or
c) a sequence which encodes a diterpene synthase and has at least 40% sequence identity to a sequence defined in a') or has at least 60% sequence identity to the sequence defined in a) or the partial sequence defined in b); or
d) a sequence which encodes a diterpene synthase and which is degenerate as a result of the genetic code to a sequence defined in any one of a), a'), b) and c); or
e) a sequence which encodes a diterpene synthase and which is capable of hybridizing to SEQ ID NO: 8 and/or SEQ ID NO: 13 under stringent conditions,
C) at least 18 consecutive nucleotides of a nucleotide sequence as defined in item B, and/or
D) at least 18 consecutive nucleotides and capable of hybridizing to a nucleic acid molecule having a nucleotide sequence as defined in item A or item B under stringent conditions.

6. The isolated polypeptide according to any one of claims 1 to 4 or the isolated nucleic acid molecule according to claim 5, wherein said polypeptide or nucleic acid molecule is derivable from a fungal host, particularly a fungus from the division basidomycota, more particularly from the order agaricales, even more particularly from the family entolomataceae,
especially wherein said polypeptide or nucleic acid molecule is derivable from *Clitopilus pinsitus* or *Clitopilus passeckerianus.*

7. A vector comprising a nucleic acid sequence as defined in claim 5A or 5B.

8. A non-naturally-occurring host selected from a cell, tissue and non-human organism, said host comprising at least one nucleic acid molecule comprising a nucleotide sequence as defined in claim 5A or 5B and/or at least one vector according to claim 7,
in particular wherein said host is a fungal host, more particularly a fungus from the division basidomycota, even more particularly from the order agaricales, even more particularly from the family entolomataceae.

9. A method of producing a polypeptide according to any one of claims 1 to 4, the method comprising
(i) introducing into a host selected from a cell, tissue and non-human organism at least one nucleic acid molecule according to claim 5A or 5B and/or at least one vector according to claim 7, and
(ii) cultivating the host under conditions suitable for the production of the polypeptide,
particularly wherein the method comprises a further step of (iii) recovering the polypeptide from the host.

10. A method of producing pleuromutilin, the method comprising
(i) introducing into a host selected from a cell, tissue and non-human organism a nucleic acid molecule according to claim 5Ba' and/or a vector comprising a nucleic acid molecule according to claim 5Ba', and
(ii) cultivating the host under conditions suitable for the production of pleuromutilin.

11. A method of altering the production of pleuromutilin in a host selected from a cell, tissue and non-human organism, wherein said host is capable of producing pleuromutilin and comprises at least one nucleic acid molecule comprising a nucleotide sequence as defined in claim 5A or 5B, the method comprising manipulating i) the expression, ii) the identity, or iii) both the expression and the identity of said at least one nucleic acid molecule;
particularly wherein said method is
a) a method of increasing the production of pleuromutilin, or
b) a method of decreasing the production of pleuromutilin, in particular comprising disrupting or down-regulating said at least one nucleic acid molecule;
especially wherein said host is a fungal host, more particularly a fungus from the division basidomycota, even more particularly from the order agaricales, even more particularly from the family entolomataceae.

12. Use of an isolated nucleic acid molecule according to claim 5A or 5B
a) in the production of pleuromutilin, wherein 2 to 50 nucleotides of the sequence of said nucleic acid molecule are divergent from a sequence of a gene cluster involved in the biosynthetic pathway for producing pleuromutilin comprised by a wild type organism capable of producing pleuromutilin; or
b) in the production of a pleuromutilin precursor, wherein 2 to 50 nucleotides of the sequence of said nucleic acid molecule are divergent from a sequence encoding a diterpene synthase comprised by a wild type organism capable of producing pleuromutilin, particularly wherein said pleuromutilin precursor is a compound according to formula (I).

13. Use of a host according to claim 8, in the production of pleuromutilin or of a pleuromutilin precursor, particularly wherein said pleuromutilin precursor is a compound according to formula (I).

14. Use of an isolated nucleic acid molecule according to claim 5 for identifying one or more nucleic acids encoding a polypeptide having diterpene synthase activity, particularly encoding a diterpene synthase, especially encoding a pleuromutilin synthase.

15. A method of the production of a pleuromutilin precursor, particularly of a compound according to formula (I), wherein the method
A) is a method for the fermentative production of said precursor and comprises the steps of
(i) introducing into a host selected from a cell, tissue and non-human organism at least one nucleic acid molecule according to claim 5A or 5B and/or at least one vector according to claim 7, and
(ii) cultivating the host under conditions suitable for the fermentative production of said precursor;
or
B) is a method for the synthetic production of said precursor and comprises reacting geranylgeranyl pyrophosphate with a polypeptide according to any one of claims 1 to 4 or a polypeptide obtainable by a method of claim 9.
